(19) Europäisches Patentamt
European Patent Office
Office européen des brevets

(11) EP 1 973 884 B1

(12) # EUROPEAN PATENT SPECIFICATION

(45) Date of publication and mention of the grant of the patent:
**03.08.2016 Bulletin 2016/31**

(21) Application number: **06831640.5**

(22) Date of filing: **01.12.2006**

(51) Int Cl.:
*C07D 405/14* (2006.01) *C07D 401/04* (2006.01)
*C07D 403/04* (2006.01) *C07D 403/14* (2006.01)
*C07D 239/32* (2006.01) *C07D 239/42* (2006.01)
*C07D 409/12* (2006.01) *C07D 409/14* (2006.01)
*C07D 413/04* (2006.01) *C07D 417/14* (2006.01)
*C07D 213/30* (2006.01) *C07D 403/12* (2006.01)
*C07D 401/12* (2006.01) *C07D 407/12* (2006.01)
*C07D 417/12* (2006.01)

(86) International application number:
**PCT/IB2006/003468**

(87) International publication number:
**WO 2007/083182 (26.07.2007 Gazette 2007/30)**

(54) **NOVEL HETEROCYCLES**
NEUE HETEROCYCLEN
NOUVEAUX HÉTÉROCYCLES

(84) Designated Contracting States:
**AT BE BG CH CY CZ DE DK EE ES FI FR GB GR HU IE IS IT LI LT LU LV MC NL PL PT RO SE SI SK TR**

(30) Priority: **19.01.2006 IN CH00862006**

(43) Date of publication of application:
**01.10.2008 Bulletin 2008/40**

(73) Proprietor: **Orchid Pharma Limited**
**Nungambakkam, Chennai 600 034**
**TN (IN)**

(72) Inventors:
- **SRINIVAS, Visweswara, Akella, Staya, Surya**
**Tamil Nadu 600 119 (IN)**
- **TADIPARTHI, Ravikumar**
**Tamil Nadu 600 119 (IN)**
- **SHARMA, Ganapavarapu, Veera, Raghava**
**Tamil Nadu 600 119 (IN)**
- **THIRUNAVUKKARASU, Sappanimuthu**
**Tamil Nadu 600 119 (IN)**
- **BHAKIARAJ, Durairaj, Peter**
**Tamil Nadu 600 119 (IN)**
- **KACHHADIA, Virendra**
**Tamil Nadu 600 119 (IN)**
- **NARSIMHAN, Kilambi**
**Tamil Nadu 600 119 (IN)**
- **THARA, Sathya, Narayana**
**Tamil Nadu 600 119 (IN)**
- **RAJAGOPAL, Sriram**
**Tamil Nadu 600 119 (IN)**
- **REEDY, Gaddam, Om**
**Tamil Nadu 600 119 (IN)**
- **NARAYANAN, Sukunath**
**Tamil Nadu 600 119 (IN)**
- **PARAMESWARAN, Venkatesan**
**Tamil Nadu 600 119 (IN)**
- **JANARTHANAM, Venkatesan**
**Tamil Nadu 600 119 (IN)**

(74) Representative: **Srinivasan, Ravi Chandran**
**J A Kemp**
**14 South Square**
**Gray's Inn**
**London WC1R 5JJ (GB)**

(56) References cited:
**WO-A1-96/24585**
**WO-A1-2004/009560**
**WO-A1-2004/009560**
**WO-A2-03/084935**
**WO-A2-03/084938**
**US-A- 5 486 534**
**US-A- 6 028 072**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

### Field of the Invention

The present invention relates to novel heterocyclic compounds of the general formula (I), tautomeric forms, stereoisomers, polymorphs, hydrates, solvates, and pharmaceutically acceptable salts and compositions thereof. The present invention more particularly provides novel heterocycles of the general formula (I).

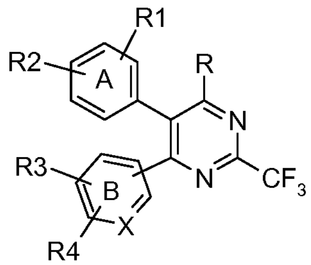

**(I)**

The present invention also provides a process for the preparation of the above said novel heterocyclic compounds of the general formula (I), tautomeric forms, stereoisomers, polymorphs, hydrates, solvates, and pharmaceutically acceptable salts and compositions thereof. This invention also relates to intermediates useful in the preparation of such compounds.

The novel heterocyclic compounds of the present invention are useful for the treatment of inflammation and immunological diseases. Particularly the compounds of the present invention are useful for the treatment of cancer, inflammation and immunological diseases those mediated by cytokines such as TNF-α, IL-1, IL-6, IL-1β, IL-8 and cyclooxygenases such as COX-1, COX-2 and COX-3. The compounds of the present invention are also useful for the treatment of rheumatoid arthritis; osteoporosis; multiple myeloma; uveititis; acute and chronic myelogenous leukemia; ischemic heart disease, atherosclerosis, cancer, ischemic-induced cell damage, pancreatic β cell destruction; osteoarthritis; rheumatoid spondylitis; gouty arthritis;

inflammatory bowel disease; adult respiratory distress syndrome (ARDS); psoriasis; Crohn's disease; allergic rhinitis; ulcerative colitis; anaphylaxis; contact dermatitis; asthma; muscle degeneration; cachexia; type I and type II diabetes; bone resorption diseases; ischemia reperfusion injury; brain trauma; multiple sclerosis; cerebral malaria; sepsis; septic shock; toxic shock syndrome; fever and myalgias due to infection; and diseases mediated by HIV-1; HIV-2; HIV-3; cytomegalovirus (CMV); influenza; adenovirus; the herpes viruses (including HSV-1, HSV-2) and herpes zoster viruses.

### Background of the Invention

The present invention is concerned with the treatment of immunological diseases or inflammation, notably such diseases are mediated by cytokines or cyclooxygenases. The principal elements of the immune system are macrophages or antigen-presenting cells, T cells and B cells. The role of other immune cells such as NK cells, basophils, mast cells and dendritic cells are known, but their role in primary immunologic disorders is uncertain. Macrophages are important mediators of both inflammation and provide the necessary "help" for T cell stimulation and proliferation. Most importantly macrophages make IL-1, IL-12 and TNF-α, all of which are potent pro-inflammatory molecules and also provide help for T cells. In addition, activation of macrophages results in the induction of enzymes, such as cyclooxygenase-2 (COX-2) and cyclooxygenase-3 (COX-3), inducible nitric oxide synthase (iNOS) and production of free radicals capable of damaging normal cells. Many factors activate macrophages, including bacterial products, superantigens and interferon gamma (IFN γ). It is believed that phosphotyrosine kinases (PTKs) and other undefined cellular kinases are involved in the activation process.

Cytokines are molecules secreted by the immune cells, large number of chronic and acute conditions have been recognized to be associated with perturbation of the inflammatory responses. A large number of cytokines participate in this response, including IL-1, IL-6, IL-8 and TNF. It appears that the activity of these cytokines in the regulation of inflammation relies at least in part on the activation of an enzyme on the cell-signaling pathway, a member of the MAP known as CSBP and RK. This kinase is activated by dual phosphorylation after stimulation by physiochemical stress, treatment with lipopolysaccharides or with proinflammatory cytokines such as IL-1 and TNF. Therefore, inhibitors of the kinase activity of p38 are useful anti-inflammatory agents.

Cytokines are molecules secreted by the immune cells that are important in mediating immune responses.

Cytokine production may lead to the secretion of other cytokines, altered cellular function, cell division or differentiation. Inflammation is the body's normal response to injury or infection. However, in inflammatory diseases such as rheumatoid arthritis, pathologic inflammatory processes can lead to morbidity and mortality. The cytokine tumor necrosis factor-alpha (TNF-α) plays a central role in the inflammatory response and has been targeted as a point of intervention in inflammatory diseases. TNF-α is a polypeptide hormone released by activated macrophages and other cells. At low concentrations, TNF-α participates in the protective inflammatory response by activating leukocytes and promoting their migration to extravascular sites of inflammation (Moser et al., J Clin Invest, 83, 444-55,1989). At higher concentrations, TNF-α can act as a potent pyrogen and induce the production of other pro-inflammatory cytokines (Haworth et al., Eur J Immunol, 21, 2575-79, 1991; Brennan et al., Lancet, 2, 244-7, 1989). TNF-α also stimulates the synthesis of acute-phase proteins. In rheumatoid arthritis, a chronic and progressive inflammatory disease affecting about 1% of the adult U.S. population, TNF-α mediates the cytokine cascade that leads to joint damage and destruction (Arend et al., Arthritis Rheum, 38, 151-60, 1995). Inhibitors of TNF-α, including soluble TNF receptors (etanercept) (Goldenberg, Clin Ther, 21, 75-87, 1999) and anti-TNF-α antibody (infliximab) (Luong et al., Ann Pharmacother, 34, 743-60, 2000), are recently approved by the U.S.FDA as agents for the treatment of rheumatoid arthritis.

Elevated levels of TNF-α have also been implicated in many other disorders and disease conditions, including cachexia, septic shock syndrome, osteoarthritis, inflammatory bowel disease (IBD) such as Crohn's disease and ulcerative colitis etc.

Elevated levels of TNF-α and/or IL-1 over basal levels have been implicated in mediating or exacerbating a number of disease states including rheumatoid arthritis; osteoporosis; multiple myeloma; uveititis; acute and chronic myelogenous leukemia; pancreatic β cell destruction; osteoarthritis; rheumatoid spondylitis; gouty arthritis; inflammatory bowel disease; adult respiratory distress syndrome (ARDS); psoriasis; Crohn's disease; allergic rhinitis; ulcerative colitis; anaphylaxis; contact dermatitis; asthma; muscle degeneration; cachexia; type I and type II diabetes; bone resorption diseases; ischemia reperfusion injury; atherosclerosis; brain trauma; multiple sclerosis; cerebral malaria; sepsis; septic shock; toxic shock syndrome; fever, and myalgias due to infection. HIV-1, HIV-2, HIV-3, cytomegalovirus (CMV), influenza, adenovirus, the herpes viruses (including HSV-1, HSV-2), and herpes zoster are also exacerbated by TNF-α.

It can be seen that inhibitors of TNF-α are potentially useful in the treatment of a wide variety of diseases. Compounds that inhibit TNF-α have been described in several patents.

Cytokines play an important role in the communication between cells of multicellular organisms. Early studies indicate that B cells lineage tend to secrete IL-6 in response to host immune defense mechanisms, but in recent decades studies have indicated elevated levels of IL-6 in various cancer phenotypes.

IL-6 has been found to be a growth factor for multiple myeloma cells; anti IL-6 antibodies were shown to block myeloma cell proliferation in leukemic patients (Lkein et al., Blood, 78, (5), pp 1198-1204,1991 and Lu et al., Err. J. Immunol., 22. 2819 -24,1992).

Elevation of inflammatory cytokine levels, particularly IL-6 and TNF-α also appears to be associated with the Cancer-related Cachexia, a syndrome involving loss of adipose and skeletal muscle tissue, and one that is not responsive to increased caloric intake. Cachexia may also be related to the role of acute phase proteins. The acute phase response and production of acute phase proteins (e.g., C-reactive protein [CRP]) are mediated by IL-6. Studies correlate elevated levels of IL-6 elevate acute phase proteins, which, interestingly, are also associated with increased weight loss and decreased survival. Thus, with elevated IL-6 levels, amino acid metabolism is directed away from peripheral tissues to the liver for production of acute phase proteins. This in turn leads to muscle wasting, which is a component of cachexia. Accordingly, the cytokine-induced acute phase response may be a primary component of cancer-related cachexia. Moreover, diminishing or blocking IL-6 activity in animal models attenuates cachexia, further demonstrating the essential role IL-6 plays in the development of this syndrome (For an excellent review see: Michael. J. Tisdale in "Biology of Cachexia", Journal of National Cancer Institute, Vol.89, No.23, Dec.3, 1997).

Thus having an IL-6 inhibitory activity, compound may be useful for various inflammatory diseases, sepsis, multiple myeloma, plasmacytoid leukemia, osteoporosis, cachexia, psoriasis, Nephritis, Kaposi's sarcoma, rheumatoid arthritis autoimmune disease, endometriosis and solid cancer (PCT: WO02/074298 A1). Compounds that inhibit IL-6 have been described in U.S. Patents: 6,004,813; 5,527,546 and 5,166,137.

The cytokine IL-1β also participates in the inflammatory response. It stimulates thymocyte proliferation, fibroblast growth factor activity, and the release of prostaglandins from synovial cells. Elevated or unregulated levels of the cytokine IL-1β have been associated with a number of inflammatory diseases and other disease states, including but not limited to adult respiratory distress syndrome, allergy, Alzheimer's disease etc. Since overproduction of IL-1β is associated with numerous disease conditions, it is desirable to develop compounds that inhibit the production or activity of IL-1β.

In rheumatoid arthritis models in animals, multiple intra-articular injections of IL-1 have led to an acute and destructive form of arthritis (Chandrasekhar et al., Clinical Immunol Immunopathol. 55, 382, 1990). In studies using cultured rheumatoid synovial cells, IL-1 is a more potent inducer of stromelysin than TNF-α. (Firestein, Am. J. Pathol. 140, 1309, 1992). At sites of local injection, neutrophil, lymphocyte, and monocyte emigration has been observed. The

emigration is attributed to the induction of chemokines (e.g., IL-8), and the up-regulation of adhesion molecules (Dinarello, Eur. Cytokine Netw. 5, 517-531, 1994).

In rheumatoid arthritis, both IL-1 and TNF-$\alpha$ induce synoviocytes and chondrocytes to produce collagenase and neutral proteases, which leads to tissue destruction within the arthritic joints. In a model of arthritis (collagen-induced arthritis, CIA in rats and mice) intra-articular administration of TNF-$\alpha$ either prior to or after the induction of CIA led to an accelerated onset of arthritis and a more severe course of the disease (Brahn et al., Lymphokine Cytokine Res. 11, 253, 1992; and Cooper, Clin. Exp.Immunol. 898, 244, 1992).

IL-8 has been implicated in exacerbating and/or causing many disease states in which massive neutrophil infiltration into sites of inflammation or injury (e.g., ischemia) is mediated; chemotactic nature of IL-8, including, but is not limited to, the following: asthma, inflammatory bowel disease, psoriasis, adult respiratory distress syndrome, cardiac and renal reperfusion injury, thrombosis and glomerulonephritis. In addition to the chemotaxis effect on neutrophils, IL-8 also has ability to activate neutrophils. Thus, reduction in IL-8 levels may lead to diminish neutrophil infiltration.

It has been reported that the cyclooxygenase enzyme exists in three isoforms, namely, COX-1, COX-2 and COX-3. COX-1 enzyme is essential and primarily responsible for the regulation of gastric fluids whereas COX-2 enzyme is present at the basal levels and is reported to have a major role in the prostaglandin synthesis for inflammatory response. These prostaglandins are known to cause inflammation in the body. Hence, if the synthesis of these prostaglandins is stopped by way of inhibiting COX-2 enzyme, inflammation and its related disorders can be treated. COX-3 possesses glycosylation-dependent cyclooxygenase activity. Comparison of canine COX-3 activity with murine COX-1 and COX-2 demonstrated that this enzyme is selectively inhibited by analgesic/antipyretic drugs such as acetaminophen, phenacetin, antipyrine, and dipyrone, and is potently inhibited by some nonsteroidal anti-inflammatory drugs. Thus, inhibition of COX-3 could represent a primary central mechanism by which these drugs decrease pain and possibly fever. Earlier reports prior to Coxib's development show that inhibitors of COX-1 enzyme causes gastric ulcers, where as selective COX-2 and COX-3 enzyme inhibitors are devoid of this function and hence are found to be safe. But, recent reports show that the selective COX-2 inhibitors (COXIBs) are associated with cardiovascular risks. So, inhibition of COX-2 without causing cardiovascular risks and gastric ulcers due to inhibition of COX-1 are shown to be safe.

**Few prior art references, which disclose the closest compounds, are given here:**

**i)** US 6,420,385 discloses novel compounds of the formula (IIa),

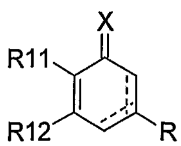

(IIa)

wherein:

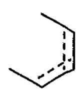

represents

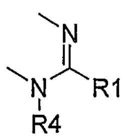

or

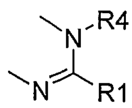

X is O, S or $NR_5$; $R_1$ and $R_2$ each independently represent --Y or --Z--Y, and $R_3$ and $R_4$ each independently --Z--Y or $R_3$ is a hydrogen radical; provided that $R_4$ is other than a substituted-aryl, (substituted-aryl)methyl or (substituted-aryl)ethyl radical; wherein each Z is independently optionally substituted alkyl, alkenyl, alkynyl, heterocyclyl, aryl or heteroaryl; Y is independently a hydrogen; halo, cyano, nitro, etc., $R_5$ is independently a hydrogen, optionally substituted alkyl, alkenyl, alkynyl etc., $R_{11}$ and $R_{12}$ are each independently represent optionally substituted aryl or heteroaryl.

An example of these compounds is shown in the formula (IIb),

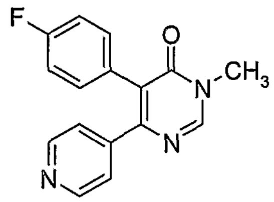

(IIb)

ii) US 5,728,704 discloses novel pyrimidines of the formula (I),

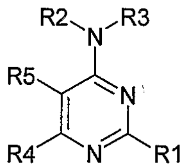

(I)

wherein $R^1$ is hydrogen, $CF_3$, $(C_1\text{-}C_6)$alkyl, $(C_1\text{-}C_6)$alkyl-S-$(C_1\text{-}C_6)$alkyl, $(C_1\text{-}C_6)$alkyl-SO-$(C_1\text{-}C_6)$alkyl, $(C_1\text{-}C_6)$alkyl-$SO_2$--$(C_1\text{-}C_6)$alkyl, hydroxy-$(C_1\text{-}C_6)$alkyl, dihydroxy-$(C_1\text{-}C_6)$alkyl, $C_1\text{-}C_6$)alkoxy, $(C_1\text{-}C_6)$alkoxycarbonyl-$(C_1\text{-}C_6)$alkyl, aryl selected from phenyl and naphthyl, aryl-$(C_1\text{-}C_6)$alkyl; $R_2$ and $R_3$ are independently selected from hydrogen, $(C_1\text{-}C_6)$alkyl, phenyl and phenyl-$(C_1\text{-}C_4)$alkyl, or $R^2$ and $R^3$ form, together with the nitrogen to which they are attached, a cyclic group selected from azetidino, pyrrolidino, piperidino, piperazino and morpholino, wherein said cyclic group may optionally be substituted; $R^4$ is hydrogen, chloro, bromo, cyano, nitro, trifluoromethyl, amino, $(C_1\text{-}C_6)$alkyl, $(C_1\text{-}C_6)$hydroxyalkyl, $(C_1\text{-}C_6)$alkoxy, phenyl, naphthyl or furyl, wherein said phenyl, naphthyl and furyl may optionally be substituted; $R^5$ is hydrogen, $(C_1\text{-}C_6)$alkyl, $(C_1\text{-}C_6)$alkoxy, trifluoromethyl, $(C_1\text{-}C_6)$hydroxyalkyl, -S-$(C_1\text{-}C_6)$alkyl, -SO-$(C_1\text{-}C_6)$alkyl, -$SO_2$-$(C_1\text{-}C_6)$alkyl, phenyl or furyl.

iii) US 6,420,385 and 6,410,729 discloses novel compounds of the formula (IIe),

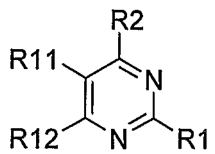

(IIe)

wherein $R_1$ and $R_2$ are each independently -Z-Y, preferably, $R_2$ is a radical of hydrogen, $C_1$-$C_4$ alkyl, halo, hydroxy, amino, etc., Z is independently a bond, alkyl, alkenyl etc., Y is independently a hydrogen radical, halo, nitro radical; $R_{20}$ is independently (1) alkyl, alkenyl, heterocyclyl radical, aryl, heteroaryl; $R_{21}$ is independently hydrogen radical, $R_{20}$; $R_{22}$ is independently hydrogen, heterocyclyl, aryl or heteroaryl.

iv) US 2005/0107413 discloses novel compounds of the formula (I),

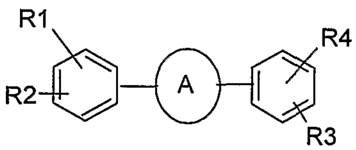

(I)

wherein $R_1$, $R_2$, $R_3$ and $R_4$ may be same or different and independently represent hydrogen, hydroxy, nitro, nitroso, formyl, azido, halo or substituted or unsubstituted groups selected from alkyl, haloalkyl, alkoxy, aryl, aryloxy, aralkyl, aralkoxy, heteroaryl, heterocyclyl, acyl, acyloxy, cycloalkyl, amino, hydrazine, monoalkylamino, dialkylamino, acylamino, alkylsufonyl, arylsulfonyl, alkylsulfinyl, arylsulfinyl, alkylthio, arylthio, alkoxycarbonyl, aryloxycarbonyl, alkoxyalkyl, sulfamoyl, carboxylic acid or its derivatives; A represents pyrimidine derivative of the formula

| | | | |
|---|---|---|---|
| 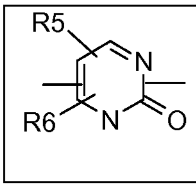 | 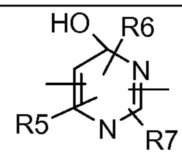 | 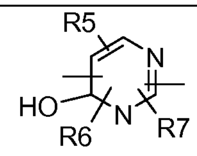 | 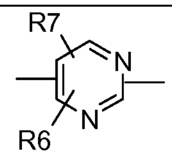 |

wherein $R_5$, $R_6$, $R_7$, may be same or different and represent, hydrogen, nitro, nitroso, formyl, azido, halo, or substituted or unsubstituted groups selected from alkyl, alkoxy, acyl, cycloalkyl, haloalkyl, amino, hydrazine, monoalkylamino, dialkylamino, acylamino, alkylsufonyl, alkylsulfinyl, arylsulfonyl, arylsulfinyl, alkylthio, arylthio, alkoxycarbonyl, aryloxycarbonyl, alkoxyalkyl, sulfamoyl, carboxylic acid or its derivatives; the pyrimidine group may be attached to the phenyl ring through carbon or nitrogen atom.

WO 2004/009560 describes a class of pyrimidine derivatives for use in the treatment of inflammation and immunological diseases.

WO 03/084935 describes a class of diaryl pyrimidinedione derivatives for use in the treatment of inflammation and immunological diseases.

WO 96/24585 describes a class of substituted pyridyl compounds for use in treating inflammation and inflammation-related disorders.

US 6,028,072 describes a class of pyrazolyl compounds for use in treating inflammation and inflammation-related disorders.

US 5,486,534 describes a class of pyrazolyl compounds for use in treating inflammation and inflammation-related disorders.

## Objective of the Invention

We have focused our research to identify cytokine inhibitors predominantly acting through the inhibition of the tumor necrosis factor-α (TNF-α), which are devoid of any side effects normally associated with TNF-α inhibitors, and to identify novel small molecule anticancer agents. Our sustained efforts have resulted in novel heterocyclic compounds of the formula (I). The derivatives may be useful in the treatment of inflammation, cancer and immunological diseases. Particularly the compounds of the present invention are useful for the treatment of immunological diseases those mediated by cytokines such as TNF-α, IL-1, IL-6, IL-1β, IL-8, IL-12 and inflammation. The compounds of the present invention are also useful in the treatment of rheumatoid arthritis; osteoporosis; multiple myeloma; uveititis; acute and chronic myelogenous leukemia; ischemic heart disease; atherosclerosis; ischemic-induced cell damage; pancreatic β-cell destruction; osteoarthritis; rheumatoid spondylitis; gouty arthritis; inflammatory bowel disease; adult respiratory distress syndrome (ARDS); psoriasis; Crohn's disease; allergic rhinitis; ulcerative colitis; anaphylaxis; contact dermatitis; asthma; muscle degeneration; cachexia; bone resorption diseases; ischemia reperfusion injury; brain trauma; multiple sclerosis; sepsis; septic shock; toxic shock syndrome; fever, and myalgias due to infection.

## Summary of the Invention

The present invention provides a compound of the general formula (I),

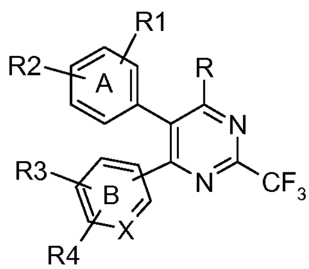

**(I)**

tautomeric forms, stereoisomers, polymorphs, solvates, and pharmaceutically acceptable salts and compositions thereof, wherein A represents substituted or unsubstituted phenyl group; B represents substituted or unsubstituted phenyl; X represents carbon atom;

- R represents substituted or unsubstituted groups selected from aryl; heteroaryl, the heteroaryl groups being selected from pyridyl, thienyl, furyl, pyrrolyl, oxazolyl, thiazolyl, imidazolyl, thiadiazolyl, tetrazolyl, pyrimidinyl, pyrazine, benzofuranyl, benzimidazolyl and benzothiazolyl; $-OSO_2R'$, wherein R' is selected from substituted or unsubstituted alkyl, aryl, alkyldialkylamino, haloalkyl, heterocyclyl and heteroaryl groups; or R is a substituted or unsubstituted heterocyclyl group chosen from morpholine, piperazine, piperidine, pyrrolidine and thiazolidine, the heterocyclyl group being optionally substituted with substitutents independently selected from substituted or unsubstitued heteroaryl, alkylaryl ($-CH_2$-Aryl), alkylheteroaryl ($-CH_2$-Heteroaryl), substituted heteroarylcarbonyl (-CO-Heteroaryl), cyanoalkyl, alkylsulfonyl, haloalkylsulfonyl, formyl and another substituted or unsubstituted heterocyclyl group; the attachment of the heterocyclyl group to the pyrimidine ring being through carbon or nitrogen;
- $R_1$ represents hydrogen, hydroxy, nitro, azido, halogens, substituted or unsubstituted groups selected from alkyl, haloalkyl, alkoxy, aryl, aryloxy, acyloxy, amino, hydrazine, monoalkylamino, dialkylamino, acylamino, alkylsufonyl, alkylsulfinyl, alkylthio, alkoxycarbonyl, alkoxyalkyl, sulfamoyl, $-SO_2NHNH_2$ $-SO_2Cl$, and carboxylic acid;
- $R_2$ represents hydrogen, hydroxy, nitro, azido, halogens, substituted or unsubstituted groups selected from alkyl, haloalkyl, alkoxy, aryl, aryloxy, acyloxy, amino, hydrazine, monoalkylamino, dialkylamino, acylamino, alkylsufonyl, alkylsulfinyl, alkylthio, alkoxycarbonyl, alkoxyalkyl, sulfamoyl, $-SO_2NHNH_2$, $-SO_2Cl$, and carboxylic acid;
- $R_3$ represents hydrogen, hydroxy, nitro, azido, halogens, substituted or unsubstituted groups selected from alkyl, haloalkyl, alkoxy, aryl, aryloxy, acyloxy, amino, hydrazine, monoalkylamino, dialkylamino, acylamino, alkylsufonyl, alkylsulfinyl, alkylthio, alkoxycarbonyl, alkoxyalkyl, sulfamoyl, $-SO_2NHNH_2$, $-SO_2Cl$, and carboxylic acid;
- $R_4$ represents hydrogen, hydroxy, nitro, azido, halogens, substituted or unsubstituted groups selected from alkyl, haloalkyl, alkoxy, aryl, aryloxy, acyloxy, amino, hydrazine, monoalkylamino, dialkylamino, acylamino, alkylsufonyl, alkylsulfinyl, alkylthio, alkoxycarbonyl, alkoxyalkyl, sulfamoyl, $-SO_2NHNH_2$, $-SO_2Cl$, and carboxylic acid; and

when the groups R, $R_1$, $R_2$, $R_3$, $R_4$ and R' are substituted by one or more substituents these substituents are selected from halogens, hydroxy, nitro, cyano, ureas, azido, amino, imino-1-phenyl butanone, amide, thioamide, hydrazine, alkyl, alkoxy, haloalkyl, haloalkoxy, cycloalkyl, aryloxy, acyl groups comprising acetyl and benzoyl, haloacyl, acyloxyacyl, heterocyclyl, aryl, heteroaryl, monoalkylamino, dialkylamino, acylamino, alkoxycarbonyl groups comprising methoxycarbonyl and ethoxycarbonyl, aryloxycarbonyl, alkylsulfonyl, haloalkylsulfonyl, arylsulfonyl, alkylsulfinyl, arylsulfinyl, thioalkyl, thioaryl, sulfamoyl, alkoxyalkyl groups, carboxylic acids and its derivatives comprising hydroxamic acid, hydroxamates, esters, amides and acid halides; these substituents being further optionally substituted with substituents selected from hydroxy, alkoxy, halogens, haloalkyl, alkyl and aryl which in turn is optionally further substituted by groups comprising halogens and alkyl.

The present invention also provides a pharmaceutical composition comprising a compound according to the invention, as an active ingredient along with a pharmaceutically acceptable carrier, diluent, excipient or solvate.

The present invention also provides the use of a compound according to the invention in the manufacture of a medicament for treatment of a pain disorder, inflammation, or an immunological disease.

The present invention also provides the use of a compound according to the invention in the manufacture of a medicament for treatment of rheumatoid arthritis; osteoporosis; multiple myeloma; uveitis; acute and chronic myelogenous leukemia; ischemic heart disease; atherosclerosis; cancer; ischemic-induced cell damage; pancreatic beta cell destruction; osteoarthritis; rheumatoid spondylitis; gouty arthritis; inflammatory bowel disease; adult respiratory distress syndrome (ARDS); psoriasis; Crohn's disease; allergic rhinitis; ulcerative colitis; anaphylaxis; contact dermatitis; muscle degeneration; cachexia; asthma; bone resorption diseases; ischemia reperfusion injury; brain trauma; multiple sclerosis; sepsis; septic shock; toxic shock syndrome or fever and myalgias due to infection.

The present invention also provides the use of a compound according to the invention in the manufacture of a medicament for lowering plasma concentrations of any one of, a combination of, or all of TNF-$\alpha$, IL-1$\beta$ and IL-6.

The present invention also provides the use of a compound according to the invention in the manufacture of a medicament for inhibiting production of cytokines as selected from TNF-$\alpha$, IL-1$\beta$ and IL-6.

The present invention also provides the use of a compound according to the invention in the manufacture of a medicament for treating immunological diseases, those mediated by cytokines such as TNF-$\alpha$, IL-1$\beta$ and IL-6.

The present invention also provides a compound according to the invention for use in a method of treatment of the human or animal body by therapy, for example a method of treatment of a pain disorder, inflammation or an immunological disease.

## Detailed Description of the Invention

The present invention relates to novel heterocyclic compounds of the formula (I),

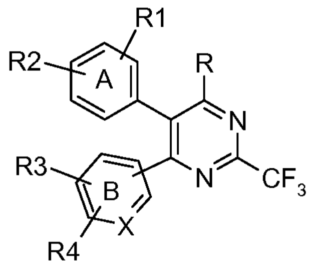

(I)

tautomeric forms, stereoisomers, polymorphs, solvates, and pharmaceutically acceptable salts and compositions thereof, wherein A, B, X, R, $R_1$, $R_2$, $R_3$ and $R_4$ are as defined above.

The term stereoisomer includes isomers that differ from one another in the way the atoms are arranged in space, but whose chemical formulas and structures are otherwise identical. Stereoisomers include enantiomers and diastereoisomers.

The term tautomers include readily interconvertible isomeric forms of a compound in equilibrium. The enol-keto tautomerism is an example.

The term polymorphs include crystallographically distinct forms of compounds with chemically identical structures.

The term pharmaceutically acceptable solvates includes combinations of solvent molecules with molecules or ions of the solute compound.

Pharmaceutically acceptable salts of the present invention include alkali metals like Li, Na, and K, alkaline earth metals like Ca and Mg, salts of organic bases such as diethanolamine, $\alpha$-phenylethylamine, benzylamine, piperidine, morpholine, pyridine, hydroxyethylpyrrolidine, choline hydroxyethylpiperidine, and the like, ammonium or substituted ammonium salts, aluminum salts. Salts also include amino acid salts such as glycine, alanine, cystine, cysteine, lysine, arginine, phenylalanine, guanidine etc. Salts may include acid addition salts where appropriate, which are, sulphates, nitrates, phosphates, perchlorates, borates, hydrohalides, acetates, tartrates, maleates, citrates, succinates, palmoates, methanesulphonates, tosylates, benzoates, salicylates, hydroxynaphthoates, benzenesulfonates, ascorbates, glycerophosphates, ketoglutarates and the like. Pharmaceutically acceptable solvates may be hydrates or comprising other solvents of crystallization such as alcohols.

A term once described, the same meaning applies for it, throughout the patent.

**Particularly useful compounds according to the present invention include:**

**18.** 6-[4-(Methylsulfonyl)phenyl]-5-phenyl-2-(trifluoromethyl)pyrimidin-4-ylnapthalenesulfonate;
**19.** 6-[4-(Methylsulfonyl)phenyl]-5-phenyl-2-(trifluoromethyl)pyrimidin-4-yl-3-chloropropane-1-sulfonate;
**20.** 6-[4-(Methylsulfonyl)phenyl]-5-phenyl-2-(trifluoromethyl)pyrimidin-4-yl-3-(trifluoromethyl)benzenesulfonate;
**21.** 6-[4-(Methylsulfonyl)phenyl]-5-phenyl-2-(trifluoromethyl)pyrimidin-4-yl-2-(trifluoromethyl)benzenesulfonate;
**22.** 6-[4-(Methylsulfonyl)phenyl]-5-phenyl-2-(trifluoromethyl)pyrimidin-4-yl-4-methylbenzenesulfonate;
**23.** 6-[4-(Methylsulfonyl)phenyl]-5-phenyl-2-(trifluoromethyl)pyrimidin-4-yl-4-nitrobenzenesulfonate;
**24.** 6-[4-(Methylsulfonyl)phenyl]-5-phenyl-2-(trifluoromethyl)pyrimidin-4-yl-4-trifluoromethoxybenzenesulfonate;
**25.** 6-[4-(Methylsulfonyl)phenyl]-5-phenyl-2-(trifluoromethyl)pyrimidin-4-yl thiophene-2-sulfonate;
**26.** 6-[4-(Methylsulfonyl)phenyl]-5-phenyl-2-(trifluoromethyl)pyrimidin-4-yl-4-fluorobenzenesulfonate;
**27.** 6-[4-(Methylsulfonyl)phenyl]-5-phenyl-2-(trifluoromethyl)pyrimidin-4-yl-2-fluorobenzenesulfonate;
**28.** 6-[4-(Methylsulfonyl)phenyl]-5-phenyl-2-(trifluoromethyl)pyrimidin-4-yl-(dimethylamino)propanesulfonate;
**29.** 6-[4-(Methylsulfonyl)phenyl]-5-phenyl-4-(N-benzyl-piperazin-1-yl)-2-(trifluoromethyl)pyrimidine;
**30.** 4-[4-(4-Fluorophenyl)-6-piperazin-1-yl-2-(trifluoromethyl)pyrimidin-5-yl] benzenesulfonamide;
**31.** 4-[5-(4-Fluorophenyl)-6-piperazin-1-yl-2-(trifluoromethyl)pyrimidin-4-yl] benzenesulfonamide;
**32.** *N*-Methyl-4-[4-(methylsulfonyl)phenyl]-6-piperazin-1-yl-2-(trifluoromethyl)pyrimidin-5-yl]benzenesulfonamide;
**33.** 4-[4-(Methylsulfonyl)phenyl]-6-piperazin-1-yl-2-(trifluoromethyl) pyrimidin-5-yl]benzenesulfonamide;
**34.** 4-{4-(Morpholin-4yl)-6-[4-(methylsulfonyl)phenyl]-2-(trifluoromethyl) pyrimidin-5-yl}-*N*-methylbenzenesulfonamide;

**35.** 5-{4-[4-(Methylsulfonyl)phenyl]-6-piperidin-1-yl-2-(trifluoromethyl) pyrimidin-5-yl}-*N*-methylbenzenesulfonamide;
**36.** 4-[4-(Methylsulfonyl)phenyl]-6-{4-[(5-methylpyrazin-2-yl)carbonyl] piperazin-1-yl}-5-phenyl-2-(trifluoromethyl)pyrimidine;
**37.** 6-[4-(Methylsulfonyl)phenyl]-5-phenyl-4-{4-[(1-methyl-1*H*-pyrrol-2-yl) carbonyl]piperazin-1-yl}-2-(trifluoromethyl)pyrimidine;
**38.** 6-[4-(Methylsulfonyl)phenyl]-4-[4-(5-nitro-2-furoyl)piperazin-1-yl]-5-phenyl-2-(trifluoromethyl)pyrimidine;
**39.** *N*-Methyl-4-{4-[4-(5-nitro-2-furoyl)piperazin-1-yl]-6-[4-(methylsulfonyl) phenyl]-2-(trifluoromethyl)pyrimidin-5-yl}benzenesulfonamide;
**40.** 4-{5-[4-Fluorophenyl]-4-[4-(5-nitro-2-furoyl)piperazin-1-yl]-2-(trifluoro methyl)pyrimidin-6-yl}benzenesulfonamide;
**41.** 4-{6-[4-Fluorophenyl]-4-[4-(5-nitro-2-furoyl)piperazin-1-yl]-2-(trifluoro methyl)pyrimidin-5-yl}benzenesulfonamide;
**42.** 6-[4-(Methylsulfonyl)phenyl]-4-{4-[(5-nitro-1*H*-pyrazol-3-yl)carbonyl] piperazin-1-yl}-5-phenyl-2-(trifluoromethyl)pyrimidine;
**43.** 5,6-Diphenyl-4-[4-(5-nitro-2-furoyl)piperazin-1-yl]-2-(trifluoromethyl) pyrimidine;
**45.** 6-[4-(Methylsulfonyl)phenyl]-5-phenyl-4-[4-(1,3-thiazol-2-yhnethyl) piperazin-1-yl]-2-(trifluoromethyl)pyrimidine;
**46.** 4-[4-(Methylsulfonyl)phenyl]-5-phenyl-6-[4-(pyridin-4-ylmethyl) piperazin-1-yl]-2-(trifluoromethyl)pyrimidine;
**47.** 6-[4-(Methylsulfonyl)phenyl]-4-{4-[(5-nitro-2-thienyl)methyl]piperazin-1-yl}-5-phenyl-2-(trifluoromethyl)pyrimidine;
**48.** 4,5-Diphenyl-6-(4-pyridin-2-yl-piperazin-1-yl)-2-(trifluoromethyl) pyrimidine;
**49.** 4-[4-(Methylsulfonyl)phenyl]-5-phenyl-6-(4-pyridin-2-yl-piperazin-1-yl)-2-(trifluoromethyl)pyrimidine;
**50.** 3-[4-(4-Fluorophenyl)-6-piperazin-1-yl-2-(trifluoromethyl) pyrimidin-5-yl]benzenesulfonamide;
**51.** 3-[5-Phenyl-6-piperazin-1-yl-2-(trifluoromethyl)pyrimidin-4-yl] benzenesulfonamide;
**52.** 3-[5-(3-Aminosulfonylphenyl)]-6-piperazin-1-yl-2-(trifluoromethyl) pyrimidin-4-yl]benzenesulfonamide;
**53.** 3-[4-(4-Fluorophenyl)-6-(4-pyridin-2-ylpiperazin-1-yl)-2-(trifluoromethyl) pyrimidin-5-yl]benzenesulfonamide;
**54.** 3-[4-(4-Fluorophenyl)-6-(4-pyrimidin-2-ylpiperazin-1-yl)-2-(trifluoromethyl)pyrimidin-5-yl]benzenesulfonamide;
**55.** 3-[5-Phenyl-6-(1,3-thiazolidin-3-yl)-2-(trifluoromethyl)pyrimidin-4-yl] benzenesulfonamide;
**57.** 3-[6-(4-Pyrimidin-2-ylpiperazin-1-yl)]-5-phenyl-2-(trifluoromethyl) pyrimidin-4-yl]benzenesulfonamide;
**58.** 3-[6-(4-Pyridin-2-ylpiperazin-1-yl)]-5-phenyl-2-(trifluoromethyl) pyrimidin-4-yl]benzenesulfonamide;
**59.** Ethyl-1-[5-(3-aminosulfonylphenyl)-6-(4-fluorophenyl)-2 -(trifluoromethyl)pyrimidin-4-yl]piperidine-4-carboxylate;
**61.** Ethyl 1-[5-phenyl-6-(3-aminosulfonylphenyl)1-2-(trifluoromethyl) pyrimidin-4-yl]piperidine-4-carboxylate;
**62.** 4-[5-Phenyl-6-(3-morpholinosulfonylphenyl)-2-(trifluoromethyl) pyrimidin-4-yl]morpholine;
**63.** 3-[4-(4-Fluorophenyl)-6-morpholin-4-yl-2-(trifluoromethyl)pyrimidin-5-yl]benzenesulfonamide;
**64.** (3*R*)-1-[6-(4-Fluorophenyl)-5-(3-aminosulfonylphenyl)-2-(trifluoro methyl)pyrimidin-4-yl]pyrrolidin-3-ol;
**65.** Ethyl (2*S*,4*R*)-4-hydroxy-1-[6-(4-fluorophenyl)-5-(3-aminosulfonyl phenyl)-2-(trifluoromethyl)pyrimidin-4-yl]pyrrolidine-2-carboxylate;
**66.** 4-[4-(2,6-Dimethoxypyrimidin-4-yl)piperazin-1-yl]-5-(3-aminosulfonyl phenyl)-6-(4-fluorophenyl)-2-(trifluoromethyl)pyrimidine;
**67.** 5-(4-Fluorophenyl)-4-(4-pyridin-2-ylpiperazin-1-yl)-6-[4-(methylsulfonyl) phenyl]-2-(trifluoromethyl)pyrimidine;
**68.** 4-(4-Methylsulfonylphenyl)-5-(4-fluorophenyl)-6-(4-pyrimidin-2-yl piperazin-1-yl)-2-(trifluoromethyl)pyrimidine;
**69.** 4-[5-(4-Fluorophenyl)-6-[4-(methylsulfonyl)phenyl]-2-(trifluoromethyl) pyrimidin-4-yl]piperazine-1-carbaldehyde;
**70.** 1'-[5-(4-Flurophenyl)-6-(4-methylsulfonylphenyl)-2-(trifluoromethyl) pyrimidin-4-yl]-1,4'-bipiperidine;
**71.** 3-[4-(4-Fluorophenyl)-6-(1,4'-bipiperidin-1'-yl)-2-(trifluoromethyl) pyrimidin-5-yl]benzenesulfonamide;
**72.** 3-[4-(2-Furoyl)piperazin-1-yl)-6-(4-fluorophenyl)-2-(trifluoromethyl) pyrimidin-5-yl]benzenesulfonamide;
**73.** 5-(3-Aminosulfonylphenyl)-4-(4-fluorophenyl)-2-(trifluoromethyl)-6-{4-[3-(trifluoromethyl)phenyl]piperazin-1-yl}pyrimidine;
**74.** 5-(4-Fluorophenyl)-4-(4-methylsulfonylphenyl)-2-(trifluoromethyl)-6-{4-[3-(trifluoromethyl)phenyl]piperazin-1-yl}pyrimidine;
**75.** 3-[4-(4-Fluorophenyl)-6-(1,3-thiazolidin-3-yl)-2-(trifluoromethyl) pyrimidin-5-yl]benzenesulfonamide;
**76.** 1-[5-[3-(Aminosulfonyl)phenyl]-6-(4-fluorophenyl)-2-(trifluoromethyl) pyrimidin-4-yl]pyrrolidine-2-carboxamide;
**77.** 5-(3-Aminosulfonylphenyl)-4-(4-fluorophenyl)-2-(trifluoromethyl)-6-{4-[(trifluoromethyl)sulfonyl]piperazin-1-yl}pyrimidine;
**78.** 3-[4-[4-(Methylsulfonyl)piperazin-1-yl]-6-(4-fluorophenyl)-2-(trifluoro methyl)pyrimidin-5-yl]benzenesulfonamide;
**79.** 3-[4-[4-(Cyanomethyl)piperazin-1-yl]-6-(4-fluorophenyl)-2-(trifluoro methyl)pyrimidin-5-yl]benzenesulfonamide;

**80.** 3-[4-(4-Fluorophenyl)-6-(1*H*-imidazol-1-yl)-2-(trifluoromethyl) pyrimidin-5-yl]benzenesulfonamide;
**81.** 5-(4-Fluorophenyl)-4-(1*H*-imidazol-1-yl)-6-[4-(methylsulfonyl)phenyl]-2-(trifluoromethyl)pyrimidine;
**82.** 3-[6-{4-[5-(trifluoromethyl)pyridin-2-yl]piperazin-1-yl}5-phenyl-2-(trifluoromethyl)pyrimidin-4-yl]benzenesulfonamide;
**83.** 3-[6-{4-[2,6-Dimethoxypyrimidin-4-yl]piperazin-1-yl}-5-phenyl-2-(trifluoromethyl)pyrimidin-4-yl]benzenesulfonamide;
**84.** 3-[6-{4-[5-(Nitro)pyridin-2-yl]piperazin-1-yl}-5-phenyl-2-(trifluoromethyl)pyrimidin-4-yl]benzenesulfonamide;
**85.** 3-[6-{4-[5-(Amino)pyridin-2-yl]piperazin-1-yl}-4-[4-fluorophenyl]-2-(trifluoromethyl)pyrimidin-5-yl]benzenesulfonamide;
**86.** 4-[5-(Acetylamino)pyridin-2-yl]piperazin-1-yl-5-(4-fluorophenyl)-6-[4-(methylsulfonyl)phenyl]-2-(trifluoromethyl)pyrimidine;
**87.** N-({3-[4-pyridin-2-yl]piperazin-1-yl)-6-(4-fluorophenyl)-2-(trifluorometlryl)pyrimidin-5-yl]phenyl}sulfonyl) acetamide;
**88.** 4-(4-Fluorophenyl)-5-(3-propionylaminosulfonylphenyl)-6-([4-pyridin-2-yl]piperazin-1-yl)-2-(trifluoromethyl)pyrimidine;
**89.** 1-{5-[3-(Aminosulfonyl)phenyl]-6-(4-fluorophenyl)-2-(trifluoromethyl)pyrimidin-4-yl}piperidine-4-carboxylic acid;
**90.** 4-[4-(Methoxyaminocarbonyl)piperidin-1-yl}-5-(4-fluorophenyl)-6-[4-(methylsulfonyl)phenyl]-2-(trifluoromethyl)pyrimidine;
**94.** 5-Amino-1-[5,6-diphenyl-2-(trifluoromethyl)pyrimidin-4-yl]-3-methyl-1*H* -pyrazole-4-carbonitrile;
**95.** Ethyl-5-amino-1-[5,6-diphenyl-2-(trifluoromethyl)pyrimidin-4-yl]-3-(methylthio)-1H-pyrazole-4-carboxylate;
**96.** 5-Amino-1-[5,6-diphenyl-2-(trifluoromethyl)pyrimidin-4-yl]-1*H*-pyrazole-4-carbonitrile;
97. 3-*t*-Butyl-1-[5,6-diphenyl-2-(trifluoromethyl)pyrimidin-4-yl]-1*H*-pyrazol-5-amine;
**98.** 4-(3,5-Dimethyl-1*H*-pyrazol-1-yl)-5,6-diphenyl-2-(trifluoromethyl) pyrimidine;
**99.** 3-[4-(5-Amino-4-cyano-3-methyl-1*H*-pyrazol-1-yl)-6-(4-fluorophenyl) -2-(trifluoromethyl)pyrimidin-5-yl]benzenesulfonamide;
**100.** Ethyl-5-amino-1-[5-[3-(aminosulfonyl)phenyl]-6-(4-fluorophenyl)-2-(trifluoromethyl)pyrimidin-4-yl]-3-(methylthio)-1*H*-pyrazole-4-carboxylate;
**101.** 4-[4-(Methylsulfonyl)phenyl]-5-phenyl-2-(trifluoromethyl)-6-[5-(trifluoromethyl)-1*H*-pyrazol-1-yl]pyrimidine;
**102.** 5-Amino-1-[5,6-diphenyl-2-(trifluoromethyl)pyrimidin-4-yl]-1*H*-pyrazole-4-carbothioamide;
**104.** *N*- {1-[5,6-Diphenyl-2-(trifluoromethyl)pyrimidin-4-yl]-3-*t*-butyl-1*H*-pyrazol-5-yl}-4-methoxybenzamide;
*105. N*-{1-[5,6-Diphenyl-2-(trifluoromethyl)pyrimidin-4-yl]-3-*t*-butyl-1*H*-pyrazol-5-yl}-3-fluorobenzamide;
**106.** *N*-{1-[5,6-Diphenyl-2-(trifluoromethyl)pyrimidin-4-yl]-3-*t*-butyl-1*H*-pyrazol-5-yl}-4-(trifluoromethyl)benzamide;
**107.** Ethyl-5-amino-1-[5-phenyl-6-[4-(methylsulfonyl)phenyl]-2-(trifluoromethyl)pyrimidin-4-yl]-3-(methylthio)-1*H*-pyrazole-4-carboxylate;
**108.** 5-Amino-1-[5,6-diphenyl-2-(trifluoromethyl)pyrimidin-4-yl]-3-(methyl thio)-*N*-phenyl-1*H*-pyrazole-4-carboxamide;
**109.** 5-Amino-*N*-(4,5-dimethylphenyl)-1-[5-(4-fluorophenyl)-6-pyridin-4-yl -2-(trifluoromethyl)pyrimidin-4-yl]-3-(methylthio)-1*H*-pyrazole-4-carboxamide;
**110.** 1-(2,6-Dichlorophenyl)-3-{1-[5,6-diphenyl-2-(trifluoromethyl)pyrimidin-4-yl]-3-t-butyl-1H-pyrazol-5-yl}urea;
**111.** 4-[4-(Methylthio) phenyl]-5,6-diphenyl-2-(trifluoromethyl)pyrimidine; and
**112.** 5-Phenyl-4-[4-(methylsulfonyl)phenyl]-6-[4-(methylthio)phenyl]-2-(trifluoromethyl)pyrimidine.

According to another embodiment of the present invention, there is provided a process for the preparation of novel heterocyclic compounds of the formula (I),

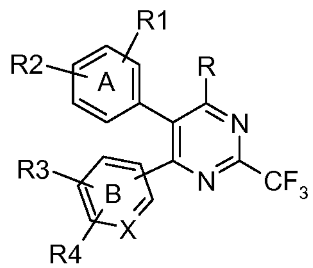

**(I)**

wherein R represents substituted or unsubstituted heterocyclyl groups, and the other symbols are as defined earlier and they may be prepared by converting the compound of formula (Ib), wherein all the symbols are as defined earlier.

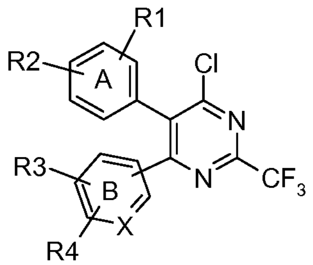

**(Ib)**

The compound of formula (Ib) is prepared according to the procedure described in our PCT/IB03/02879.

The conversion of the compound of the formula (Ib) is carried out with appropriate heterocyclyl or protected heterocyclyl groups such as morpholine, piperazine, benzylpiperazine, piperidine and the like and these heterocyclyl groups may be further substituted with heteroaryl, benzyl, alkyl heteroaryl, and other carboxylic acid heterocyclyl groups such as furoic acid, thiophene carboxylic acid, pyrazine carboxylic acid and the like in the presence or absence of appropriate solvents like toluene, xylene, tetrahydrofuran, dioxane, chloroform, dichloromethane, dichloroethane, o-dichlorobenzene, acetone, ethyl acetate, acetonitrile, N,N-dimethylformamide, dimethylsulfoxide, pyridine, diphenyl ether, ethanol, methanol, isopropylalcohol, tert-butylalchol, acetic acid, propionic acid etc or the like, or a mixture thereof or by neat reactions. The reaction may be carried out under acidic conditions using mineral or organic acids, or basic conditions viz. carbonates, bicarbonates, hydrides, hydroxides, alkyls and alkoxides of alkali metals and alkaline earth metals. The reaction mav be carried out in the presence of $Pd_2$(dba)3, racemic-2,2'-bis(diphenyl phosphino)-1,1'-dinaphthyl (BINAP), EDCI, HOBT, triethylamine, diisopropylethylamine, 4-dimethylaminopyridine or by using phase transfer catalysts viz. triethylbenzylammonium chloride, tetrabutylammonium bromide, tetrabutylammonium hydrogensulphate, tricaprylylmethylammonium chloride (aliquat 336) and the like. The reaction is usually carried out under cooling to refluxing conditions. The final product is purified by using chromatographic techniques or by recrystallization. The reaction may be carried out for a time period in the range of 2 to 20 hours.

In yet another embodiment of the present invention, there is provided a process for the preparation of novel heterocyclic compounds of the formula (I)

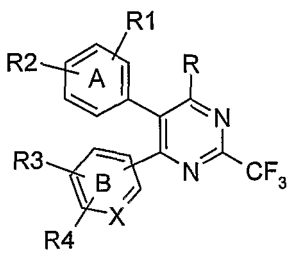

**(I)**

wherein R represents $-OSO_2R'$ and all the other symbols are as defined above, and may be prepared by converting the compound of formula (Ia), wherein all the other symbols are as defined earlier.

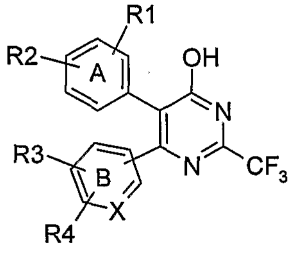

**(Ia)**

The compound of the formula (Ia) is prepared according to the procedure described in our PCT/IB03/01289.

The conversion of compound of the formula (Ia) is carried out with appropriate heterocyclyl or aryl or alkyl sulfonyl chlorides or sulfonic acids such as naphthalene sulfonyl chloride, naphthalene sulfonic acid, phenyl sulfonic acid, phenyl sulfonyl chloride, thiophene sulfonyl chloride, thiophene sulfonic acid, propyl sulfonyl chloride, propyl sulfonic acid, chloropropyl sulfonyl chloride and the like in the presence or absence of appropriate solvents like toluene, xylene, tetrahydrofuran, dioxane, chloroform, dichloromethane, dichloroethane, o-dichlorobenzene, acetone, ethyl acetate, acetonitrile, N,N-dimethylformamide, dimethylsulfoxide, pyridine, diphenyl ether, ethanol, methanol, isopropylalcohol, *t*-butylalchol, acetic acid, propionic acid etc, or the like, or a mixture thereof, or by neat reactions. The reaction may be

carried out under acidic conditions using mineral or organic acids, or basic conditions viz. carbonates, bicarbonates, hydrides, hydroxides, alkyl and alkoxides of alkali metals and alkaline earth metals. The reaction may be carried out in the presence of phase transfer catalysts viz. triethylbenzylammonium chloride, tetrabutylammonium bromide, tetrabutylammonium hydrogensulphate, tricaprylylmethylammonium chloride (aliquat 336) and the like. The reaction is usually carried out under cooling to refluxing conditions. The final product is purified by using chromatographic techniques or by recrystallization. The reaction may be carried out for a time period in the range of 2 to 20 hours.

In yet another embodiment of the present invention, there is provided a process for the preparation of novel heterocyclic compounds of the formula (I) wherein $R_1$, $R_2$, $R_3$ and $R_4$ represent $SO_2Cl$, and all other symbols are as defined earlier, and which comprises reacting compound of formula (Ic) wherein all symbols are as defined earlier. Wherein any of $R_1$, $R_2$, $R_3$, and $R_4$ which represents hydrogen on treatment with chlorosulfonic acid, is replaced by -$SO_2Cl$; this may result in both possibilities mono/disubstitution.

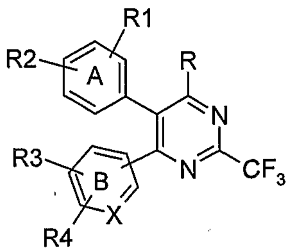

(Ic)

The reaction of the compound of formula (Ic) with chlorosulfonic acid may be carried out in the presence of solvents such as dichloromethane, acetone, tetrahydrofuran, dioxane, ethyl acetate, chloroform, and the like or a mixture thereof or in the absence of solvents. The reaction may be carried out at a temperature in the range of 0°C to reflux temperature for period in the range of 2 to 24 hours.

In yet another embodiment of the present invention, there is provided a process for the preparation of novel heterocyclic compounds of the formula (I), wherein $R_1$, $R_2$, $R_3$ or $R_4$ represent $-SO_2NHCH_3$, $-SO_2NHNH_2$ and all the other symbols are as defined earlier, which comprises reacting the compound of formula (Id), wherein $R_1$, $R_2$, $R_3$ or $R_4$ represents $-SO_2Cl$ and all the other symbols are as defined earlier; with methylamine or appropriate alkylamine or hydrazine hydrate or substituted hydrazine.

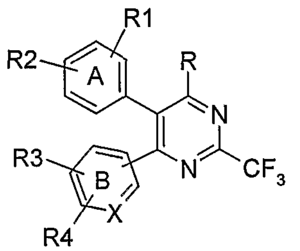

(Id)

The reaction of compound of formula (Id) with alkylamine or the appropriate hydrazine may be carried out in the presence of solvents such as acetonitrile, dichloromethane, acetone, tetrahydrofuran, dioxane, ethyl acetate, chloroform, water, an alcohol and the like or a mixture thereof or in absence of solvents. The reaction may be carried out at a temperature in the range of 0°C to reflux temperature for period in the range of 2 to 24 hours.

In yet another embodiment of the present invention, there is provided a process for the preparation of novel heterocyclic compounds of the formula (I)

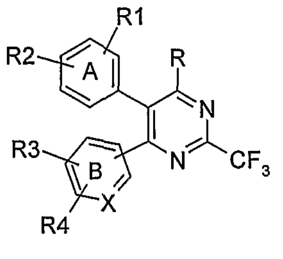

(I)

wherein R represents substituted or unsubstituted heteroaryl groups and the other symbols are as defined earlier and they may be prepared by converting the compound of formula (Ie), wherein all the symbols are as defined earlier.

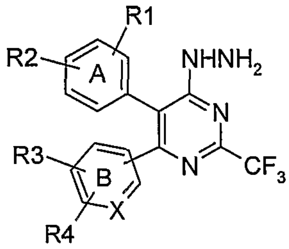

(Ie)

The reaction of (Ie) with reagents such as 1-methoxyethylidene malononitrile, ethyl-2-cyano-3,3-*bis*(methylthio)acrylate, ethoxymethylene malononitrile, pivaloyl nitrile, acetyl acetone, 1-ethoxyethylidene malononitrile etc., in alcoholic solvents such as ethanol, methanol, isopropanol, butanol etc., or in chlorinated solvents such as dichloromethane, dichloroethane, chloroform etc., or hydrocarbon solvents such as toluene etc., at temperatures ranging from 0 to 200°C for 0.5 to 24 hours. Some of the heterocyclic compounds thus obtained are further reacted with acyl or aroyl halides such as substituted or unsubstituted benzoyl chlorides in the presence of bases such as triethyl amine, diisopropylamine etc., in chlorinated solvents such as dichloromethane, dichloroethane, chloroform etc., at temperatures ranging from 0 to 100°C for 0.5 to 24 hours.

The reactions of (Ie) with 1,3-diketones such as acetylacetone, phenyltrifluoroacetyl acetone etc., was carriedout in alcoholic solvents such as ethanol, methanol etc., at temperatures ranging from 0 to 150°C for 0.5 to 24 hours.

The pharmaceutically acceptable salts are prepared by reacting the compound of formula (I) with 1 to 10 equivalents of a base such as sodium hydroxide, sodium methoxide, sodium hydride, potassium t-butoxide, calcium hydroxide, magnesium hydroxide and the like, in solvents like ether, tetrahydrofuran, methanol, t-butanol, dioxane, isopropanol, ethanol etc. Mixture of solvents may also be used. Organic bases such as diethanolamine, $\alpha$-phenylethylamine, benzylamine, piperidine, morpholine, pyridine, hydroxyethylpyrrolidine, hydroxyethylpiperidine, choline, guanidine and the like, ammonium or substituted ammonium salts, aluminum salts. Amino acids such as glycine, alanine, cystine, cysteine, lysine, arginine, phenylalanine etc may be used for the preparation of amino acid salts. Alternatively, acid addition salts wherever applicable are prepared by treatment with acids such as hydrochloric acid, hydrobromic acid, nitric acid, sulfuric acid, phosphoric acid, p-toluenesulphonic acid, methanesulfonic acid, acetic acid, citric acid, maleic acid, salicylic acid, hydroxynaphthoic acid, ascorbic acid, palmitic acid, succinic acid, benzoic acid, benzenesulfonic acid, tartaric acid, oxalic acid and the like in solvents like ethyl acetate, ether, alcohols, acetone, tetrahydrofuran, dioxane etc. Mixture of solvents may also be used.

It should be noted that compounds of the invention may contain groups that may exist in tautomeric forms, and though one form is named, described, displayed and/or claimed herein, all the hydrazine forms are intended to be inherently included in such name, description, display and/or claim.

The stereoisomers of the compounds forming part of this invention may be prepared by using reactants in their single enantiomeric form, in the process wherever possible or by conducting the reaction in the presence of reagents or catalysts in their single enantiomeric form or by resolving the mixture of stereoisomers by conventional methods. Some of the preferred methods include use of microbial resolution, resolving the diastereomeric salts formed with chiral acids such as mandelic acid, camphorsulfonic acid, tartaric acid, lactic acid, and the like wherever applicable or by using chiral bases such as brucine, cinchona alkaloids, their derivatives and the like. Commonly used methods are compiled by Jaques et al in "Enantiomers, Racemates and Resolution" (Wiley Interscience, 1981).

Various polymorphs of the compounds of the general formula (I), forming part of this invention may be prepared by crystallization of the compounds of formula (I) under different conditions. For example, using different commonly used solvents, or their mixtures for recrystallization; crystallizations at different temperatures; various modes of cooling, ranging from very fast to very slow cooling during crystallizations. Heating or melting the compounds followed by cooling gradually or immediately, one can also obtain polymorphs. The presence of polymorphs may be determined by solid probe NMR spectroscopy, IR spectroscopy, differential scanning calorimetry and powder X-ray diffraction or other such techniques.

Pharmaceutically acceptable solvates of the compounds of the formula (I) forming part of this invention may be prepared by conventional methods such as dissolving the compounds of the formula (I) in solvents such as water, methanol, ethanol, mixture of solvents such as acetone:water, dioxane:water, N,N-dimethylformamide:water and the like, preferably water and recrystallization by using different crystallization techniques

The present invention also provides a pharmaceutical composition, containing one or more of the compounds of the general formula (I) as defined above, their tautomeric forms, stereoisomers, polymorphs, hydrates, pharmaceutically acceptable salts, pharmaceutically acceptable solvates in combination with the usual pharmaceutically employed carriers, diluents and the like, useful for the treatment of inflammation, arthritis, pain, fever, psoriasis, allergic diseases, asthma, inflammatory bowel syndrome, gastrointestinal ulcers, cardiovascular disorders including ischemic heart disease, atherosclerosis, cancer, ischemic-induced cell damage, particularly brain damage caused by stroke and other pathological disorders associated with free radicals.

The pharmaceutical composition may be in the forms normally employed, such as tablets, capsules, powders, syrups, solutions, suspensions and the like, may contain flavorants, sweeteners etc. in suitable solid or liquid carriers or diluents, or in suitable sterile media to form injectable solutions or suspensions. The compositions may be pepared by processes known in the art. The amount of the active ingredient in the composition may be less than 70% by weight. Such compositions typically contain from 1 to 25%, preferably 1 to 15% by weight of active compound, the remainder of the composition being pharmaceutically acceptable carriers, diluents, excipients or solvents.

Suitable pharmaceutically acceptable carriers include solid fillers or diluents and sterile aqueous or organic solutions. The active compound will be present in such pharmaceutical compositions in the amounts sufficient to provide the desired dosage in the range as described above. Thus, for oral administration, the compounds can be combined with a suitable solid or liquid carrier or diluent to form capsules, tablets, powders, syrups, solutions, suspensions and the like. The pharmaceutical compositions, may, if desired, contain additional components such as flavorants, sweeteners, excipients and the like. For parenteral administration, the compounds can be combined with sterile aqueous or organic media to form injectable solutions or suspensions. For example, solutions in sesame or peanut oil, aqueous propylene glycol and the like can be used, as well as aqueous solutions of water-soluble pharmaceutically-acceptable acid addition salts or alkali or alkaline earth metal salts of the compounds. The injectable solutions prepared in this manner can then be, administered intravenously, intraperitoneally, subcutaneously, or intramuscularly, with intramuscular administration being preferred in humans.

The pharmaceutical compositions of the invention are effective in lowering TNF-$\alpha$, IL-1$\beta$, IL-6 levels, COX-1, and COX-2 activity without causing ulcers. The pharmaceutical compositions of the invention are thus effective for treating rheumatoid arthritis, osteoarthritis, rheumatoid spondylitis, gouty arthritis, inflammatory bowel disease, psoriasis, Crohn's disease, allergic rhinitis, ulcerative colitis, bone resorption diseases, and osteoporosis. The pharmaceutical compositions of the invention are also effective in the treatment of ischemic heart disease, ischemic-induced cell damage, ischemia reperfusion injury, atherosclerosis, brain trauma, multiple sclerosis, sepsis, septic shock, toxic shock syndrome, fever, and myalgias due to infection. The pharmaceutical compositions of the present invention are also effective in treating cancer, acute and chronic myelogenous leukemia, multiple myeloma, and pancreatic $\beta$ cell destruction. Furthermore, pharmaceutical compositions of the present invention are useful for the treatment of disorders, which includes adult respiratory distress syndrome (ARDS), anaphylaxis, contact dermatitis, asthma, muscle degeneration, cachexia, type I and type II diabetes.

Generally, the effective dose for treating a particular condition in a patient may be readily determined and adjusted by the physician during treatment to alleviate the symptoms or indications of the condition or disease. Generally, a daily dose of active compound in the range of about 0.01 to 1000 mg/kg of body weight is appropriate for administration to obtain effective results. The daily dose may be administered in a single dose or divided into several doses. In some cases, depending upon the individual response, it may be necessary to deviate upwards or downwards from the initially prescribed daily dose. Typical pharmaceutical preparations normally contain from about 0.2 to about 500 mg of active compound of formula I and/or its pharmaceutically active salts or solvates per dose.

While the compounds of the invention can be administered as the sole active pharmaceutical agent, they can also be used in combination with one or more compounds of the invention or other agents. When administered as a combination, the therapeutic agents can be formulated as separate compositions that are given at the same time or different times, or the therapeutic agents can be given as a single composition.

The term "therapeutically effective amount" or "effective amount" refers to that amount of a compound or mixture of compounds of Formula I that is sufficient to effect treatment, as defined below, when administered alone or in combination with other therapies to a mammal in need of such treatment. More specifically, it is that amount that is sufficient to lower the cytokines such as TNF-$\alpha$, IL-1$\beta$, IL-6, and to treat autoimmune diseases, inflammation, immunological diseases, and cancer.

The term "animal" as used herein is meant to include all mammals, and in particular humans. Such animals are also referred to herein as subjects or patients in need of treatment. The therapeutically effective amount will vary depending upon the subject and disease condition being treated, the weight and age of the subject, the severity of the disease condition, the particular compound of Formula I chosen, the dosing regimen to be followed, timing of administration, the manner of administration and the like, all of which can readily be determined by one of ordinary skill in the art.

The term "treatment" or "treating" means any treatment of a disease in a mammal, including:

a) Preventing the disease, that is, causing the clinical symptoms of the disease not to develop;

b) Inhibiting the disease, that is, slowing or arresting the development of clinical symptoms; and/or

c) Relieving the disease, that is, causing the regression of clinical symptoms.

From the foregoing description, one skilled in the art can easily ascertain the essential characteristics of this

invention, and without departing from the scope thereof, make various changes and modifications of the invention to adapt it to various usages and conditions.

The present invention is provided by the examples given below, which are provided by the way of illustration only, and should not be considered to limit the scope of the invention. Examples 18 to 43, 45 to 55, 57 to 59, 61 to 90, 94 to 102 and 104 to 112 are of the invention. Examples 1 to 17, 44, 56, 60, 91 to 93, and 103 are provided for reference. Variation and changes, which are obvious to one skilled in the art, are intended to be within the scope and nature of the invention, which are defined in the appended claims.

**Preparation 1**

**Synthesis of 6-[4-(methylsulfonyl)phenyl]-5-phenyl-2-(trifluoromethyl) pyrimidin-4-amine**

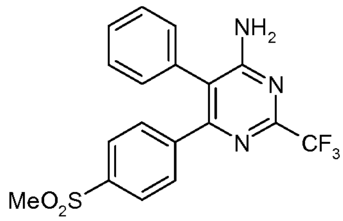

Ammonia gas was purged through a solution of 4-chloro-6-[4-(methylsulfonyl)phenyl]-5-phenyl-2-(trifluoromethyl)pyrimidine (5.5g, 13.33 mmol, prepared according to the procedure described in PCT/IB03/02879) in THF (500 ml), continuously for 30 hours under stirring at 0-10°C. Then after completion of the reaction the THF was distilled completely in-vacuo, water (100 ml) was added, and extracted with ethyl acetate (200 ml x 3). The combined organic layer was washed with brine (150 ml), dried over anhydrous sodium sulphate and concentrated under reduced pressure to give 6-[4-(methylsulfonyl)phenyl]-5-phenyl-2-(trifluoromethyl)pyrimidin-4-amine (3.6g, yield 87.79%).

**The following compounds were prepared according to the above procedure**

| Ex. | Structure | Analytical Data |
|---|---|---|
| 1 | 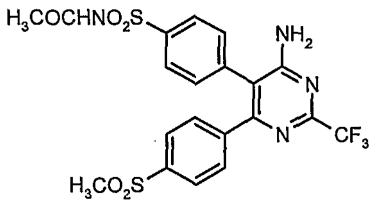 m.p: 153-158°C. | $^1$H-NMR (DMSO-$d_6$) δ: 1.86 (s, 3H), 3.16 (s, 3H), 6.77 (bs, 1H, $D_2O$ exchangeable), 7.38 - 7.55 (m, 2H), 7.59 - 7.73 (m, 2H), 7.75 - 7.86 (m, 5H), 12.04(s, 1H, $D_2O$ exchangeable). IR (KBr) $cm^{-1}$: 3472, 3342, 3204, 1715, and 1630. MS m/z: 515.1 ($M^+$+1). |
| 2 | 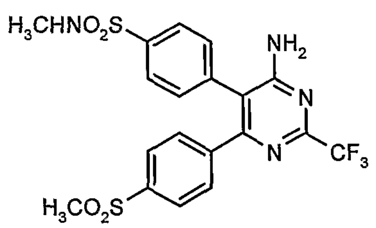 m.p: 272 - 276°C. | $^1$H-NMR (DMSO-$d_6$) δ: 2.04 - 2.05 (d, 3H), 3.16 (s, 3H), 6.66 (bs, $D_2O$ exchangeable), 7.29 - 7.30 (m, 1H, $D_2O$ exchangeable), 7.42 - 7.45 (m, 3H), 7.61 - 7.7 (m, 2H), 7.72 - 7.77 (m, 3H), 8.11 (bs, 1H, $D_2O$ exchangeable). IR (KBr) $cm^{-1}$: 3423, 3339, 3242, 1639, and 1579. MS m/z: 487.2 ($M^+$+1). |

**Example 3**

**Synthesis of 4-{4-chloro-6-[4-(methylsulfonyl)phenyl]-2-(trifluoromethyl) pyrimidin-5-yl}benzenesulfonyl chloride**

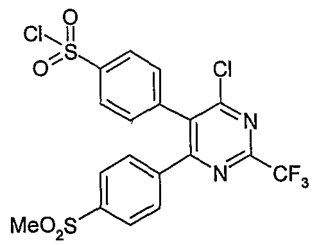

To a solution of chlorosulphonic acid (605mmol, 40.4ml) was added 4-chloro-6-[4-(methylsulfonyl)phenyl]-5-phenyl-2-(trifluoromethyl)pyrimidine (5.0g, 12.1mmol, prepared according to the procedure described in PCT/IB03/02879) slowly under continuous stirring at 0°C until the completion of the addition. Then the reaction mixture was stirred at 32°C until TLC confirmed the completion of the reaction. The resulting reaction mass was poured slowly under vigorous stirring onto crushed ice and the solid obtained was filtered, washed thoroughly with water (100 ml) and extracted with ethyl acetate (200 ml x 3). The combined organic layer was washed with brine (150 ml), dried over anhydrous sodium sulphate and concentrated under reduced pressure to afford 4-{4-chloro-6-[4-(methylsulfonyl)phenyl]-2-(trifluoromethyl)pyrimidin-5-yl}benzenesulfonyl chloride (2.75g, yield 65.59%, purity by HPLC 98.17%). M.p: 207 - 210°C. $^1$H-NMR (400 MHz, $CDCl_3$) δ: 3.01 (s, 3H), 7.511 - 7.515 (d, 2H), 7.53 - 7.67 (m, 1H), 7.72 - 7.76 (t, 1H), 7.86 - 7.89 (m, 3H), 8.11 - 8.13 (d, 1H). IR (KBr) $cm^{-1}$: 1557, 1524. MS m/z: 511.6($M^+$).

**Example 4**

**Synthesis of 4-{4-chloro-6-[4-(methylsulfonyl)phenyl]-2-(trifluoromethyl) pyrimidin-5-yl}-*N*-methylbenzenesulfonamide**

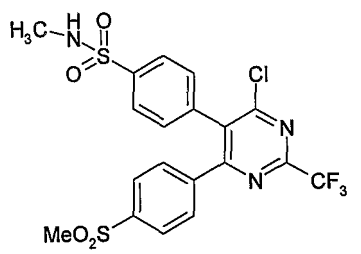

To a solution of 4-{4-chloro-6-[4-(methylsulfonyl)phenyl]-2-(trifluoromethyl)pyrimidin-5-yl}benzenesulfonyl chloride (0.5g, 0.98mmol, prepared according to the procedure described for Example 3) in dichloromethane (5ml) was added methylamine solution (0.08ml, 0.98mmol, 40% aqueous solution) under stirring at 0-10°C. After 15 minutes of the stirring, TLC confirmed the completion of the reaction. The solvent was distilled off under reduced pressure and the solid thus obtained was filtered, washed thoroughly with cold water to yield the title compound (0.446g, yield 90.1%, purity by HPLC 98.1%). M.p: 226 - 230°C. $^1$H-NMR (400 MHz, DMSO-$d_6$) δ: 2.09 - 2.10 (d, 3H), 3.1 (s, 3H), 7.43 - 7.44 (m, 1H, $D_2O$ exchangeable), 7.54 - 7.56 (d, 2H), 7.69 - 7.81 (m, 4H), 7.86 - 7.88 (d, 2H). IR (KBr) $cm^{-1}$: 3335, 1562, and 1530. MS m/z: 506.1 ($M^+$+1).

**The following compounds were prepared according to the procedure described for example 4**

| Ex. No | Structure | Analytical Data |
|---|---|---|
| 5. | 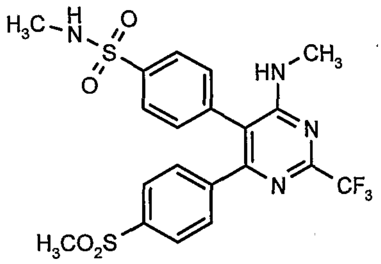 m.p: 265 - 267°C. | $^1$H-NMR (DMSO-$d_6$) $\delta$: 2.03 - 2.04 (d, 3H), 2.85 - 2.87 (d, 3H), 3.15 (s, 3H), 7.12 (s, 1H, $D_2O$ exchangeable), 7.13 - 7.31 (m, 1H, $D_2O$ exchangeable), 7.32 - 7.44 (m, 3H), 7.64 - 7.4 (m, 2H), 7.75 - 7.77 (m, 3H). IR (KBr) $cm^{-1}$: 3372, 3326, 1589, and 1551. MS m/z: 501.2 ($M^+$+1). |
| 6 | 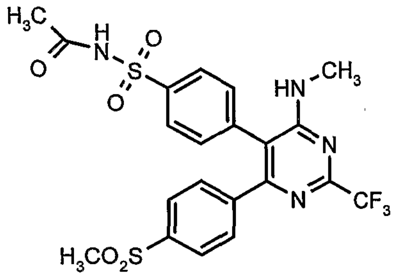 m.p: >285°C. | $^1$H-NMR (DMSO-$d_6$) $\delta$: 1.18 (s, 3H), 2.85 - 2.86 (d, 3H), 3.16 (s, 3H), 7.08-7.09 (m, 1H, $D_2O$ exchangeable), 7.4-7.42 (m, 2H), 7.55 - 7.57 (m, 1H), 7.62 - 7.66 (m, 1H), 7.72 - 7.76 (3H), 7.87 - 7.89 (d, 1H), 12.04 (s, 1H, $D_2O$ exchangeable). IR (KBr) $cm^{-1}$: 3413, 3151, 1719, and 1588. MS m/z: 529 ($M^+$+1). |

## Example 7

**Synthesis of 4-{4-hydrazino-6-[4-(methylsulfonyl)phenyl]-2-(trifluoro methyl)pyrimidin-5-yl}benzenesulfonohydrazide**

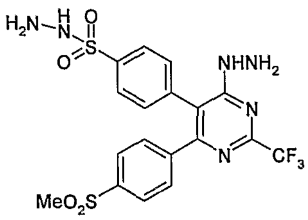

To a suspension of 4-{4-chloro-6-[4-(methylsulfonyl)phenyl]-2-(trifluoromethyl)pyrimidin-5-yl}benzenesulfonyl chloride (1g, 1.9 mmol, prepared according to the procedure described for Example 3) in ethanol (10ml) was added hydrazine hydrate (0.195g, 3.9mmol), at 0-10°C under stirring. The stirring was continued at the same temperature for 3 hours until TLC using ethyl acetate and hexane as solvent system confirmed the completion of the reaction. The solid that reappeared was filtered and washed with ethanol (5ml) and finally with hexane (10ml) to afford the desired compound (0.9g, yield 91.56%, purity by HPLC 94.49%). M.p. 203-206°C. $^1$H-NMR (DMSO-$d_6$) $\delta$: 3.17 (s, 3H), 4.0 (s, 2H, $D_2O$ exchangeable), 7.44-7.46 (d, 3H), 7.59 - 7.64 (m, 1H), 7.76 - 7.77 (d, 3H), 8.33s, 1H), 8.44 (s, 1H, $D_2O$ exchangeable). IR (KBr) $cm^{-1}$: 3353, 1581,1567. MS m/z: 503.1 ($M^+$+1).

**The following compounds were prepared according to the procedure described for example 7**

| Ex. | Structure | Analytical Data |
|---|---|---|
| **8** | 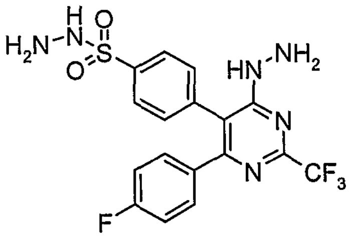 m.p:183 -184°C | $^{1}$H-NMR (DMSO-$d_6$) $\delta$: 4.04 (s, 2H, $D_2O$ exchangeable), 4.37 (s, 2H, $D_2O$ exchangeable), 7.05 - 7.1 (m, 2H), 7.18 - 7.27 (m, 3H), 7.41 - 7.43 (m, 2H), 7.59 - 7.63 (m, 1H), 8.30 - 8.35 (m, 1H, $D_2O$ exchangeable), 8.77 (bs, 1H, $D_2O$ exchangeable). MS m/z: 443 ($M^{+}$+1) |
| **9** | 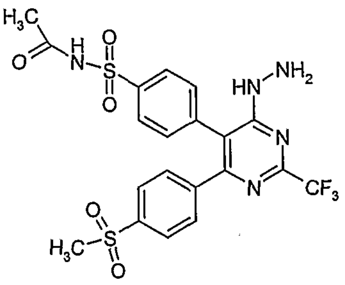 m.p: 255 - 257°C. | $^{1}$H-NNM (DMSO-$d_6$) $\delta$: 1.87 (s, 3H), 3.16 (s, 3H), 4.65 (bs, 2H, $D_2O$ exchangeable), 7.4 - 7.5 (m, 3H), 7.57 - 7.61 (m, 1H), 7.71 - 7.80 (m, 3H), 7.84 - 7.86 (d, 1H), 8.52 (s, 1H), 12.05 (s, 1H, $D_2O$ exchangeable). IR (KBr) $cm^{-1}$: 3388, 3209, 1734, and 1583. MS m/z: 530 ($M^{+}$+1). |
| **10** | 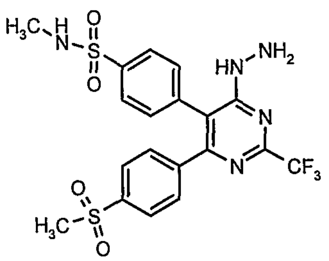 m.p: 252 - 254°C. | $^{1}$H-NMR (DMSO-$d_6$) $\delta$: 2.06 - 2.07 (d, 3H), 3.15 - 3.17 (s, 3H), 4.61 (bs, 2H, $D_2O$ exchangeable), 7.28 - 7.32 (m, 1H), 7.42 - 7.44 (m, 3H), 7.55-7.65 (m, 2H), 7.7 - 7.79 (m, 3H), 8.53 (s, 1H, $D_2O$ exchangeable). MS m/z: 502.2 ($M^{+}$+1). |
| **11** | 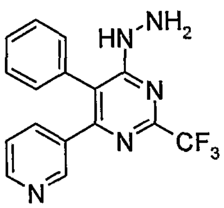 m.p: 141 - 144°C | $^{1}$H-NMR ($CDCl_3$) $\delta$: 4.09 (bs, 2H, $D_2O$, exchangeable), 6.27 (s, 1H, $D_2O$ exchangeable), 7.16 - 7.19 (m, 3H), 7.41 - 7.72 (m, 3H), 7.25 - 7.74 (m, 1H), 8.47 - 8.5 (m, 2H). MS m/z: 332 ($M^{+}$+1). |
| **12** | 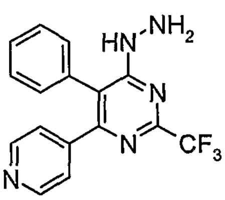 m.p: 156 - 159°C. | $^{1}$H-NMR ($CDCl_3$) $\delta$: 4.08 (bs, 2H, $D_2O$ exchangeable), 6.28 (s, 1H, $D_2O$ exchangeable), 7.13 - 7.18 (m, 2H), 7.21 - 7.22 (m, 2H), 8.46 (s, 3H), 8.46 - 8.47 (m, 2H). MS m/z: 332 ($M^{+}$+1). |
| **13** | 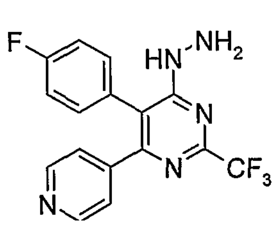 m.p: 187 - 189°C. | $^{1}$H-NMR ($CDCl_3$) $\delta$: 4.09 (bs, 2H, $D_2O$ exchangeable), 6.24 (s, 1H, $D_2O$ exchangeable), 7.14 - 7.16 (m, 4H), 7.18 - 7.20 (m, 2H), 8.49 - 8.5 (d, 2H). MS m/z: 350.2 ($M^{+}$+1). |

**Example 14**

**Synthesis of 2,2,2-trifluoro-*N'*-[5-(4-fluorophenyl)-6-pyridin-4-yl-2-(trifluoromethyl)pyrimidin-4-yl]acetohydrazide**

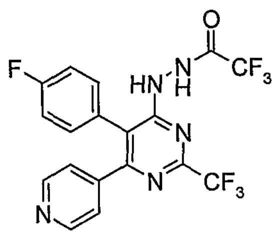

To a solution of 5-(4-fluorophenyl)-4-hydrazino-6-pyridin-4-yl-2-(trifluoromethyl)pyrimidine (0.5g, 1.43mmol, example 13, prepared according to the procedure described for example 7) in dichloromethane (10ml) was added trifluoroacetic anhydride (0.22ml, 1.57mmol) under stirring at -40°C. The stirring was continued at the same temperature for 30 minutes, until TLC using ethyl acetate and hexane (3:7) as solvent system confirmed the completion of the reaction. Subsequently the reaction mixture was poured onto ice and extracted with ethyl acetate (50ml), dried over anhydrous sodium sulphate and evaporated to afford the desired compound (0.34g, yield 53.3%, purity by HPLC 97%). M.p. 240-242 °C. $^1$H-NMR (DMSO-$d_6$) δ: 7.17 - 7.35 (m, 6H), 8.49 - 8.50 (d, 2H), 9.3 (s, 1H), 11.7 (s, 1H, $D_2O$ exchangeable). IR (KBr) cm$^{-1}$: 3678, 3395, 3208, and 1742. MS m/z: 446.1 ($M^+$+1).

**Following compounds were prepared according to the procedure described for example 14**

| Ex. | Structure | Analytical data |
|---|---|---|
| **15** | 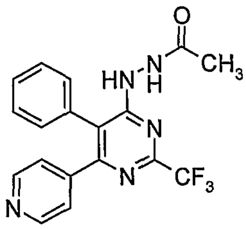 m.p.: 197 -198°C. | $^1$H-NMR ($CDCl_3$) δ: 2.12 (s, 3H), 7.21 - 7.22 (m, 2H), 7.46 - 7.47 (m, 3H), 8.0 (s, 1H), 8.48 - 8.49 (d, 2H). IR (KBr) cm$^{-1}$: 3257, 1694, 1577,1571. MS m/z: 374 ($M^+$+1). |
| **16** | 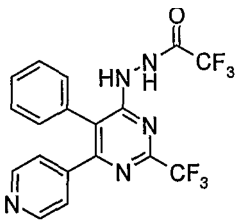 | $^1$H-NMR (DMSO-$d_6$) δ: 7.21 - 7.22 (m, 2H), 7.7.27 - 7.29 (m, 2H), 7.42 - 7.43 (d, 2H), 9.26 (s, 1H, $D_2O$ exchangeable), 10.8 (s, 1H, $D_2O$ exchangeable). MS m/z: 428 ($M^+$+1). |

**Example 17**

**Synthesis of N-[(4-{4-chloro-6-[4-(methylsulfonyl)phenyl]-2-(trifluoromethyl)pyrimidin-5-yl}phenyl)sulfonyl]acetamide**

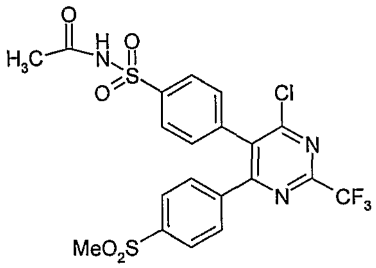

A solution of 4-{4-chloro-6-[4-(methylsulfonyl)phenyl]-2-(trifluoromethyl)pyrimidin-5-yl}benzenesulfonamide (0.5g, 1.01mmol, prepared according to the procedure described in PCT/IB03/02879), DMAP (0.01g) and acetyl chloride (0.5g, 6.36mmol) were stirred at 30°C. TLC confirmed the completion of the reaction after 4 hours of stirring under the same conditions. Subsequently the resulting mass was poured onto the ice, and the procedure described for example 14 was followed to afford the title compound (0.252g, 46.41%, purity by HPLC 98.82%). M.p.: 230 - 235°C. $^1$H-NMR (DMSO-$d_6$) $\delta$: 1.87 (s, 3H), 3.17 (s, 3H), 7.51 - 7.53 (m, 2H), 7.66-7.67 (m, 2H), 7.8 - 7.82 (d, 2H), 7.9 (s, 1H), 7.99 (s, 1H), 12.14 (s, 1H, $D_2O$ exchangeable). IR(KBr) $cm^{-1}$: 3150, 1720, and 1525. MS m/z: 533.7($M^+$).

## Example 18

**Synthesis of 6-[4-(methylsulfonyl)phenyl]-5-phenyl-2-(trifluoromethyl) pyrimidin-4-yl napthalenesulfonate**

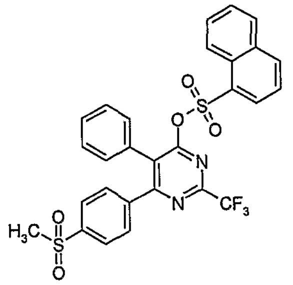

To a solution of 6-[4-(methylsulfonyl)phenyl]-5-phenyl-2-(trifluoromethyl)pyrimidin-4-ol (0.1g, 0.25 mmol, prepared according to the procedure described in PCT/IB03/01289) in dichloromethane (10 ml) was added naphthalene-1-sulfonyl chloride (0.086 g, 0.37 mmol) in dichloromethane (4ml) at 0°C and the mixture was stirred for 10 minutes. Subsequently pyridine (0.04 ml, 0.5 mmol) was added under stirring and the stirring was continued further for 6 hours at 37°C. Dichloromethane was removed under vacuo and the resultant mixture was diluted with water:ethyl acetate (1:1, 100 ml) and then extracted with ethyl acetate (25 ml x 3). The organic layer was washed with brine (100ml), dried over anhydrous sodium sulphate, and evaporated to afford the title compound (0.096 g, yield 67%), m.p. 204-206°C. $^1$H-NMR ($CDCl_3$) $\delta$: 2.99 (s, 3H), 7.12 - 7.14 (d, 2H), 7.26-7.29 (dd, 3H), 7.37 - 7.39 (m, 2H), 7.48 - 7.50 (m, 2H), 7.61 - 7.64 (m, 3H), 7.77 - 7.79(d, 2H), 7.84 - 7.86 (d, 2H), 7.94 - 7.96 (d, 2H). MS m/z: 585.1 ($M^+$+1).

**The following compounds were prepared according to the procedure described for Example 18**

| Ex. | Structure | Analytical Data |
|---|---|---|
| **19** | 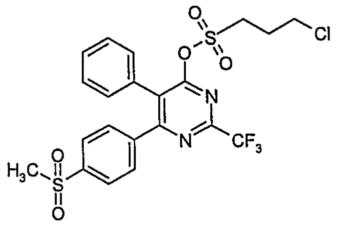 m.p: 202 - 204°C. | $^1$H-NMR ($CDCl_3$) $\delta$: 2.42 - 2.45 (m, 2H), 2.99 (s, 3H), 3.68 - 3.71 (t, 2H), 3.97 - 4.01 (t, 2H), 7.19-7.86 (m, 9H). MS m/z: 535.5 ($M^+$+1). |
| **20** | 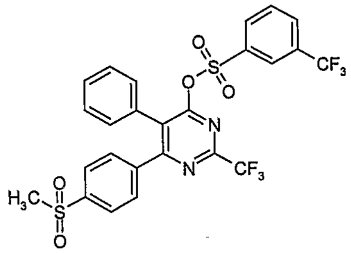 m.p: 174 - 176°C. | $^1$H-NMR ($CDCl_3$) $\delta$: 3.02 (s, 3H), 7.18 - 7.20 (d, 2H), 7.21 - 7.26 (dd, 3H), 7.41 - 7.43 (m, 2H), 7.55 - 7.57 (dd, 1H), 7.76 - 7.81 (dd, 2H), 7.97-8.44 (m, 3H). MS m/z: 603.54 ($M^+$+1). |
| **21** | 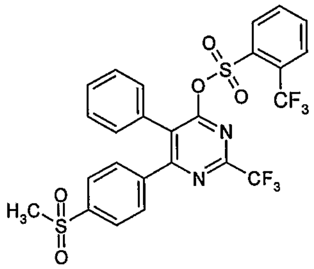 m.p: 174 - 176°C. | $^1$H-NMR ($CDCl_3$) $\delta$: 3.02 (s, 3H), 7.17 - 7.20 (d, 2H), 7.21 - 7.26 (dd, 3H), 7.40 - 7.43 (m, 2H), 7.81 - 7.83 (m, 4H), 7.84 - 7.86 (dd, 1H), 8.41 - 8.43 (dd, 1H). MS m/z: 603.51 ($M^+$+1). |
| **22** | 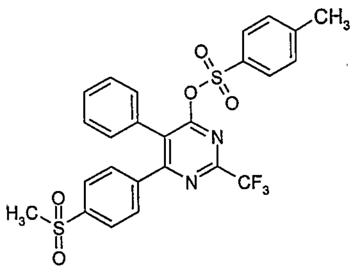 m.p: 200 - 206°C. | $^1$H-NMR ($CDCl_3$) $\delta$: 2.47 (s, 3H), 3.02 (s, 3H), 7.17 - 7.20 (d, 2H), 7.21 - 7.26 (dd, 3H), 7.40 - 7.43 (m, 2H), 7.81 - 7.83 (m, 4H), 7.84 - 7.86 (dd, 1H), 8.41 - 8.43 (dd, 1H). MS m/z: 603.51 ($M^+$+1). |
| **23** | 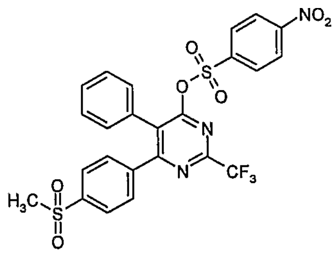 m.p: 205 - 208°C. | $^1$H-NMR ($CDCl_3$) $\delta$: 3.02 (s, 3H), 7.18 - 7.20 (d, 2H), 7.21 - 7.26 (dd, 3H), 7.41 - 7.43 (m, 2H), 7.55 - 7.57 (dd, 1H), 7.76 - 7.81 (dd, 2H), 7.97 - 8.44 (m, 3H). MS m/z: 603.54 ($M^+$+1). |

(continued)

| Ex. | Structure | Analytical Data |
|---|---|---|
| **24** | 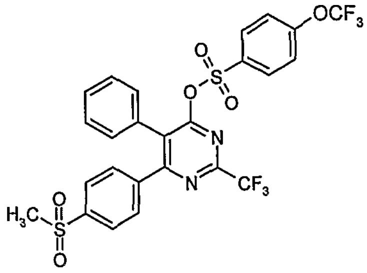 m.p: 165 - 168°C. | $^1$H-NMR ($CDCl_3$) $\delta$: 3.00 (s, 3H), 7.17 - 7.20 (d, 2H), 7.21 - 7.26 (dd, 3H), 7.40 - 7.43 (m, 2H), 7.81 - 7.80 (m, 4H), 7.84 - 7.87 (dd, 1H), 8.40 - 8.42 (dd, 1H). MS m/z: 603.51 ($M^+$+1). |
| **25** | 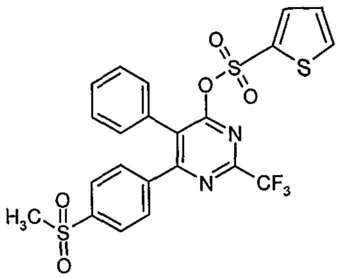 m.p: 184 - 188°C. | $^1$H-NMR ($CDCl_3$) $\delta$: 3.03 (s, 3H), 7.17 - 7.20 (d, 2H), 7.21 - 7.26 (dd, 3H), 7.81 - 7.83 (m, 4H), 7.84 - 7.86 (dd, 1H), 8.41 - 8.43 (dd, 1H). MS m/z: 603.51 ($M^+$+1). |
| **26** | 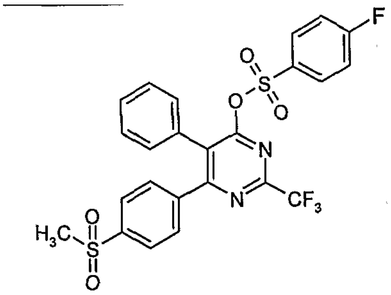 m.p: 193 - 195°C. | $^1$H-NMR ($CDCl_3$) $\delta$: 3.01 (s, 3H), 7.17 - 7.20 (d, 2H), 7.21 - 7.24 (dd, 3H), 7.40 - 7.43 (m, 2H), 7.81 - 7.83 (m, 4H), 7.84 - 7.86 (dd, 1H), 8.41 - 8.43 (dd, 1H). MS m/z: 603.51 ($M^+$+1). |
| **27** | 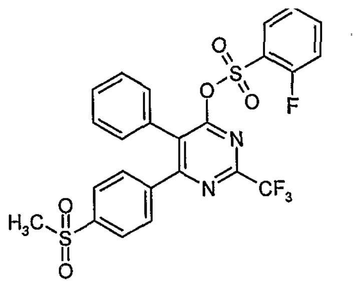 m.p: 200 - 204°C. | $^1$H-NMR ($CDCl_3$) $\delta$: 3.01 (s, 3H), 7.17 - 7.20 (d, 2H), 7.21 - 7.24 (dd, 3H), 7.40 - 7.43 (m, 2H), 7.81 - 7.83 (m, 4H), 7.84 - 7.86 (dd, 1H), 8.41 - 8.43 (dd, 1H). MS m/z: 603.51 ($M^+$+1). |
| **28** | 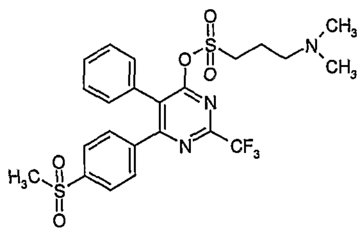 Sticky material | $^1$H-NMR ($CDCl_3$) $\delta$: 0.88 - 0.89 (m, 2H), 1.25 - 1.28 (t, 2H), 1.33-1.38 (t, 2H), 2.85 (s, 6H), 2.97 (s, 3H), 7.04 - 7.74 (m, 9H). MS m/z: 544.59 ($M^+$+1). |

**Example 29**

**Synthesis of 6-[4-(methylsulfonyl)phenyl]-5-phenyl-4-(N-benzyl-piperazin-1-yl)-2-(trifluoromethyl)pyrimidine**

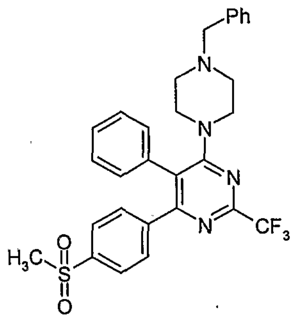

To a suspension of $Pd_2(dba)_3$(0.443 g, 0.484 mmol) in toluene (50ml) was added racemic-2,2'-*bis*(diphenyl phosphino)-1,1'-dinaphthyl (0.151 g, 0.242 mmol) at 37°C under stirring. After 10 minutes of stirring the resulting solution was added to a suspension of N-benzyl piperazine (2.51 ml, 14.55 mmol), 4-chloro-6-[4-(methylsulfonyl)phenyl]-5-phenyl-2-(trifluoromethyl) pyrimidine (5 g, 12.12 mmol, prepared according to the procedure described in PCT/IB03/02879) and cesium carbonate (5.53 g, 16.9 mmol) in toluene (80 ml) under stirring. Subsequently the reaction mixture was refluxed for 6 hours and filtered. The solid obtained was washed thoroughly with ethyl acetate and the resultant organic layer was washed successively with water (100 ml x 3), brine (200 ml) then dried over anhydrous sodium sulphate and evaporated to afford the title compound (5.5 g, yield 82.21%).

**Preparation 2**

**Method A:**

**Synthesis of 6-[4-(methylsulfonyl)phenyl]-5-phenyl-4-piperazin-1-yl-2-(trifluoromethyl)pyrimidine**

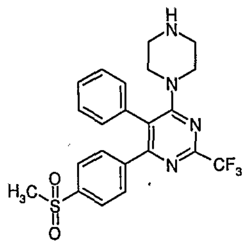

To a solution of 6-[4-(methylsulfonyl)phenyl]-5-phenyl-4-(N-benzyl-piperazin-1-yl)-2-(trifluoromethyl)pyrimidine (5g, 9mmol) in dry dichloromethane (20 ml) was added diisopropyl ethylamine (2.5 ml, 18 mmol) under stirring at 0°C. 1-chloro-ethyl chloroformate (1.35 ml, 13.5 mmol) was later added to the above, under stirring, and the stirring was further continued at 37°C for 5 hours. Subsequently dichloromethane was evaporated upto dryness and methanol (20 ml) was added dropwise to the resulting mass, which was refluxed for 3 hours at 60°C. The reaction mixture was poured onto ice and filtered; the solid obtained was washed with hexane (100 ml) and diisopropyl ether (50ml) to give the title compound (4 g, yield 95.9 %).

**Method B:**

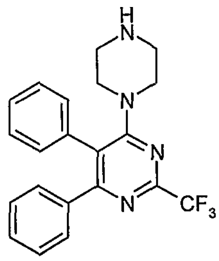

Piperazine (4.12g, 47.8mmol) was added to a solution of 4-chloro-5,6-diphenyl-2-trifluoromethylpyrimidine (4.0g, 11.95 mmol, prepared according to the procedure described in the PCT/IB03/02879) in acetonitrile (30ml) under stirring at 37°C. TLC confirmed the completion of the reaction after 2 hours, and then the reaction mixture was poured onto ice and extracted with ethyl acetate (250 ml). The organic layer was dried over anhydrous sodium sulphate and evaporated to afford the title compound (3.5g, yield 76.25%, purity by HPLC 100%). M.p: 160-162°C. $^1$H-NMR (DMSO-$d_6$) $\delta$: 2.5 (s, 4H), 3.16 (t, 4H), 7.07 - 7.08 (m, 2H), 7.17 - 7.31 (m, 9H, 1H, $D_2O$ exchangeable). IR $cm^{-1}$ (KBr): 3304, 3058, and 1559. MS m/z: 385.2 ($M^+$+1).

**The following compounds were prepared according to the procedure described for the preparation 2**

| Ex. | Structure | Analytical Data |
|---|---|---|
| **30** | 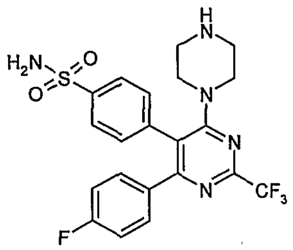 m.p:>285°C. | $^1$H-NMR (DMSO-$d_6$) $\delta$: 2.98 (s, 4H), 3.35 - 3.42 (m, 4H), 7.36 - 7.38 (m, 1H), 7.44 - 7.48 (m, 2H), 7.52 - 7.56 (m, 1H), 7.77 - 7.79 (m, 2H), 9.24 (s, 2H, $D_2O$ exchangeable). MS m/z: 482.1 ($M^+$+1). |
| **31** | 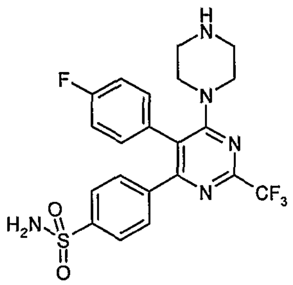 m.p: >285°C. | $^1$H-NMR (DMSO-$d_6$) $\delta$: 3.01(s, 4H), 3.42 (s, 4H), 7.1 - 7.11 (m, 2H), 7.26 - 7.32 (m, 3H), 7.38 - 7.41 (m, 2H), 7.75 - 7.77 (s, 3H), 9.0 (s, 2H, $D_2O$ exchangeable). MS m/z: 482.1 ($M^+$+1). |
| **32** | 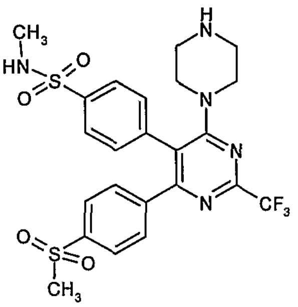 m.p: 245 - 247°C. | $^1$H-NMR (DMSO-$d_6$) $\delta$: 2.09 (s, 4H), 2.7 (s, 3H), 3.15 (s, 3H), 3.22 - 3.32 (d, 4H), 7.35 - 7.37 (m, 2H), 7.51 - 7.55 (m, 3H), 7. 62 - 7.64 (m, 1H), 7.70 - 7.79 (m, 2H). MS m/z: 556.1 ($M^+$+1). |

(continued)

| Ex. | Structure | Analytical Data |
|---|---|---|
| **33** | 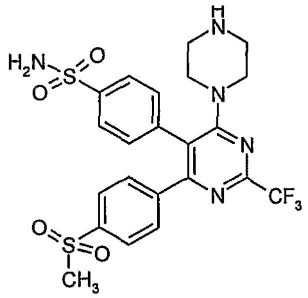 m.p: 230 - 235°C. | $^1$H-NMR ($CDCl_3$) δ: 2.15(s, 3H), 3.02 (s, 4H), 3.47 (s, 4H), 4.5 (s, 1H, $D_2O$ exchangeable), 7.26 (s, 2H), 7.32 - 7.36 (m, 3H), 7.47 - 7.51 (m, 2H), 7.77 - 7.79 (m, 4H). MS m/z: 542.1($M^+$+1). |
| **34** | 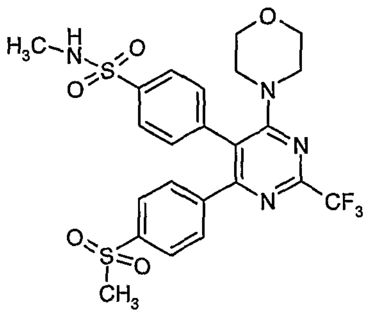 m.p: 175 - 177°C. | $^1$H-NMR ($CDCl_3$) δ: 2.4 - 2.41 (d, 3H), 3.0 (s, 3H), 3.34 - 3.37 (m, 4H), 3.58 - 3.6 (m, 4H), 4.1 - 4.3 (m, 1H, $D_2O$ exchangeable), 7.26 - 7.31 (m, 2H), 7.46 (s, 2H), 7.54 - 7.58 (m, 1H), 7.78 - 7.8 (m, 3H). IR $cm^{-1}$ (KBr): 3205, 2886, 2856, 1693, and 1557. MS m/z: 557.1 ($M^+$). |
| **35** | 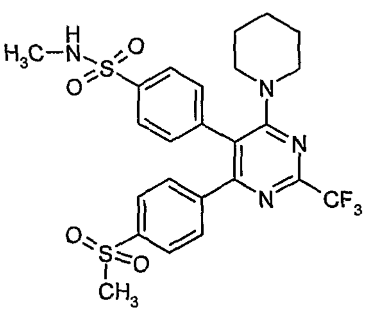 m.p: 204 - 206°C. | $^1$H-NMR ($CDCl_3$) δ: 1.19 - 1.22 (m, 4H), 1.56 - 1.62 (m, 2H), 2.39 - 2.41 (d, 3H), 3.0 (s, 3H), 3.29 - 3.31 (m, 4H), 4.13 - 4.15 (m, 1H, $D_2O$ exchangeable), 7.26 - 7.29 (m, 2H), 7.442 - 7.446 (m, 2H), 7.51 - 7.54 (m, 1H), 7.74 - 7.78 (m, 3H). IR $cm^{-1}$ (KBr): 3280, 2938, 2851, 1561, and 1525. MS m/z: 555 ($M^+$). |

<u>**Preparation 3**</u>

**Preparation of 6-[4-(methylsulfonyl)phenyl]-5-phenyl-4-[4-(2-thienyl carbonyl)piperazin-1-yl]-2-(trifluoromethyl)pyrimidine**

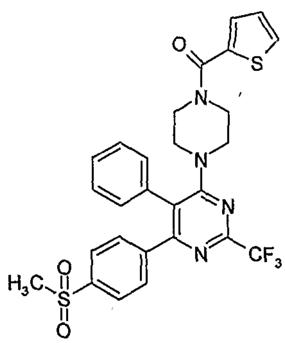

To a solution of 6-[4-(methylsulfonyl)phenyl]-5-phenyl-4-piperazin-1-yl-2-(trifluoromethyl)pyrimidine (0.2 g, 0.43 mmol, prepared according to the procedure described in preparation 2) in dimethylformamide (20 ml) was added thiophene-2-carboxylic acid (0.149 g, 0.78 mmol) under stirring at 37°C, and then after 10 minutes EDCI (0.149 g, 0.78 mmol), and HOBt (0.023 g, 0.173 mmol) were added. Further TEA (0.179 ml, 2.9 mmol) was added to the resultant clear solution and it was stirred for 18 hours. Subsequently the reaction mixture was diluted with ethyl acetate (50 ml) and the organic layer was washed successively with water, 10% sodium bi carbonate solution (100 ml), and brine (100 ml) respectively and dried over anhydrous sodium sulphate. Evaporation of the solvent and purification by column chromatography (0.4 % MeOH:DCM) gave the title compound (0.12g, yield 48.6 %), m.p. 189-194°C. $^1H$- NMR ($CDCl_3$) δ: 2.98 (s, 3H), 3.4 - 3.41 (t, 4H), 3.66 - 3.68 (t, 4H), 7.02 - 7.77 (m, 12H). MS m/z: 573.2 ($M^+$+1).

**The following compounds were prepared according to the procedure described for the preparation 3**

| Ex. | Structure | Analytical data |
|---|---|---|
| **36** | 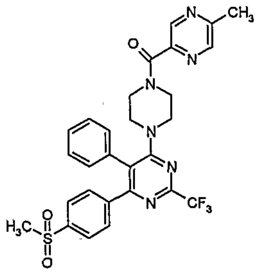 m.p: 163 - 168°C. | $^1H$-NMR ($CDCl_3$) δ: 2.67 (s, 3H), 2.98 (s, 3H), 3.43 - 3.45 (t, 4H), 3.63 - 3.64 (t, 2H), 3.68 - 3.7 (t, 2H), 7.11 - 8.84 (m, 11H). MS m/z: 583.2 ($M^+$+1). |
| **37** | 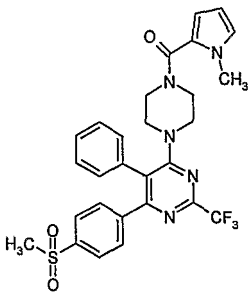 m.p: 235 - 238°C. | $^1H$-NMR ($CDCl_3$) δ: 2.98 (s, 3H), 3.37 - 3.39 (t, 4H), 3.63 - 3.67 (t, 4H), 3.75 (s, 3H), 6.95 - 7.77 (m, 11H). MS m/z: 570.63 ($M^+$+1). |
| **38** | 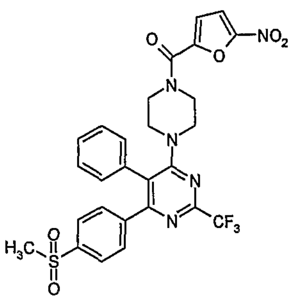 m.p: 158-161°C. | $^1H$-NMR ($CDCl_3$) δ: 2.99 (s, 3H), 3.49 - 3.51 (t, 4H), 3.82 - 3.83 (t, 4H), 7.13 - 7.18 (m, 3H), 7.26-7.34 (m, 6H), 7.76 - 7.78 (dd, 2H). MS m/z: 602.1 ($M^+$+1). |

(continued)

| Ex. | Structure | Analytical data |
|---|---|---|
| **39** | 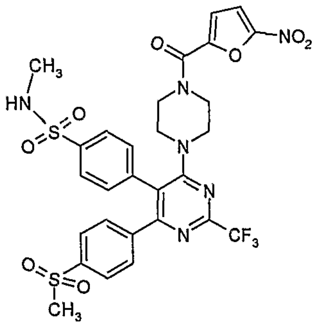 m.p: 114 - 118 °C. | $^1$H-NMR ($CDCl_3$) $\delta$: 2.43 - 2.44 (d, 3H), 3.01 (s, 3H), 3.47 (s, 4H), 3.71 - 3.85 (d, 4H), 4.39 (s, 1H, $D_2O$ exchangeable), 7.19 (s, 1H), 7.26-7.33 (m, 3H), 7.43 - 7.45 (m, 1H), 7.54 - 7.59 (m, 2H), 7.79 - 7.81 (d, 3H). MS m/z: 695.1 ($M^+$+1). |
| 40 | 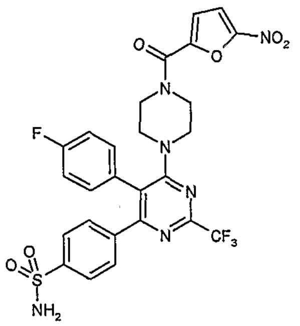 5H), m.p: 250 - 253°C. | $^1$H-NMR (DMSO-$d_6$) $\delta$: 3.35 (s, 4H), 3.54 - 3.67 (d, 4H), 7.06 - 7.16 (m, 5H), 7.23 - 7.24 (d, 1H), 7.35 - 7.73 (m, 3H), 7.74 - 7.78 (m, 3H). MS m/z: 621.2 ($M^+$+1). |
| **41** | 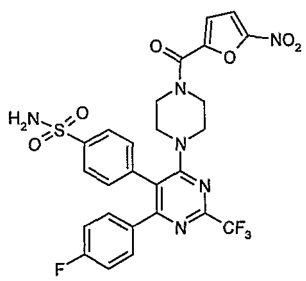 m.p: 225 - 227°C. | $^1$H-NNM (DMSO-$d_6$) $\delta$: 3.36 (s, 4H), 3.33 - 3.67 (d, 4H), 7.13 - 7.21 (m, 4H), 7.26 - 7.4 (m, 4H), 7.72 - 7.74 (d, 4H). MS m/z: 621.2 ($M^+$+1). |
| **42** | 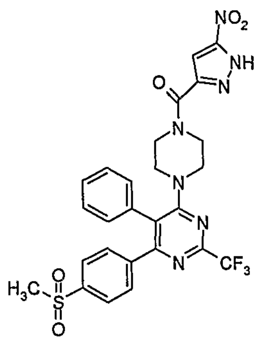 m.p: 127 - 132°C. | $^1$H-NMR ($CDCl_3$) $\delta$: 2.99 (s, 3H), 3.48 - 3.51 (t, 4H), 3.87 - 3.88 (t, 4H), 7.12 - 7.13 (m, 1H), 7.14 - 7.15 (t, 2H), 7.26 - 7.28 (m, 5H), 7.76 - 7.78 (dd, 2H). MS m/z: 602.3 ($M^+$+1). |

(continued)

| Ex. | Structure | Analytical data |
|---|---|---|
| 43 | 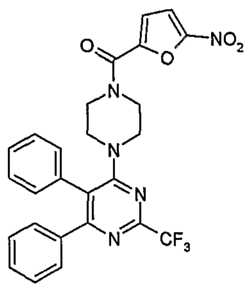 m.p: 230 - 233°C. | $^1$H-NMR ($CDCl_3$) $\delta$: 3.37 - 3.45 (d, 4H), 3.63 - 3.81 (d, 4H), 7.13 - 7.26 (m, 8H), 7.30 - 7.33 (m, 4H). MS m/z: 524.1 ($M^+$+1). |
| 44 | 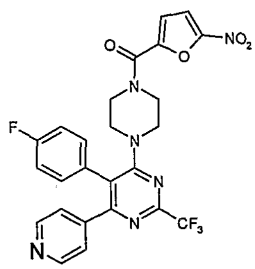 m.p: 185 - 187°C. | $^1$H-NMR ($CDCl_3$) $\delta$: 3.43 - 3.51 (d, 4H), 3.68 - 3.87 (d, 4H), 7.02 - 7.15 (m, 7H), 7.34 - 7.35 (d, 1H), 8.49-8.5 (d, 2H). IR (KBr) $cm^{-1}$: 3428, 1633. MS m/z: 543.1 ($M^+$+1). |

Example 45

**Synthesis of 6-[4-(methylsulfonyl)phenyl]-5-phenyl-4-[4-(1,3-thiazol-2-yl methyl)piperazin-1-yl]-2-(trifluoromethyl)pyrimidine**

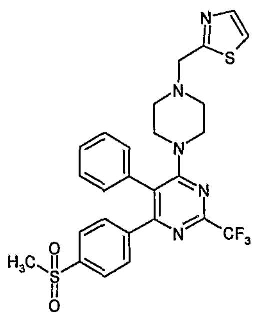

To a solution of 6-[4-(methylsulfonyl)phenyl]-5-phenyl-4-piperazin-1-yl-2-(trifluoromethyl)pyrimidine (0.2 g, 0.43 mmol, prepared according to the procedure described in preparation 2) in dichloroethane (25 ml) was added thiazole-2-carboxaldehyde (0.144 g, 1.3 mmol) under stirring at 37°C. After five minutes, sodium triacetoxy borohydride (0.364 g, 1.72 mmol) was added to reaction mixture and then after 10 minutes, acetic acid (0.1 ml) was added to it. The reaction mixture was stirred for 36 hours under the same conditions. Subsequently the reaction mixture was treated with ethyl acetate:water (1:1, 100 ml) and extracted with ethyl acetate (50 ml x 3). The organic layer was washed with brine (100 ml) and dried over anhydrous sodium sulphate. The solid obtained upon evaporation was purified by column chromatography using methanol:dichloromethane (0.5:99.5) as an eluent to afford the title compound (0.11g, yield 45.6 %). $^1$H-NMR ($CDCl_3$) $\delta$: 0.88 - 0.89 (m, 2H), 1.25 - 1.28 (t, 2H), 1.33 - 1.38 (t, 2H), 2.85 (s, 6H), 2.97 (s, 3H), 7.04 - 7.74 (m, 9H). MS m/z: 544.59 ($M^+$+1).

**The following compounds were prepared according to the procedure described for example 45**

| Ex. | Structure | Analytical Data |
|---|---|---|
| **46** | 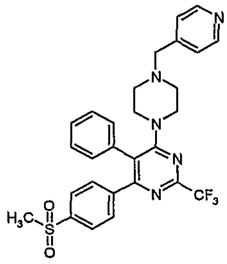 m.p: 228 - 232°C | $^1$H-NMR ($CDCl_3$) δ: 2.50 - 2.53 (t, 4H), 2.98 (s, 3H), 3.44 - 3.46 (t, 4H), 3.88 (s, 2H), 7.14 - 7.78 (m, 13H). MS m/z: 554.3 ($M^+$+1). |
| **47** | 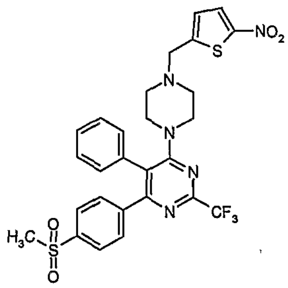 m.p: 168 - 174°C | $^1$H-NMR ($CDCl_3$) δ: 2.51 - 2.52 (t, 4H), 2.99 (s, 3H), 3.44 - 3.46 (t, 4H), 3.88 (s, 2H), 7.10 - 7.81 (m, 11H). MS m/z: 604.7 ($M^+$+1). |

**Example 48**

**Synthesis of 4,5-diphenyl-6-(4-pyridin-2-yl-piperazin-2-yl)-2-(trifluoro methyl)pyrimidine.**

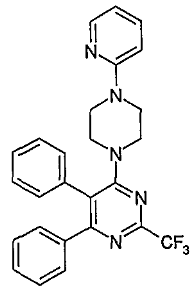

To a solution of 4-chloro-5,6-diphenyl-2-(trifluoromethyl)pyrimidine (0.5g, 1.49mmol, prepared according to the procedure described in PCT/IB03/02879) in dimethylformamide (5 ml) was added 1-pyridin-2-yl piperazine (0.107g, 0.66mmol) and anhydrous potassium carbonate (0.2g) under stirring at 28°C. The reaction mixture was stirred for 4 hours, and when TLC confirmed the completion, it was poured onto ice. The solid thus obtained was filtered and washed with water (20ml)). The above solid was then dissolved in dichloromethane (50ml) and dried over anhydrous sodium sulphate, evaporation of the solvent afforded the title compound (0.48g, yield 69.66%, purity by HPLC 99.8%). M.p. 165-167°C. $^1$H-NMR ($CDCl_3$) δ: 3.43 (s, 8H), 6.58 - 6.64 (m, 2H), 7.14 - 7.21 (m, 7H), 7.26 - 7.29 (m, 3H), 7.45 - 7.49 (t, 1H), 8.14 - 8.15 (d, 1H). IR $cm^{-1}$ (KBr): 3436, 2839, 1591, and 1557. MS m/z: 462.1 ($M^+$+1).

**Similar to the above the following compound was prepared**

| Ex. | Structure | Analytical Data |
|---|---|---|
| 49 | 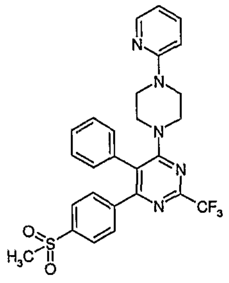 m.p.: 229 - 233°C | $^1$H-NMR (DMSO-$d_6$) δ: 3.18-3.21 (d, 3H), 3.17-3.4(d, 8H), 6.63-6.66 (t, 1H), 6.76-6.78 (d, 1H), 7.27-7.28 (d, 2H), 7.35-7.37 (d, 5H), 7.49-7.53 (t, 1H), 7.76-7.78 (d, 2H), 8.07-8.08 (d, 1H). MS m/z: 540.4 ($M^+$+1). |

Preparation 4

**Preparation of 3-[4-(4-fluorophenyl)-6-piperazin-1-yl-2-(trifluoromethyl) pyrimidin-5-yl]benzenesulfonamide hydrochloride (Hydrochloride of the example-50) and 4-[4-(4-fluorophenyl)-6-piperazin-1-yl-2-(trifluoromethyl)pyrimidin-5-yl]benzenesulfonamide hydrochloride (example-30).**

Step1:

**Preparation of 3-[4-chloro-6-(4-fluorophenyl)-2-(trifluoromethyl) pyrimidin-5-yl]benzenesulfonyl chloride and 4-[4-chloro-6-(4-fluorophenyl)-2-(trifluoromethyl)pyrimidin-5-yl]benzenesulfonyl chloride**

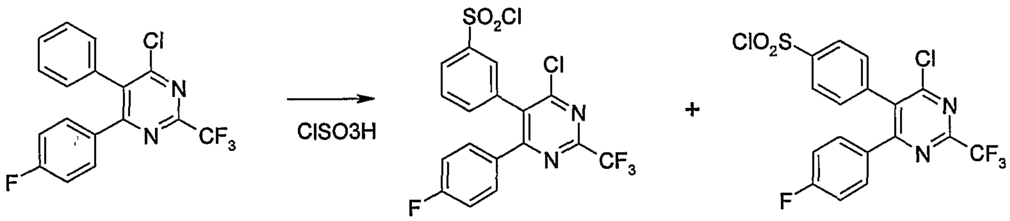


4-Chloro-6-(4-fluorophenyl)-5-phenyl-2-(trifluoromethyl)pyrimidine (30g, 0.085mol) was added to cold chlorosulfonic acid (200mL, 3.0mol) and the reaction mixture was stirred for 48 hours at room temperature. Subsequently it was poured into crushed ice (~3 kg) and was extracted with dichloromethane (1000mL). The organic layer was washed with sodium bicarbonate (7%, 1000mL), and with brine solution (500mL). The crude material obtained after evaporation was recrystallised from hexane-EtOAc to obtain the pure *para* and *meta* substituted sulphonyl chlorides. However, the crude product containing mixture of *meta* and *para* isomers was used as such in the examples mentioned below unless otherwise mentioned.

**3-[4-Chloro-6-(4-fluorophenyl)-2-(trifluoromethyl)pyrimidin-5-yl]benzenesulfonyl chloride:** $^1$H-NMR ($CDCl_3$) δ : 6.97 - 7.01 (m, 2H), 7.32 - 7.36 (m, 2H), 7.63 - 7.65 (d, 1H), 7.71 - 7.74 (t, 1H), 7.90 - 7.91 (s, 1H), 8.10 - 8.12 (d, 1H).

**4-[4-Chloro-6-(4-fluorophenyl)-2-(trifluoromethyl)pyrimidin-5-yl]benzenesulfonyl chloride:** $^1$H-NMR ($CDCl_3$) δ : 6.97 - 7.01 (m, 2H), 7.34 - 7.38 (m, 2H), 7.50 - 7.53 (d, 2H), 8.11 - 8.13 (d, 2H).

Step 2a:

**Synthesis of 3-[4-chloro-6-(4-fluorophenyl)-2-(trifluoromethyl) pyrimidin-5-yl]benzenesulfonamide.**

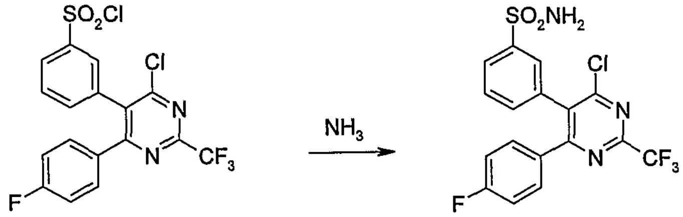


The sulphonyl chloride group is converted into the sulphonamido group by treatment with ammonia gas under cold conditions (0-5°C) by dissolving the substance in dichloromethane. Subsequently the reaction mixture was washed with water (100mL) and then with brine solution; evaporation of the solvent furnished the titled compound. $^1$H-NMR (DMSO-$d_6$) δ: 7.14 - 7.18 (m, 2H), 7.38 - 7.42 (m, 2H), 7.48 (brs, 2H, $D_2O$ exchangeable), 7.56 (d, 1H), 7.62 - 7.66 (t, 1H), 7.86 (s, 1H), 7.88 (d, 1H); MS m/z: 433 ($M^+$+1).

**Step 2b:**

**Preparation of 4-[4-chloro-6-(4-fluorophenyl)-2-(trifluoromethyl) pyrimidin-5-yl]benzenesulfonamide.**

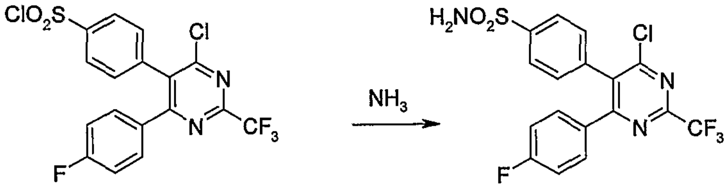


Similarly 4-[6-chloro-5-phenyl-2-(trifluoromethyl)pyrimidin-4-yl]benzenesulfonamide was prepared from 4-chloro-5,6-diphenyl-2-(trifluoromethyl)pyrimidine which in turn was prepared according to the procedure described in PCT/IB03/02879). $^1$H-NMR (DMSO-$d_6$) δ: 7.16 - 7.21 (m, 2H), 7.38 - 7.41 (m, 2H), 7.48 (brs, 2H, $D_2O$ exchangeable), 7.57 (d, 2H), 7.86 (d, 2H). MS m/z: 432 ($M^+$).

**Example 50**

**Synthesis of 3-[4-(4-fluorophenyl)-6-piperazin-l-yl-2-(trifluoromethyl) pyrimidin-5-yl]benzenesulfonamide.**

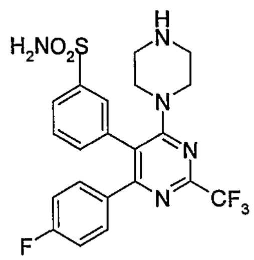

The solution of 3-[4-chloro-6-(4-fluorophenyl)-2-(trifluoromethyl) pyrimidin-5-yl]benzenesulfonamide (0.1g, 0.24mmol, prepared according to the procedure described above in preparation 4, Step 2a), in acetonitrile (2mL) was treated with piperazine (0.104g, 1.203mmol) and the reaction mixture was stirred overnight at room temperature. Subsequently the reaction mixture was poured into ice-cold water and was extracted with ethylacetate (25mL). The organic layer was washed with water, brine solution, and then evaporated to obtain the title compound. $^1$H-NMR (DMSO-$d_6$) δ: 2.58 (s, 4H), 3.19 (s, 4H), 7.03 - 7.14 (m, 4H), 7.32 (d, 1H), 7.41 (brs, $D_2O$ exchangeable, 2H), 7.46 - 7.50 (t, 1H), 7.72 (s, 1H), 7.73 (d, 1H). MS m/z: 482.1 ($M^+$+1).

**The following compounds were prepared according to the above procedure.**

| | | |
|---|---|---|
| **51** | 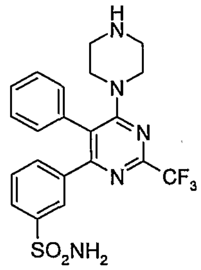 | $^1$H-NMR (DMSO-$d_6$) $\delta$: 2.52 - 2.54 (m, 4H), 3.18 (m, 4H), 7.04 (d, 1H), 7.26 - 7.33 (m, 2H), 7.41 - 7.48 (m, 4H), 7.73 (d, 1H), 7.75 (d, 1H), 7.81 (s, 1H). MS m/z: 464.1 ($M^+$). |
| **52** | 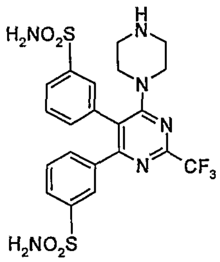 | $^1$H-NMR ($CDCl_3$) $\delta$: 2.56 (m, 4H), 3.18 (m, 4H), 7.02 - 7.09 (m, 1H), 7.31 - 7.33 (m, 2H), 7.40 - 7.48 (m, 5H), 7.72 - 7.75 (d, 3H), 7.81 (s, 1H). MS m/z: 543.0 ($M^+$+1). |
| **53** | 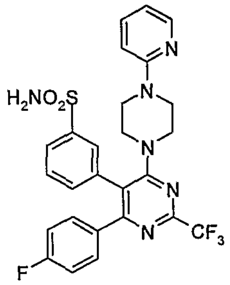 | $^1$H-NMR ($CDCl_3$) $\delta$: 3.44 (s, 8H), 6.61 - 6.66 (m, 2H), 6.90 (d, 2H), 7.09 (d, 2H), 7.31 - 7.35 (m, 2H), 7.47 - 7.51 (m, 2H), 7.76 (s, 1H), 7.85 - 7.90 (m, 1H), 8.17 (d, 2H). MS m/z: 559.1 ($M^+$+1). |
| **54** | 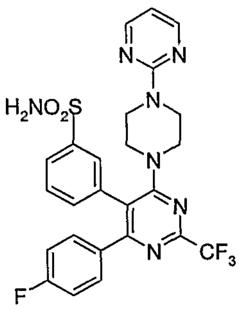 | $^1$H-NMR ($CDCl_3$) $\delta$: 3.38 - 3.41 (m, 4H), 3.71 - 3.74 (m, 4H), 6.61 - 6.66 (m, 1H), 6.90 (d, 2H), 7.09 (d, 2H), 7.31-7.35 (m, 1H), 7.47 - 7.51 (m, 1H), 7.76 (s, 1H), 7.85 - 7.90 (m, 1H), 8.29 (d, 2H); MS m/z: 560.1 ($M^+$+1). |
| **55** | 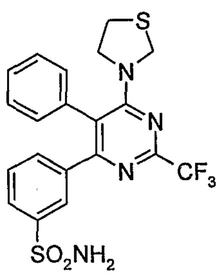 | $^1$H-NMR ($CDCl_3$) $\delta$: 2.94 - 2.97 (m, 2H), 3.60 - 3.63 (m, 2H), 4.25 (s, 2H), 7.06 - 7.08 (m, 2H), 7.18 - 7.21 (m, 2H), 7.41 - 7.47 (m, 3H), 7.61 (s, 1H), 7.82 (d, 1H). MS m/z: 467.0 ($M^+$+1). |

(continued)

| | | |
|---|---|---|
| **56** | 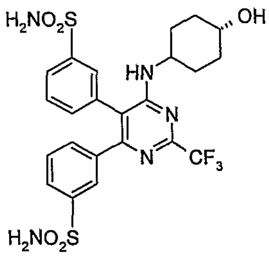 | $^1$H-NMR (DMSO-$d_6$) δ: 1.20 - 1.25 (m, 4H), 1.42 - 1.49 (m, 4H), 3.61 (m, 1H), 4.09 (m, 1H), 4.77 (br, 1H, $D_2O$ exchangeable), 5.14 (br, 1H, $D_2O$ exchangeable), 7.51 - 7.56 (m, 2H), 7.75 - 7.77 (d, 2H), 7.88 - 7.95 (m, 4H). MS m/z: 572.0 ($M^+$+1). |
| **57** | 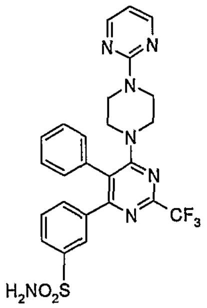 | $^1$H-NMR ($CDCl_3$) δ: 3.36 - 3.42 (m, 4H), 3.72 - 3.81 (m, 4H), 6.53 (d, 1H), 7.08 (d, 2H), 7.19 - 7.25 (m, 2H), 7.34 (d, 1H), 7.40 (d, 1H), 7.46 (d, 2H), 7.68 (s, 1H), 7.82 - 7.85 (t, 1H), 8.28 - 8.31 (m, 2H). MS m/z: 541.8 ($M^+$). |
| **58** | 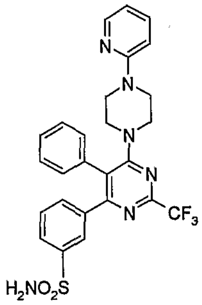 | $^1$H-NMR ($CDCl_3$) δ: 3.45 (m, 8H), 6.62 - 6.64 (m, 1H), 6.75 (d, 1H), 7.01 (d, 2H), 7.20 - 7.26 (m, 3H), 7.31 - 7.33 (m, 1H), 7.40 (br s, 2H), 7.48 - 7.53 (m, 2H), 7.74 (d, 1H), 7.78 (s, 1H), 8.05 (d, 1H). MS m/z: 540.9 ($M^+$). |
| **59** | 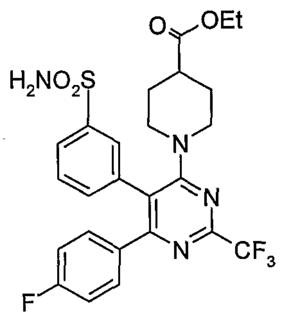 | $^1$H-NMR (DMSO-$d_6$) δ: 1.03 (t, 3H), 1.39 - 1.47 (m, 2H), 1.66 - 1.69 (m, 2H), 2.11 (m, 1H), 2.84 - 2.90 (m, 2H), 3.66 - 3.69 (m, 2H), 4.00 - 4.06 (q, 2H), 7.03 - 7.07 (m, 2H), 7.14 - 7.15 (m, 2H), 7.36 (br, 2H, $D_2O$ exchangeable), 7.37 - 7.39 (d, 1H), 7.49 - 7.53 (t, 1H), 7.69 (s, 1H), 7.73 - 7.75(d, 1H). MS m/z: 553.1 ($M^+$+1) |
| **60** | 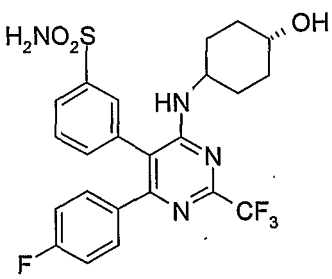 | $^1$H-NMR (DMSO-$d_6$) δ: 1.21 - 1.39(m, 4H), 1.79 (m, 4H), 3.3 - 3.35 (m, 1H), 3.94 (m, 1H), 4.53 - 4.54 (br, 1H, $D_2O$ exchangeable), 6.27 (br, 1H, $D_2O$ exchangeable), 7.01 - 7.06 (m, 2H), 7.19 - 7.23 (m, 2H) 7.33 (br, 2H, $D_2O$ ex), 7.40 (d, 1H), 7.53 - 7.61 (m, 2H), 7.79 (d, 1H). MS m/z: 511.0 ($M^+$+1). |

(continued)

| | | |
|---|---|---|
| **61** | 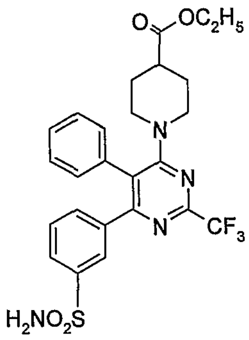 | $^1$H-NMR (DMSO-d$_6$) δ: 1.13 - 1.16 (t, 3H), 1.41 - 1.44 (m, 2H), 1.66 - 1.68 (m, 2H), 2.25 (m, 1H), 2.84 - 2.89 (m, 2H), 3.65 - 3.67 (m, 2H), 4.02 - 4.05 (q, 2H), 7.08 (d, 2H), 7.20 - 7.24 (m, 3H), 7.33 - 7.36 (m, 2H), 7.40 - 7.70 (m, 2H), 7.72 - 7.74 (m, 2H). MS m/z: 535.1 ($M^+$+1). |
| **62** | 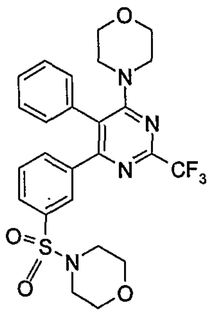 | $^1$H-NMR (DMSO-d$_6$) δ: 3.21 (m, 4H), 3.29 (m, 4H), 3.48 (m, 4H), 3.56 (m, 4H), 7.10 - 7.12 (m, 2H), 7.23 - 7.25 (m, 3H), 7.48 (s, 1H), 7.63 - 7.69 (m, 2H), 7.76 (d. 1H). MS m/z: 535.1 ($M^+$1). |
| **63** | 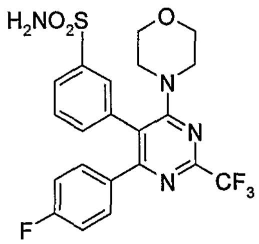 | $^1$H-NMR (DMSO-d$_6$) δ: 3.32 (m, 4H), 3.47 (m, 4H), 7.05 - 7.15 (m, 4H), 7.33 (d, 1H), 7.42 (brs, 1H, $D_2O$ exchangeable), 7.47-7.51 (t, 1H), 7.73 (d, 1H), 7.76 (s, 1H). MS m/z: 483 ($M^+$+1). |
| **64** | 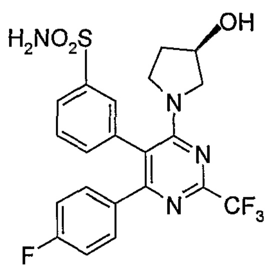 | $^1$H-NMR (DMSO-d$_6$) δ: 0.82 - 0.87 (m, 3H), 1.15 - 1.27 (m, 3H), 1.70 - 1.79 (m, 1H), 4.93 (brs, 1H, $D_2O$ exchangeable), 7.01 - 7.07 (m, 2H), 7.14 - 7.18 (m, 2H), 7.39 - 7.47 (m, 4H), 7.71 - 7.73 (m, 2H). MS m/z: 483 ($M^+$+1). |
| **65** | 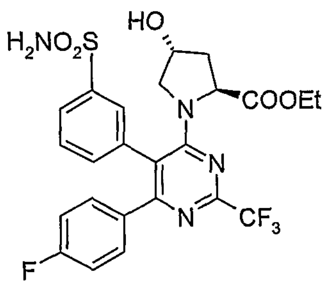 | $^1$H-NMR (DMSO-d$_6$) δ: 1.17 - 1.27 (t, 3H), 1.86 - 1.89 (m, 1H), 2.08 - 2.14 (m, 2H), 2.66 - 2.68 (m, 1H), 4.13 (q, 2H), 4.68 - 4.70 (m, 1H), 5.09 (br, 1H, $D_2O$ exchangeable), 7.03 - 7.09 (m, 2H), 7.18 - 7.21 (m, 2H), 7.25 - 7.28 (br, 2H, $D_2O$ exchangeable), 7.37 - 7.45 (m, 2H), 7.75 - 7.77 (m, 2H). MS m/z: 555.1 ($M^+$+1). |

(continued)

| | | |
|---|---|---|
| **66** | 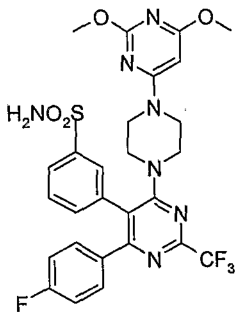 | $^1$H-NMR ($CDCl_3$) δ: 3.38 (s, 4H), 3.51 (s, 4H), 3.88 (s, 3H), 3.89 (s, 3H), 6.89 - 6.93 (m, 2H), 7.07 - 7.10 (m, 2H), 7.36 (d, 2H), 7.51 - 7.53 (m, 1H), 7.72 (s, 1H), 7.87 (d, 1H). MS m/z: 619.8 ($M^+$+1). |
| **67** | 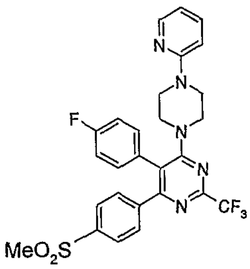 | $^1$H-NMR ($CDCl_3$) δ: 2.99 (s, 3H), 3.47 (s, 8H), 6.60 (d, 1H), 6.66 (t, 1H), 7.02 - 7.12 (m, 4H), 7.33 (d, 2H), 7.47 (t, 1H), 7.79 (d, 2H), 8.15 (d, 1H). MS m/z: 558.1 ($M^+$+1). |
| **68** | 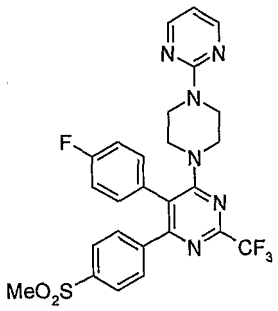 | $^1$H-NMR ($CDCl_3$) δ: 2.99 (s, 3H), 3.44 (t, 4H), 3.73 (t, 4H), 6.53 (t, 1H), 7.02 - 7.12 (m, 4H), 7.33 (d, 2H), 7.79 (d, 2H), 8.29 (d, 2H). MS m/z: 559.1 ($M^+$+1). |
| **69** | 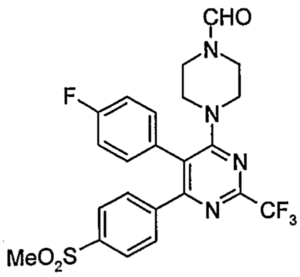 | $^1$H-NMR ($CDCl_3$) δ: 3.00 (s, 3H), 3.27 - 3.32 (m, 4H), 3.40 - 3.46 (m, 4H), 7.05 - 7.10 (m, 4H), 7.33 (d, 2H), 7.80 (m, 2H), 8.03 (s, 1H). MS m/z: 509.1 ($M^+$+1). |
| **70** | 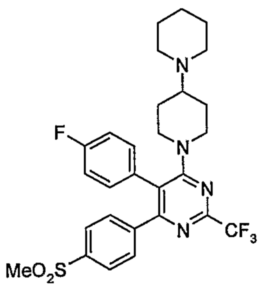 | $^1$H-NMR ($CDCl_3$) δ: 1.34 - 1.42 (m, 4H), 1.74 (d, 2H), 2.39 (t, 1H), 2.43 (s, 4H), 2.69 (t, 4H), 2.98 (s, 3H), 3.95 (d, 4H), 6.99 - 7.05 (m, 4H), 7.30 (d, 2H), 7.77 (d, 2H). MS m/z: 563.2 ($M^+$+1). |

(continued)

| | | |
|---|---|---|
| **71** | 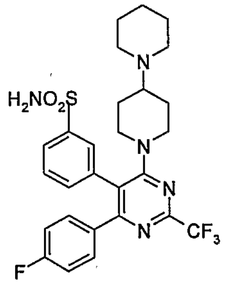 | $^1$H-NMR ($CDCl_3$) δ: 1.88 - 1.91 (m, 8H), 2.02 - 2.07 (m, 4H), 2.68 - 2.71 (m, 5H), 3.62 - 3.65 (m, 2H), 6.90 (t, 2H), 7.03 (d, 1H), 7.09 - 7.13 (m, 2H), 7.36 - 7.40 (t, 1H), 7.88 (d, 1H), 8.06 (d, 1H); MS m/z: 564.2 ($M^+$+1). |
| **72** | 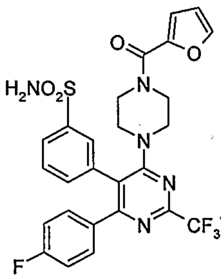 | $^1$H-NMR ($CDCl_3$) δ: 3.38 (s, 4H), 3.74 (s, 4H), 6.48 (s, 1H), 6.91 (t, 2H), 7.04 (d, 1H), 7.08 - 7.11(m, 2H), 7.33 (d, 2H), 7.47 - 7.50 (m, 2H), 7.77 (s, 1H), 7.92 (d, 1H). MS m/z: 576.1 ($M^+$+1). |
| **73** | 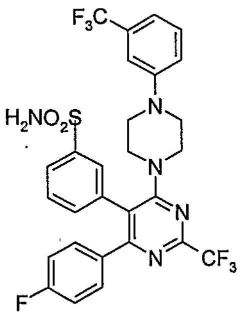 | $^1$H-NMR ($CDCl_3$) δ: 3.14 (d, 4H), 3.47 (s, 4H), 6.91 (t, 2H), 7.02 (d, 2H), 7.08 - 7.11 (m, 2H), 7.29 - 7.39 (m, 4H), 7.52 (t, 1H), 7.72 (s, 1H), 7.88 (d, 1H). MS m/z: 626.1 ($M^+$+1). |
| **74** | 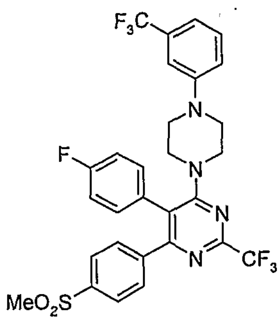 | $^1$H-NMR ($CDCl_3$) δ: 3.00 (s, 3H), 3.14 (d, 4H), 3.51 (d, 4H), 6.99 - 7.11 (m, 7H), 7.33 (d, 3H), 7.80 (d, 2H). HPLC (purity): 96.4 %. MS m/z: 625.1 ($M^+$+1). |
| **75** | 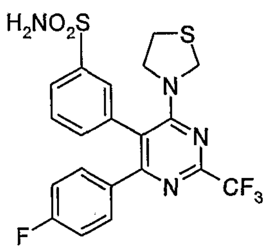 | $^1$H-NMR ($CDCl_3$) δ: 2.93 - 2.96 (m, 2H), 3.57 - 3.62 (m, 2H), 4.22 (m, 2H), 6.87 - 6.91 (m, 2H), 7.07 - 7.12 (m, 2H), 7.31 - 7.37 (m, 2H), 7.86 (d, 2H). HPLC (purity): 89 %. MS m/z: 485.0 ($M^+$+1). |

(continued)

| | | |
|---|---|---|
| **76** | 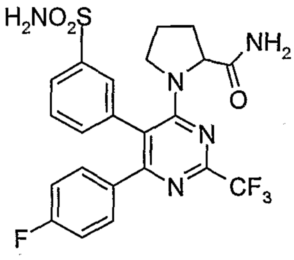 | $^1$H-NMR ($CDCl_3$) δ: 1.94 - 2.08 (m, 6H), 3.84 (d, 1H), 6.85 (t, 3H), 7.06 - 7.10 (m, 3H), 7.52 (bs, 2H), 7.84 (d, 2H). MS m/z: 510.1 ($m^+$+1). |
| **77** | 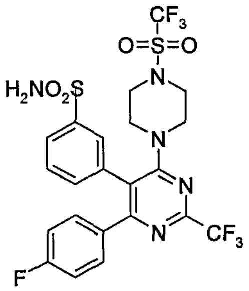 | $^1$H-NMR ($CDCl_3$) δ: 3.40 (s, 8H), 6.90 - 6.94 (m, 2H), 7.00 - 7.12 (m, 2H), 7.31 (d, 1H), 7.52 (t, 1H), 7.77 (s, 1H), 7.90 (d, 1H). MS m/z: 614.0 ($M^+$+1). |
| **78** | 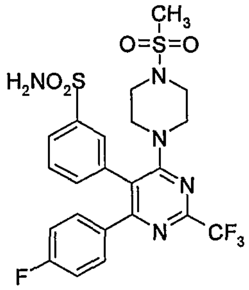 | $^1$H-NMR ($CDCl_3$) δ: 2.83 (s, 3H), 2.99 (s, 4H), 3.32 (s, 4H), 7.06 - 7.17 (m, 4H), 7.31 (d, 1H), 7.51 (t, 1H), 7.78 (d, 1H), 7.80 (s, 1H). MS m/z: 560.1 ($M^+$+1). |
| **79** | 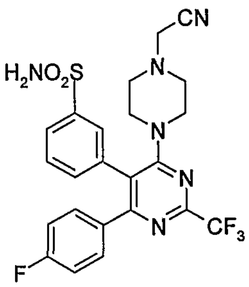 | $^1$H-NMR ($CDCl_3$) δ: 2.37 (d, 4H), 3.30 (d, 4H), 3.67 (s, 2H), 7.04 - 7.08 (m, 2H), 7.12 - 7.15 (m, 2H), 7.33 (d, 3H), 7.51 (t, 1H), 7.68 (s, 1H), 7.74 (d, 1H). MS m/z: 521.1 ($M^+$+1). |
| **80** | 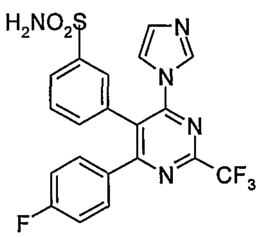 | $^1$H-NMR ($CDCl_3$) δ: 6.97 - 7.03 (m, 3H), 7.15 (s, 1H), 7.31 (d, 4H), 7.46 (s, 1H), 7.58 (t, 1H), 7.68 (s, 1H), 8.01 (d, 1H). MS m/z: 464.0 ($M^+$+1). |

(continued)

| | | |
|---|---|---|
| 81 | 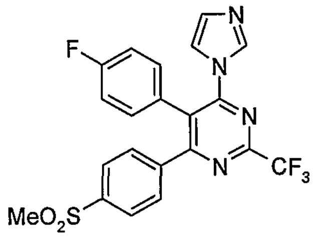 | $^1$H-NMR ($CDCl_3$) $\delta$: 3.04 (s, 3H), 7.03 (d, 2H), 7.09 - 7.15 (m, 4H), 7.50 (d, 2H), 7.73 (s, 1H), 7.88 (d, 2H); MS m/z: 463.0 ($M^+$+1). |

**Example 82**

**Synthesis of 3-[6-{4-[5-(trifluoromethyl)pyridin-2-yl]piperazin-1-yl}-5-phenyl-2-(trifluoromethyl)pyrimidin-4-yl]benzenesulfonamide.**

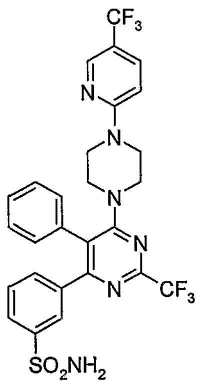

**Step 1:**

**Preparation of 1-[5-(trifluoromethyl)pyridin-2-yl]piperazine**

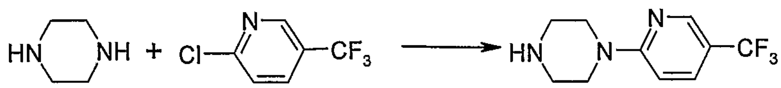

Piperazine (1.2g, 13.77mM) was heated with 2-chloro-5-(trifluoromethyl)pyridine (0.5g, 2.75mmol) in THF (2mL) for 2 hours. Subsequently the reaction mixture was poured onto crushed ice and extracted with ethyl acetate. The organic layer was washed with sodium bicarbonate and evaporated to furnish the required product.

**Step 2:**

**Synthesis of 3-[6-{4-[5-(trifluoromethyl)pyridin-2-yl]piperazin-1-yl}-5-phenyl-2-(trifluoromethyl)pyrimidin-4-yl]benzenesulfonamide.**

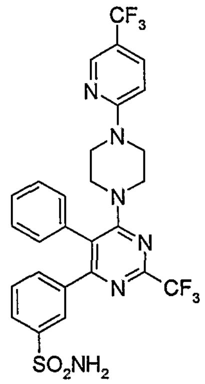

The solution of 3-[6-chloro-5-phenyl-2-(trifluoromethyl)pyrimidin-4-yl]benzenesulfonamide (0.1g, 0.242mM) in pyridine (2mL) was treated with 1-[5-(trifluoromethyl)pyridin-2-yl]piperazine (0.3g, 0.68mmol) and the reaction mixture was stirred for 1 hour at room temperature. Subsequently the reaction mixture was poured onto crushed ice containing two drops of concentrated hydrochloric acid, extracted with ethyl acetate (25mL), and the organic layer was washed with brine and evaporated. The title compound was obtained by column chromatographic purification of the crude material with 30% ethyl acetate in hexane. $^1$H-NMR (DMSO-$d_6$) δ: 3.33 - 3.37 (m, 4H), 3.56 (m, 4H), 6.86 - 6.88 (d, 1H), 7.10 - 7.12 (d, 2H), 7.22 - 7.29 (m, 3H), 7.35 - 7.37 (m, 1H), 7.44 - 7.48 (m, 1H), 7.74 - 7.79 (m, 3H), 8.38 (s, 1H). MS m/z: 608.8 ($M^+$).

**Example 83**

**Synthesis of 3-[6-{4-[2,6-dimethoxypyrimidin-4-yl]piperazin-1-yl}-5-phenyl-2-(trifluoromethyl)pyrimidin-4-yl]benzenesulfonamide.**

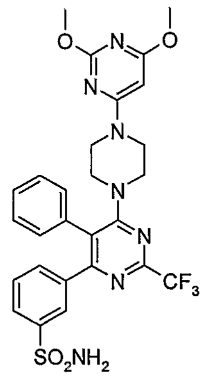

**Step 1:**

**Preparation of 2,4-dimethoxy-6-piperazin-1-ylpyrimidine.**

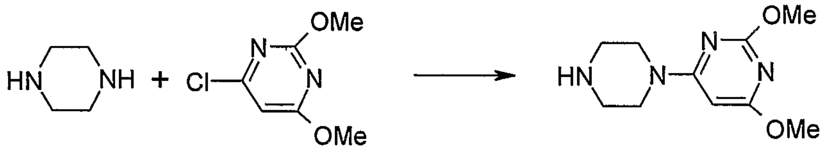

Piperazine (1.23g, 14.32mmol) was treated with 6-chloro-2,4-dimethoxy pyrimidine (0.5g, 2.86mmol) in acetonitrile (5mL) and stirred at room temperature for 6 hours. Subsequently the reaction mixture was poured onto ice-cold water (25mL) and extracted with ethyl acetate (25mL). The organic layer was washed with aqueous sodium bicarbonate

solution and evaporated to furnish the required compound.

**Step 2:**

**Synthesis of 3-[6-{4-[2,6-dimethoxypyrimidin-4-yl]piperazin-1-yl}-5-phenyl-2-(trifluoromethyl)pyrimidin-4-yl]benzenesulfonamide.**

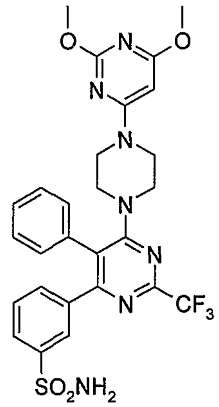

The solution of 3-[6-chloro-5-phenyl-2-(trifluoromethyl)pyrimidin-4-yl]benzenesulfonamide (0.1g, 0.242mmol) in pyridine (1.5mL) was treated with 2,4-dimethoxy-6-piperazin-1-ylpyrimidine (0.081g, 0.363mmol) and the reaction mixture was stirred for 8 hours. Subsequently the reaction mixture was poured onto ice-cold water and extracted with ethyl acetate (25mL). The organic layer was washed with brine and evaporated to furnish the title compound. $^1$H-NMR ($CDCl_3$) $\delta$: 3.39 - 3.41 (m, 4H), 3.51 - 3.52 (m, 4H), 3.87 - 3.88 (s, 6H), 7.07 - 7.09 (d, 2H), 7.20 - 7.26 (m, 3H), 7.30 - 7.32 (m, 1H), 7.47 - 7.61 (t, 1H), 7.64 (s, 1H), 7.82 - 7.87 (m, 2H). MS m/z: 601.8 ($M^+$).

**Example 84**

**Synthesis of 3-[6-14-[5-(nitro)pyridin-2-yllpiperazin-1-yl}-5-phenyl-2-(trifluoromethyl)pyrimidin-4-yl]benzenesulfonamide.**

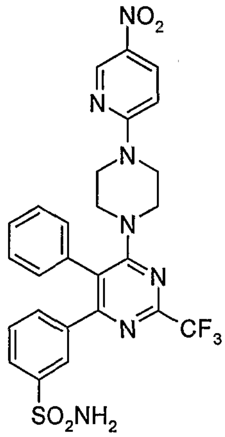

**Step 1:**

**Preparation of 1-(5-nitropyridin-2-yl) piperazine.**

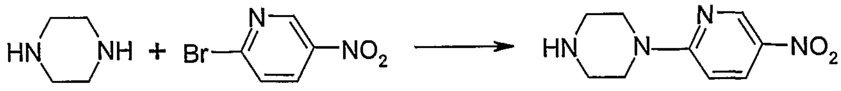

2-Bromo-5-nitropyridine (0.3g, 1.48mmol) was treated with piperazine (0.64g, 7.89mmol) in tetrahydrofuran (4mL) and the reaction mixture was stirred for 30 minutes. Subsequently the reaction mixture was poured onto ice-cold water (25mL) and extracted with ethyl acetate (25mL). The organic layer was washed with brine and evaporated to furnish the product.

**Step 2:**

**Synthesis of 3-[6-{4-[5-(nitro) pyridin-2-yl]piperazin-1-yl}-5-phenyl-2-(trifluoromethyl) pyrimidin-4-yl] benzenesulfonamide.**

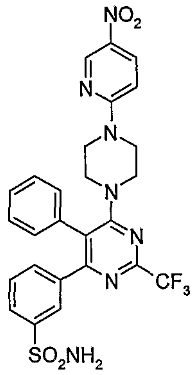

The solution of 3-[6-chloro-5-phenyl-2-(trifluoromethyl)pyrimidin-4-yl]benzenesulfonamide (0.1g, 0.242mmol) in pyridine (2mL) was treated with 1-(5-nitropyridin-2-yl)piperazine (0.075g, 0.363mmol) and stirred for 11 hours. Subsequently the reaction mixture was poured onto ice-cold water and extracted with ethyl acetate (25mL). The organic layer was washed with brine and evaporated to give the crude material. Purification by column chromatography (elution with 70% ethyl acetate in hexane) yielded the title compound. $^1H$-NMR ($CDCl_3$) δ: 3.45 - 3.46 (m, 4H), 3.72 (m, 4H), 6.52 - 6.54 (d, 1H), 7.08 - 7.11 (d, 2H), 7.21 - 7.26 (m, 2H), 7.32 - 7.34 (d, 1H), 7.40 - 7.42 (d, 1H), 7.50 (m, 1H), 7.68 (s, 1H), 7.84 - 7.89 (m, 1H), 8.21 - 8.23 (d, 1H), 8.99 (s, 1H). MS m/z: 585.8 ($M^+$).

**Example 85**

**Synthesis of 3-[6-{4-[5-(amino)pyridin-2-yl]piperazin-1-yl}-4-[4-fluorophenyl]-2-(trifluoromethyl)pyrimidin-5-yl]benzenesulfonamide.**

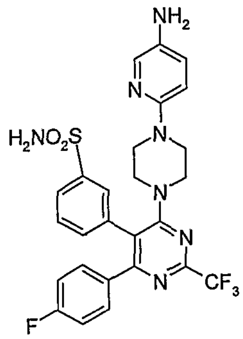

3-[6-{4-[5-(Nitro)pyridin-2-yl]piperazin-1-yl}-4-[4-fluorophenyl]-2-(trifluoro methyl)pyrimidin-5-yl]benzenesulfonamide (0.13g, 0.22mmol) was taken in concentrated hydrochloric acid (1.5 mL) and to this tin (II) chloride dihydrate

(0.145g, 0.65mmol) was added and the reaction mixture was stirred for 24 hours at room temperature. Subsequently the reaction mixture was poured onto crushed ice, neutralized with sodium bicarbonate, and extracted with ethyl acetate. Evaporation of the solvent yielded the required product. $^1$H-NMR (DMSO-$d_6$) δ: 3.13 (m, 4H), 3.30 (m, 4H), 4.58 (br, 2H, $D_2O$ exchangeable), 6.57 - 6.59 (m, 1H), 6.88 (d, 1H), 7.04 - 7.09 (m, 2H), 7.12 - 7.14 (m, 2H), 7.39 (br, 2H, $D_2O$ exchangeable), 7.41 (d, 1H), 7.50 - 7.55 (m, 2H), 7.73 - 7.77 (m, 2H). MS m/z: 574.1 ($M^+$+1).

## Example 86

**Synthesis of 4-[5-(acetylamino)pyridin-2-yl]piperazin-1-yl-5-(4-fluorophenyl)-6-[4-(methylsulfonyl)phenyl]-2-(trifluoromethyl)pyrimidine**

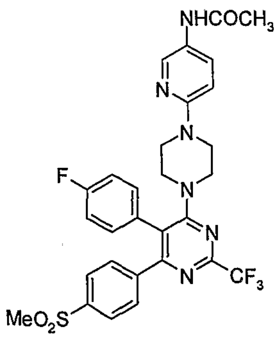

4-Chloro-5-(4-fluorophenyl)-6-[4-(methylsulfonyl)phenyl]-2-(trifluoro methyl)pyrimidine (0.15g, 0.35mmol) was treated with 1-(5-nitropyridin-2-yl)piperazine (0.087g, 0.418mmol) and diisopropylethylamine (0.06mL, 0.35mmol), acetonitrile (2.5mL) and heated at 60-65°C for 2 hours. Subsequently the reaction mixture was precipitated by the addition of diisopropyl ether (2mL). The above-obtained solid (0.11g, 0.182mmol) was taken up in acetic acid (2mL) and tin (II) chloride dihydrate (0.123g, 0.182mmol) was added to it. Stirring was continued further for 11 hours, and then the reaction mixture was poured onto ice-cold water, and extracted with ethyl acetate (25mL x 2). After neutralization with sodium bicarbonate, the organic layer was evaporated to obtain the crude material, which was purified by column chromatography (2% MeOH in dichloromethane) to yield the title compound. $^1$H-NMR (DMSO-$d_6$) δ: 1.99 (s, 3H), 3.19 (s, 3H), 3.29 - 3.40 (m, 8H), 6.77 (d, 1H), 7.19 - 7.23 (m, 2H), 7.31 - 7.34 (m, 2H), 7.36 (d, 2H), 7.74 (d, 1H), 7.80 (d, 2H), 8.24 (d, 1H), 9.77 (s, 1H, $D_2O$ exchangeable). MS m/z: 615.1 ($M^+$+1).

## Example 87

**Synthesis of N-({3-[4-pyridin-2-yl]piperazin-1-yl)-6-(4-fluorophenyl)-2-(trifluoromethyl) pyrimidin-5-yl]phenyl}sulfonyl) acetamide.**

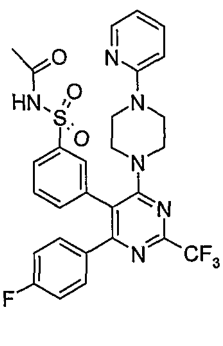

3-[4-{4-(5-Pyridin-2-yl)piperazin-1-yl)}-6-(4-fluorophenyl)-2-(trifluoro methyl)pyrimidin-5-yl]benzenesulfonamide (0.1g, 0.178mmol) was treated with acetyl chloride (0.35mL) and the reaction mixture was stirred for 40 hours. Subsequently it was poured onto crushed ice, extracted with dichloromethane (25mL), and washed with brine. The organic layer was evaporated to furnish the required compound. $^1$H-NMR (DMSO-$d_6$) δ: 1.81 (s, 3H), 3.32 - 3.37 (m,

8H), 6.62 (t, 1H), 6.75 (d, 1H), 7.02 - 7.07 (m, 2H), 7.11 - 7.14 (m, 2H), 7.50 - 7.59 (m, 3H), 7.80 (s, 1H), 7.84 (d, 1H), 8.06 (d, 1H), 12.09 (brs, 1H, $D_2O$ exchangeable). MS m/z: 601.1 ($M^+$+1).

**The following compound was prepared according to the procedure described above.**

| | | |
|---|---|---|
| **88** | 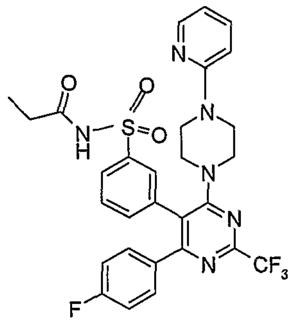 | $^1$H-NMR ($CDCl_3$) δ: 1.02 - 1.05 (t, 3H), 2.18 - 2 .24 (q, 2H), 3.45 - 3.49 (m, 8H), 6.60 - 6.65 (m, 2H), 6.85 - 6.89 (m, 2H), 7.08 - 7.10 (m, 2H), 7.37 (d, 1H), 7.47 - 7.50 (m, 2H), 7.94 (s, 1H), 8.02 (d, 1H), 8.12(d, 1H). MS m/z: 615.1 ($M^+$+1). |

## Example 89

**Synthesis of 1-{5-[3-(aminosulfonyl)phenyl]-6-(4-fluorophenyl)-2-(trifluoromethyl)pyrimidin-4-yl}piperidine-4-carboxylic** acid.

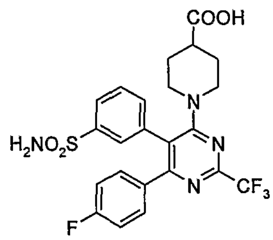

A solution of ethyl 1-{5-[3-(aminosulfonyl)phenyl]-6-(4-fluorophenyl)-2-(trifluoromethyl)pyrimidin-4-yl]piperidine-4-carboxylate (0.4g, 0.725mmol) in tetrahydrofuran (4mL) was treated with lithium hydoxide monohydrate (0.036g, 0.87mmol) in water (0.2mL) and was stirred for 17 hours. Subsequently the reaction mixture was poured onto ice-cold water, acidified with dilute hydrochloric acid and extracted with dichloromethane (25mL). Evaporation of organic layer furnished the required product. $^1$H-NMR (DMSO-$d_6$) δ: 1.39 (m, 2H), 1.64 (m, 2H), 2.09 (m, 1H), 2.84 - 2.89 (m, 2H), 3.66 (m, 2H), 7.02 - 7.07 (m, 2H), 7.07 - 7.13 (m, 2H), 7.37 - 7.39 (m, 2H), 7.49 - 7.53 (m, 1H), 7.69 (s, 1H), 7.72 - 7.74 (d, 1H), 12.25 (br, 1H, $D_2O$ exchangeable). MS m/z: 525.0 ($M^+$+1).

## Example 90

**Synthesis of 4-[4-(methoxyaminocarbonyl)piperidin-1-yl}-5-(4-fluoro phenyl)-6-[4-(methylsulfonyl)phenyl]-2-(trifluoromethyl) pyrimidine.**

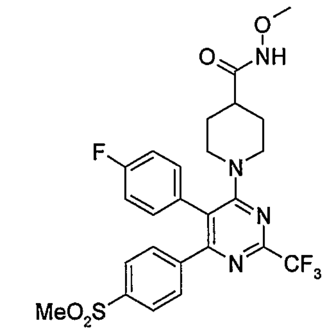

A solution of 1-{5-(4-fluorophenyl)-6-[4-(methylsulfonyl)phenyl]-2-(trifluoromethyl)pyrimidin-4-yl}piperidine-4-carboxylic acid (0.15g, 0.30mmol) in dichloromethane (5mL) was treated with O-methyl hydroxylamine hydrochloride (0.028g, 0.30mmol), 1-(3-dimethylaminopropyl)-3-ethylcarbodiimide hydrochloride (0.127g, 0.663mmol), 1-hydroxybenzotriazole (0.008g, 0.066mmol) and diisopropylethylamine (0.042, 0.33mmol). After 2 hours of stirring the reaction mixture was poured onto ice-cold water, extracted with dichloromethane, and washed with brine. Evaporation of the organic layer furnished the required compound. $^1$H-NMR (DMSO-$d_6$) δ: 1.46 (m, 4H), 2.14 (m, 1H), 2.73 - 2.78 (m, 2H), 3.18 (s, 3H), 3.53 (s, 3H), 3.81 - 3.84 (m, 2H), 7.24 (d, 2H), 7.31 - 7.36 (m, 5H), 7.75 (d, 2H), 11.01 (br, 1H, $D_2O$ exchangeable). MS m/z: 535.1 ($M^+$+1).

## Example 91

**Synthesis of methyl 3-methoxy-4-({6-[4-(methylsulfonyl)phenyl]-5-(4-fluorophenyl)-2-(trifluoromethyl)pyrimidin-4-yl}oxy)benzoate.**

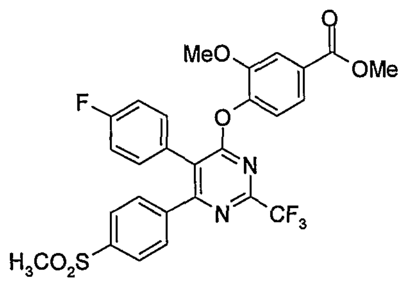

4-Chloro-5-(4-fluorophenyl)-6-[4-(methylsulfonyl)phenyl]-2-(trifluoro methyl)pyrimidine (0.5g, 1.63mmol), methyl vanillate (0.423g, 2.33mmol), potassium carbonate (0.24g, 1.74mmol) and acetonitrile (7mL) were stirred at room temperature for 2 hours, and subsequently the reaction mixture was refluxed for 6 hours. Further, potassium carbonate (0.08g, 0.58mmol) and vanillic ester (0.12g, 0.66mmol) were added to the reaction mixture and the refluxing was continued for another 4 hours. Subsequently the reaction mixture was poured onto ice-cold water, extracted with ethyl acetate (25mL), and washed with brine solution. Evaporation of the organic layer yielded the required product. $^1$H-NMR (DMSO-$d_6$) δ: 3.24 (s, 3H), 3.81 (s, 3H), 3.88 (s, 3H), 7.26 - 7.30 (m, 2H), 7.44 (d, 1H), 7.49 - 7.53 (m, 2H), 7.61 - 7.69 (m, 4H), 7.91 (d, 2H). MS m/z: 576.8 ($M^+$+1).

## Example 92

**Synthesis of 3-methoxy-4-({6-(4-fluorophenyl)-5-{3-(aminosulfonyl) phenyl]-2-(trifluoromethyl)pyrimidin-4-yl}oxy)-N-methoxybenzamide.**

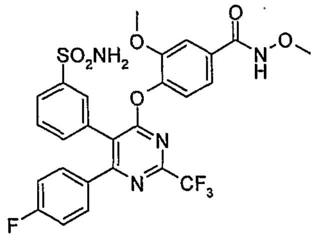

**Step 1:**

**Preparation of 4-hydroxy-N-3-dimethoxybenzamide.**

Vanillic acid (1.0g, 5.93mmol), O-methylhydroxylamine (0.5g, 5.99mmol), 1-(3-dimethylaminopropyl)-3-ethylcarbodiimide hydrochloride (1.37g, 7.12mmol), and 1-hydroxybenzotriazole (0.095g, 0.713mmol), diisopropylethylamine (0.76g, 5.93mmol) in dichloromethane (8mL) were stirred for 2 hours. Subsequently the reaction mixture was poured onto cold water and extracted with dichloromethane (50mL). The crude material obtained on evaporation of the organic layer was purified by column chromatography; elution with 1.5% MeOH in dichloromethane yielded the pure compound.

**Step 2:**

**Synthesis of 3-methoxy-4-({6-(4-fluorophenyl)-5-[3-(aminosulfonyl)phenyl]-2-(trifluoromethyl)pyrimidin-4-yl}oxy)-N-methoxybenzamide.**

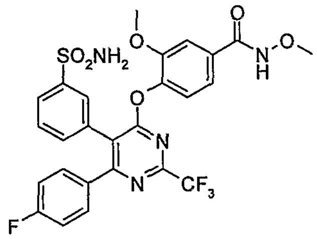

A suspension of 3-[4-chloro-6-(4-fluorophenyl)-2-(trifluoromethyl)pyrimidin-5-yl]benzenesulfonamide (0.15g, 0.35mmol), 4-hydroxy-N,3-dimethoxybenzamide (0.102g, 0.52mmol) and potassium carbonate (0.52mmol) in acetonitrle (3mL) were heated to relfux (65°C) for 2 hours. Subsequently the reaction mixture was poured onto ice-cold water, extracted with dichloromethane (50mL), and washed with brine. Evaporation of the organic layer furnished a crude material, which was purified by column chromatography; elution with 2% MeOH in dichloromethane furnished the required compound. $^1$H-NMR (DMSO-$d_6$) δ: 3.73 (s, 3H), 3.79 (s, 3H), 7.16 - 7.20 (m, 2H), 7.37 (d, 1H), 7.41 - 7.45 (m, 5H), 7.53 (s, 1H), 7.62 - 7.63 (d, 2H), 7.85 (d, 1H), 7.89 (s, 1H). 11.9 (s, 1H); MS m/z: 593 ($M^+$+1).

**Similarly the following compound was made**

| | | |
|---|---|---|
| **93** | 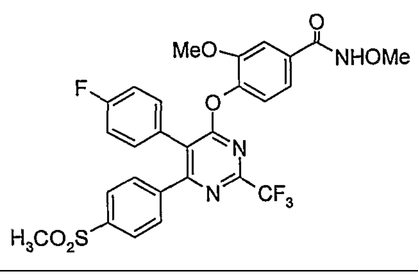 | $^1$H-NMR (DMSO-$d_6$) δ: 3.24 (s, 3H), 3.73 (s, 3H), 3.79 (s, 3H), 7.26 - 7.30 (m, 2H), 7.36 - 7.39 (m, 1H), 7.42 (s, 1H), 7.49 (d, 2H), 7.51 - 7.54 (m, 2H), 7.61 - 7.63 (m, 1H), 7.90 (d, 2H), 11.85 (br, 1H, $D_2O$ exchangeable). MS m/z: 592 ($M^+$+1). |

**Example 94**

**Synthesis of 5-amino-1-[5,6-diphenyl-2-(trifluoromethyl)pyrimidin-4-yl]-3-methyl-1H-pyrazole-4-carbonitrile.**

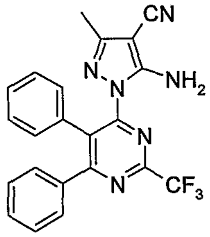

1-Methoxyethylidene malononitrile (0.17g, 1.36mmol) (prepared from malononitrile and triethyl orthoacetate by heating with acetic anhydride) was heated with 4-hydrazino-5,6-diphenyl-2-(trifluoromethyl)pyrimidine (0.15g, 0.45mmol) in methanol (6mL), overnight at 60-65°C. The solid that separated out from the reaction mixture was filtered and washed with methanol (5mL), to yield the title compound. $^1$H-NMR (DMSO-$d_6$) δ: 1.97 (s, 3H), 6.97 (br, 2H, $D_2O$ exchangeable), 7.07 (d, 2H), 7.26 - 7.38 (m, 7H), 7.40 - 7.41 (m, 1H); MS m/z: 421.1 ($M^+$+1).

**Example 95**

**Synthesis of ethyl 5-amino-1-[5,6-diphenyl-2-(trifluoromethyl) pyrimidin-4-yl]-3-(methylthio)-1H-pyrazole-4-carboxylate.**

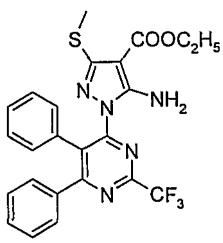

Ethyl 2-cyano-3,3-bis(methylthio)acrylate (0.3g, 1.36mmol) was heated with 4-hydrazino-5,6-diphenyl-2-(trifluoromethyl)pyrimidine (0.15g, 0.45mmol) in methanol (6mL) overnight at 60-65°C. The solid that separated out from the reaction mixture was filtered and washed with isopropylalcohol (5mL), to furnish the required compound. $^1$H-NMR (DMSO-$d_6$) δ: 1.22 - 1.25 (t, 3H), 3.16 (s, 3H), 4.11 - 4.17 (q, 2H), 4.36 (br, 2H, $D_2O$ exchangeable), 7.12 (d, 2H), 7.25 - 7.35 (m, 7H), 7.69 (d, 1H). MS m/z: 500.1 ($M^+$+1).

**The following compounds were prepared according to the procedure described above.**

| Ex. | Structure | Analytical Data |
|---|---|---|
| 96 | 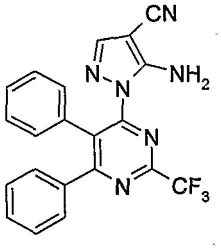 | $^1$H-NMR ($CDCl_3$) δ: 6.11 (s, 1H), 6.99 - 7.01 (d, 2H), 7.23 - 7.35 (d, 8H). MS m/z: 407.1 ($M^+$+1). |

(continued)

| Ex. | Structure | Analytical Data |
|---|---|---|
| **97** | 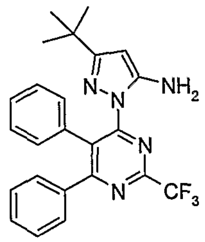 | $^1$H-NMR ($CDCl_3$) $\delta$: 0.86 (s, 9H), 5.21 (br, 2H, $D_2O$ exchangeable), 5.35 (s, 1H), 7.00 - 7.03 (d, 2H), 7.19 - 7.29 (m, 8H). MS m/z: 438.1 ($M^+$+1). |
| **98** | 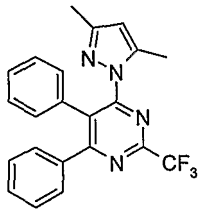 | $^1$H-NMR ($CDCl_3$) $\delta$: 2.04 (s, 3H), 2.21 (s, 3H), 5.85 (s, 1H), 6.94 (d, 2H), 7.18 - 7.32 (m, 6H), 7.35 (d, 2H). MS m/z: 395.1 ($M^+$+1). |
| **99** | 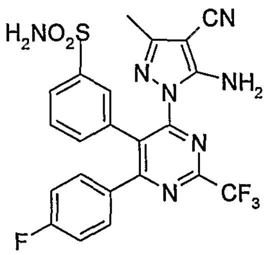 | $^1$H-NMR ($CDCl_3$) $\delta$: 1.86 (s, 3H), 6.44 (s, 2H), 6.93 - 6.98 (m, 2H), 7.20 - 7.29 (m, 4H), 7.40 - 7.44 (m, 1H), 7.66 (s, 1H), 7.83 - 7.88 (m, 1H). MS m/z: 518.0 ($M^+$+1). |
| **100** | 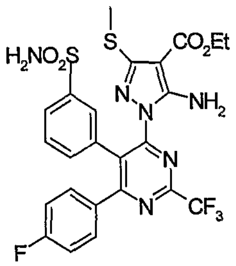 | $^1$H-NMR ($CDCl_3$) $\delta$: 1.33 - 1.35 (t, 3H), 1.56 (s, 3H), 4.27 - 4.32 (m, 2H), 6.92 - 6.96 (m, 2H), 7.18 - 7.20 (m, 2H), 7.37 - 7.47 (m, 2H), 7.66 (s, 1H), 7.81 - 7.84 (m, 1H). MS m/z: 599.0 ($M^+$+1). |
| **101** | 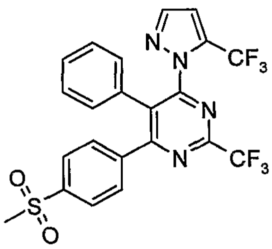 | $^1$H-NMR ($CDCl_3$) $\delta$: 3.02 (s, 3H), 6.63 (s, 1H), 7.02 (d, 2H), 7.29 - 7.31 (m, 2H), 7.35 (m, 1H), 7.55 (d, 2H), 7.83 (d, 2H), 8.40 (s, 1H). MS m/z: 513.0 ($M^+$+1). |
| **102** | 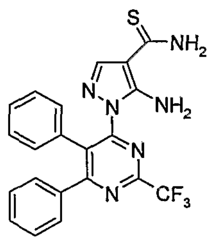 | $^1$H-NMR ($CDCl_3$) $\delta$: 6.56 (bs, 2H), 7.00 (d, 2H), 7.22 - 7.34 (m, 5H), 8.28 (s, 2H). MS m/z: 441.0 ($M^+$+1). |

(continued)

| Ex. | Structure | Analytical Data |
|---|---|---|
| **103** | 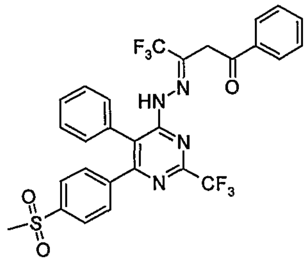 | $^1$H-NMR ($CDCl_3$) $\delta$: 3.00 (s, 3H), 3.54 (q, 2H), 6.42 (d, 2H), 6.84 (d, 1H), 7.00 - 7.05 (m, 1H), 7.20 - 7.24 (m, 2H), 7.31 - 7.33 (m, 2H), 7.45 - 7.51 (m, 3H), 7.77 - 7.81 (m, 2H), 7.96 (d, 2H). MS m/z: 607.0 ($M^+$+1). |
| **104** | 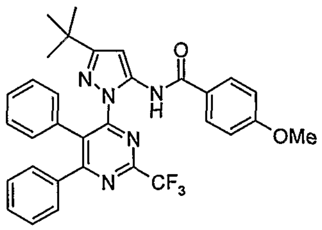 | $^1$H-NMR ($CDCl_3$) $\delta$: 0.92 (s, 9H), 3.92 (s, 3H), 6.86 (s, 1H), 7.00 - 7.03 (m, 2H), 7.21 - 7.29 (m, 10H), 7.96 (d, 2H), 11.24 (s, 1H). MS m/z: 572.1 ($M^+$+1). |
| **105** | 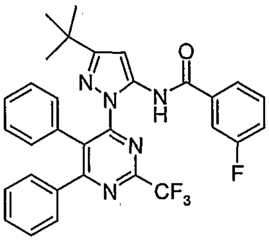 | $^1$H-NMR ($CDCl_3$) $\delta$: 0.92 (s, 9H), 6.88 (s, 1H), 7.00 - 7.01 (m, 2H), 7.23 - 7.29 (m, 6H), 7.30 (d, 2H), 7.52 (d, 2H), 7.73 (d, 1H), 7.94 (d, 1H), 11.40 (s, 1H). MS m/z: 560.1 ($M^+$+1). |
| **106** | 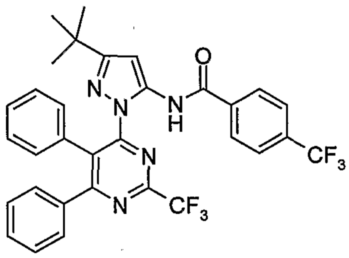 | $^1$H-NMR ($CDCl_3$) $\delta$: 0.92 (s, 9H), 6.91 (s, 1H), 7.01 - 7.03 (m, 2H), 7.23 - 7.31 (m, 8H), 7.81 (d, 2H), 8.10 (d, 2H), 11.52 (s, 1H). MS m/z: 610.1 ($M^+$+1). |
| **107** | 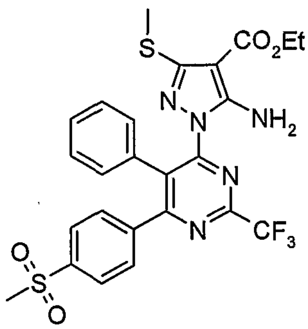 | $^1$H-NMR ($CDCl_3$) $\delta$: 1.35 (t, 3H), 1.59 (s, 3H), 3.01 (s, 3H), 4.27 - 4.32 (dd, 2H), 7.02 (s, 2H), 7.38 - 7.41 (m, 5H), 7.78 - 7.81 (m, 2H). MS m/z: 578.0 ($M^+$+1). |

Example 108

**Synthesis of 5-amino-1-[5,6-diphenyl-2-(trifluoromethyl) pyrimidin-4-yl]-3-(methylthio)-N-phenyl-1H-pyrazole-4-carboxamide.**

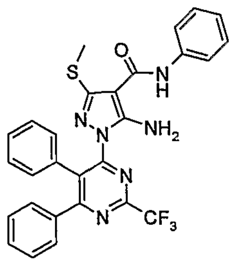

**Step 1:**

**Preparation of 2-cyano-3,3-bis(methylthio)-N-phenylacrylamide.**

2-Cyano-N-phenylacetamide (1.0gm, 6.25mmol) was treated with sodium hydride 60% (1.13g, 28.13mmol) in tetrahydrofuran under ice-cold conditions and stirring for 15 minutes. Carbon disulfide (0.9mL, 15.65mmol) was added to the above mixture and the stirring was continued at ice-cold condition for a further 15 minutes. Methyliodide (2.22g, 15.65mmol) was added to the above under the same ice-cold conditions and the stirring was continued at room temperature overnight. Subsequently the reaction mixture was acidified with dilute hydrochloric acid (5mL) and extracted with ethyl acetate (50mL). Evaporation of the organic layer yielded an oily crude material.

**Step 2:**

**Synthesis of 5-amino-1-[5,6-diphenyl-2-(trifluoromethyl)pyrimidin-4-yl]-3-(methylthio)-N-phenyl-1H-pyrazole-4-carboxamide.**

A solution of 4-hydrazino-5,6-diphenyl-2-(trifluoromethyl)pyrimidine (0.2gm, 0.63mmol) in methanol (6mL) was heated with 2-cyano-3,3-bis(methylthio)-N-phenylacrylamide (0.5g, 1.89mmol) in at 60-65°C overnight. The solid that separated out was filtered and washed with isopropyl alcohol (3mL) to yield the required product. $^1$H-NMR ($CDCl_3$) δ: 1.98 (s, 3H), 6.96 - 7.57 (m, 15H), 7.25-7.30 (2H, $D_2O$ exchangeable), 8.86 (br, 1H, $D_2O$ exchangeable). MS m/z: 547.1 ($M^+$+1).

**The following compound was made by the procedure mentioned above**

| | | |
|---|---|---|
| **109** | 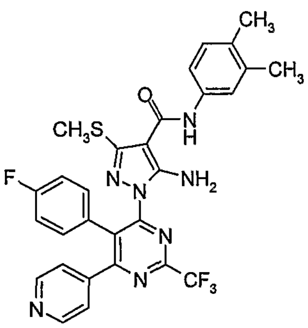 | $^1$H-NMR ($CDCl_3$) δ: 1.98 (s, 3H), 2.17 (s, 3H), 2.19 (s, 3H), 7.05 (d, 1H), 7.14 (m, 4H), 7.20 - 7.22 (m, 2H), 7.30 - 7.32 (m, 2H), 7.33 (d, 2H), 8.55 - 8.56 (d, 2H), 8.69 (br, 1H, $D_2O$ exchangeable). MS m/z: 594.1 ($M^+$+1). |

**Example 110**

**Synthesis of 1-(2,6-dichlorophenyl)-3-{1-[5,6-diphenyl-2-(trifluoromethyl)pyrimidin-4-yl]-3-t-butyl-1H-pyrazol-5-yl}urea.**

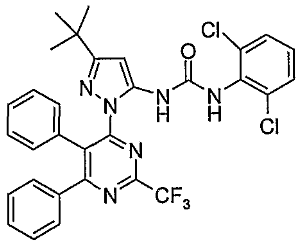

The solution of 3-*t*-butyl-1-[5,6-diphenyl-2-(trifluoromethyl)pyrimidin-4-yl]-1H-pyrazol-5-amine (0.23mmol) in dichloromethane (3mL) was treated with 2,6-dichlorophenyl isocyanate (0.056g, 0.3mmol) in the presence of triethylamine (0.05mL), and the reaction was mixture was stirred at room temperature, overnight. Subsequently water (15mL) was added to above and it was extracted with ethyl acetate (25mL). The organic layer was evaporated and the crude material purfied by column chromatography; elution with 1.5% of ethyl acetate in hexane yielded the title compound. $^1$H-NMR ($CDCl_3$) $\delta$: 0.86 (s, 9H), 6.58 (s, 1H), 7.02 - 7.05 (m, 3H), 7.14 (d, 2H), 7.24 - 7.33 (m, 8H), 8.43 - 8.44 (br, 1H, $D_2O$ exchangeable), 10.52 (br, 1H, $D_2O$ exchangeable). MS m/z: 625 ($M^+$).

## Example 111

**Synthesis of 4-[4-(methylthio)phenyl]-5,6-diphenyl-2-(trifluoro methyl)pyrimidine.**

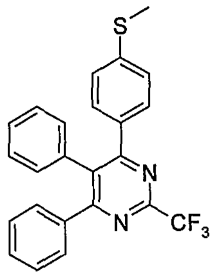

4-Chloro-5,6-diphenyl-2-(trifluoromethyl)pyrimidine (0.2g, 0.6mmol, prepared according to the procedure described in PCT/IB03/02879) was heated to reflux with *tetra-kis* triphenyl palladium (0) (0.068g, 0.058mmol), aqueous solution of potassium carbonate (0.16g in 0.6mL water), 4-(methylthio)benzene boronic acid (0.168g, 1mmol) and toluene (20mL) under a nitrogen atmosphere overnight. The reaction mixture was acidified with dilute hydrochloric acid 10mL and extracted with ethyl acetate. The organic layer was concentrated; the crude obtained was triturated with ether and filtered to yield the title compound. $^1$H-NMR (DMSO-$d_6$) $\delta$: 2.45 (s, 3H), 7.13 - 7.15 (m, 4H), 7.23 - 7.34 (m, 8H), 7.61 (d, 2H). MS m/z: 423.1 ($M^+$+1).

**Similarly the following compound was prepared.**

| | | |
|---|---|---|
| **112** | 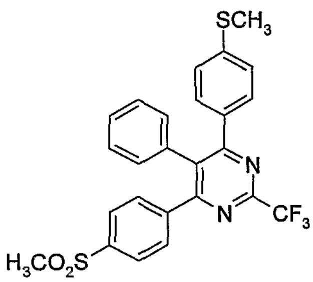 | $^1$H-NMR (DMSO-$d_6$) $\delta$: 2.45 (s, 3H), 3.22 (s, 3H), 7.15 - 7.18 (m, 4H), 7.26 - 7.34 (m, 5H), 7.52 - 7.54 (d, 2H), 7.83 - 7.85 (d, 2H). MS m/z: 501 ($M^+$+1). |

Described below are the examples of pharmacological assays used for finding out the efficacy of the compounds of the present invention wherein their protocols and results are provided.

## *In vitro* evaluation of cyclooxygenase-2 (COX-2) inhibition activity

The compounds of this invention exhibited *in vitro* inhibition of COX-2. The COX-2 inhibition activities of the compounds illustrated in the examples were determined by the following method.

## Human Whole Blood Assay

Human whole blood provides a protein and cell rich milieu appropriate for the study of the biochemical efficacy of anti-inflammatory compounds such as selective COX-2 inhibitors. Studies have shown that normal human blood does not contain the COX-2 enzyme. This correlates with the observation that COX-2 inhibitors have no effect on prostaglandin $E_2$ ($PGE_2$) production in normal blood. These inhibitors were active only after incubation of human blood with lipopolysaccharide (LPS), which induces COX-2 production in the blood.

Fresh blood was collected in tubes containing sodium heparin by vein puncture from healthy male volunteers. The subjects should have no apparent inflammatory conditions and should have not taken NSAIDs for at least 7 days prior to blood collection. Blood was preincubated with aspirin *in vitro* (12μg/ml, at time zero) to inactivate COX-1 for 6 hours. Then test compounds (at various concentrations) or vehicle were added to blood, the blood was stimulated with LPS B:4 (10 μg/ml) and incubated for another 18 hours at 37°C water bath. After which the blood was centrifuged, plasma was separated and stored at -80°C (J. Pharmacol. Exp.Ther, 271, 1705, 1994; Proc. Natl. Acad. Sci. USA, 96, 7563, 1999). The plasma was assayed for $PGE_2$ using Cayman ELISA kit as per the procedure outlined by the manufacturer (Cayman Chemicals, Ann Arbor, USA). Representative results of PGE-2 inhibition are shown in the Table I:

**Table I**

| Example No. | %PGE-2 Inhibition in hWBA | | |
|---|---|---|---|
| | 0.25μM | 1μM | 10μM |
| 4* | 6.10 | 22.85 | 35.63 |
| 7* | 26.17 | 1.90 | NA |
| 22 | 15.64 | 20.40 | 36.37 |
| *Reference compound | | | |

## COX-1 and COX-2 enzyme based assay.

COX-1 and COX-2 enzyme based assays were carried out to check the inhibitory potential of the test compounds on the production of prostaglandin by purified recombinant COX-1/COX-2 enzyme (Proc. Nat. Acad. Sci. USA, 88, 2692-2696, 1991; J. Clin. Immunoassay 15, 116-120, 1992) In this assay, the potential of the test compound to inhibit the production of prostaglandin either by COX-1 or COX-2 from arachidonic acid (substrate) was measured. This was an enzyme based *in-vitro* assay to evaluate selective COX inhibition with good reproducibility.

Arachidonic acid was converted to $PGH_2$ (Intermediate product) by COX1/COX-2 in the presence or absence of the test compound. The reaction was carried out at 37°C and after 2 minutes it was stopped by adding 1M HCl. Intermediate product $PGH_2$ was converted to a stable prostanoid product $PGF_{2\alpha}$ by $SnCl_2$ reduction. The amount of $PGF_{2\alpha}$ produced in the reaction was inversely proportional to the COX inhibitory potential of the test compound. The prostanoid product was quantified via enzyme immunoassay (EIA) using a broadly specific antibody that binds to all the major forms of prostaglandin, using Cayman ELISA kit as per the procedure outlined by the manufacturer (Cayman Chemicals, Ann Arbor, USA). Representative results of inhbition are shown in theTable II.

**Table II**

| Example No. | % Inhibition in rEnzyme Assay | | | |
|---|---|---|---|---|
| | COX-1 | | COX-2 | |
| | 1μM | 10μM | 1μM | 10μM |
| 2* | 33.84 | 50.41 | 17.31 | 48.9 |
| 51 | 8.27 | 7.1 | NA | 12.29 |
| 53 | 46.29 | 56.85 | 20.04 | 26.70 |

(continued)

| Example No. | % Inhibition in rEnzyme Assay | | | |
|---|---|---|---|---|
| | COX-1 | | COX-2 | |
| | 1μM | 10μM | 1μM | 10μM |
| 54 | 34.42 | 45.53 | NA | 13.71 |
| 57 | NA | 5.7 | 8.29 | 0.66 |
| 58 | 10.22 | 9.59 | 11.24 | 2.62 |
| 82 | 6.29 | 19.24 | NA | 7.03 |
| *Reference compound | | | | |

***In vitro*** **measurement of Tumor Necrosis Factor Alpha (TNF- α).**

This assay determines the effect of test compounds on the production of TNF α in human Peripheral Blood Mononuclear Cells (PBMC). Compounds were tested for their ability to inhibit the activity of TNF α in human PBMC. PBMC were isolated from blood (of healthy volunteers) using BD Vacutainer CPT™ (Cell preparation tube, BD Bio Science) and suspended in RPMI medium (Physiol. Res. 52: 593-598, 2003). The test compounds were pre-incubated with PBMC (0.5million/incubation well) for 15 minutes at 37°C and then stimulated with Lipopolysaccharide (*Escherichia coli*: B4; 1 μg/ml) for 18 hours at 37°C in 5% $CO_2$. The levels of TNF-α in the cell culture medium were estimated using enzyme-linked immunosorbent assay performed in a 96 well format as per the procedure of the manufacturer (Cayman Chemical, Ann Arbor, USA). Representative results of TNF-α inhibition are shown in the Table III.

**Table III**

| Example No. | TNF-α Inhibition (%) at | |
|---|---|---|
| | Conc. (1μM) | Conc. (10μM) |
| 4* | 29.98 | 107.36 |
| 5* | 35.86 | 56.34 |
| 11* | 12.71 | 50.71 |
| 12* | 34.81 | 78.42 |
| 13* | 25.77 | 35.27 |
| 14* | 25.47 | 67.54 |
| 24 | 53.86 | 32.91 |
| 25 | 38.77 | 94.02 |
| 26 | 32.70 | 50.79 |
| 27 | 43.49 | 51.92 |
| 30 | 43.23 | 100 |
| 31 | 37.39 | 62.48 |
| 34 | NA | 80.88 |
| 35 | 17.13 | 86.57 |
| 36 | 51.26 | 26.92 |
| 55 | 81.46 | 91.61 |
| 57 | 84.50 | 88.86 |
| 80 | 29.96 | 75.31 |
| 82 | 37.84 | 58.28 |

(continued)

| Example No. | TNF-$\alpha$ Inhibition (%) at | |
|---|---|---|
| | Conc. (1$\mu$M) | Conc. (10$\mu$M) |
| 66 | 57.85 | 80.68 |
| 84 | 69.71 | 86.49 |
| 86 | 88.92 | 91.50 |
| *Reference compound | | |

***In vitro* measurement of Interleukin-6 (IL-6)**

This assay determines the effect of test compounds on the production of IL-6 in human PBMC (Physiol. Res. 52: 593-598, 2003). Compounds were tested for their ability to inhibit the activity of IL-6 in human PBMC. PBMC were isolated from blood using BD Vacutainer CPT™ Cell preparation tube (BD Bio Science and suspended in RPMI medium. test compounds were pre-incubated with PBMC (0.5million/incubation well) for 15 minutes at 37° C and then stimulated with Lipopolysaccharide (*Escherichia coli*: B4; 1 $\mu$g/ml) for 18 hours at 37°C in 5% $CO_2$. The levels of IL-6 in cell culture medium were estimated using enzyme-linked immunosorbent assay performed in a 96 well format as per the procedure of the manufacturer (Cayman Chemical, Ann Arbor, USA). Representative results of IL-6 inhibition are shown in the Table IV.

**Table IV**

| Example No. | IL-6 Inhibition (%) | |
|---|---|---|
| | Conc. (1$\mu$M) | Conc. (10$\mu$M) |
| 4* | 27.17 | 100 |
| 18 | 32.83 | 41.04 |
| 19 | 27.88 | 41.91 |
| 59 | 26.14 | 36.82 |
| 61 | 24.51 | 54.49 |
| 78 | 22.28 | 25.26 |
| *Reference compound | | |

**Carrageenan induced Paw Edema test in Rat**

The carrageenan paw edema test was performed as described by Winter et al (Proc.Soc.Exp.Biol.Med, 111, 544, 1962). Male wistar rats were selected with body weights equivalent within each group. The rats were fasted for 18 hours with free access to water. The rats were dosed orally with the test compound suspended in the vehicle containing 0.25% carboxymethylcellulose and 0.5% Tween 80. The control rats were administered with vehicle alone. After an hour, the rats were injected with 0.1 ml of 1% Carrageenan solution in 0.9% saline into the sub-plantar surface of the right hind paw. Paw volume was measured using digital plethysmograph before and after 3 hours of carrageenan injection. The average of foot swelling in drug treated animals was compared with that of the control animals. Anti-inflammatory activity was expressed as the percentage inhibition of with control group [Arzneim-Forsch/Drug Res., 43 (I), 1,44-50,1993; Otterness and Bliven, Laboratory Models for Testing NSAIDs, In Non-Steroidal Anti-Inflammatory Drugs, (J. Lombardino, ed.1985)]. Representative results of edema inhibition are shown in the Table V.

**Table V**

| Example No. | Inhibition of edema (%) at 5mg/kg |
|---|---|
| 4* | 17.12 |
| 5* | 14.5 |
| *Reference compound | |

## Ulcerogenic potential

In order to evaluate the compound's role on the ulcer formation, the animals were sacrificed and the stomach was taken out and flushed with 1% formalin. Animals (male wistar 200g) were fasted for 18 hours with free access to water and the test compounds were suspended in 0.5% Tween 80 and 0.25% CMC (carboxymethylcellulose) solution to make a uniform suspension. After 4 hours of oral administration of test compounds, all the animals were sacrificed by cervical dislocation. The stomach was dissected carefully and filled up with a sterile saline solution and embedded in 6% formalin solution. Finally the stomach was cut longnitudinaly and ulcer lesions were observed with computerized stereomicroscope. The test compound treated groups were compared with the vehicle treated groups. Doses selected: 50, 100, 200mg/kg (Marco Romano et al, Journal of clinical Investigation, 1992; 2409-2421.) Representative results of ulcer incidence are shown in the Table VI.

**Table VI**

| Example No. | Ulcer incidence at 5mg/kg |
|---|---|
| 4* | Nil |
| 5* | Nil |
| *Reference compound | |

## Inhibitory Action on Adjuvant Arthritis in rats

Compounds were assayed for their activity on rat adjuvant induced arthritis model according to Theisen-Popp et al., (Agents Actions, 42, 50-55,1994). 6 to 7 weeks old, wistar rats were weighed, marked and assigned to groups [a negative control group in which arthritis was not induced (non-adjuvant control), a vehicle-treated arthritis control group, test substance treated arthritis group]. Adjuvant induced arthritis was induced by an injection of 0.1ml of *Mycobacterium butyricum* (Difco) suspended in mineral oil (5mg/ml) into the sub-plantar region of the right hind paw (J.Pharmacol.Exp.Ther., 284, 714, 1998). Body weight, and paw volumes were measured at various days (0, 4, 14, 21) for all the groups. The test compound or vehicle was administered orally, beginning post injection of adjuvant ('0'day) and continued for 21 days (pre-treatment group). In the post-treatment group, the test compound or vehicle was administered starting from day $14^{th}$ to $21^{st}$ day. On day 21, body weight and paw volume of both right and left hind paws were taken. Spleen, and thymus weights were determined. In addition, the radiographs of both hind paws were taken to assess the tibiotarsal joint integrity. Hind limb below the stifle joint was removed and fixed in 1% formalin saline for the histopathological assessment. At the end of the experiment, serum samples were analysed for inflammatory mediators. The presence or absence of lesions in the stomach was also observed.

Two-factor ('treatment' and 'time') analysis of variance with repeated measures on 'time' was applied to the percentage (%) changes for body weight and foot volumes. A post hoc Dunnett's test was conducted to compare the effect of treatments to vehicle control. A one-way analysis of variance was applied to the thymus and spleen weights followed by the Dunnett's test to compare the effect of treatments to vehicle. Dose-response curves for percentage inhibition in foot volumes on days 4, 14 and 21 were fitted by a 4-parameter logistic function using a nonlinear least Squares' regression. $IC_{50}$ was defined as the dose corresponding to a 50% reduction compared to the vehicle control and was derived by interpolation from the fitted 4-parameter equation.

## LPS induced sepsis for measurement of TNF-α inhibition in mice

The LPS induced sepsis model in mice was performed as described by Les sekut et al (J Lab Clin Med 1994; 124:813-20). Female Swiss albino mice were selected and the body weights were equivalent within each group. The mice were fasted for 20 hours with free access to water. The mice were dosed orally with the test compound suspended in vehicle containing 0.5% Tween 80 in 0.25% Carboxy-methylcellulose sodium salt. The control mice were administered the vehicle alone. After 30 minutes of oral dosing, mice were injected with 500μg of Lipopolysaccharide (*Escherichia coli*, LPS: B4 from Siga) in phosphate buffer saline solution into the intraperitoneal cavity of the mice. After 90 minutes of LPS administration mice were bled via retro-orbital sinus puncture. Blood samples were stored overnight at 4°C. Serum samples were collected by centrifuging the samples at 4000 rpm for 15 minutes at 4°C. Immediately the serum samples were analysed for TNF-α levels using commercially available mouse TNF-α ELISA kit (Amersham Biosciences) and assay was performed by the manufacturer instruction. Representative results of TNF-α inhibition are shown in the Table VII.

**Table VII**

| Example No. | TNF-α Inhibition (%) at 50mg/kg |
|---|---|
| 4* | 38.37 |
| 30 | 84.84 |
| 35 | 59.62 |
| 53 | 70.93 |
| 54 | 63.44 |
| 57 | 52.02 |
| 82 | 54.23 |
| *Reference compound | |

**Anti-cancer screen**

Experimental drugs are screened for anti-cancer activity in three cell lines for their $GI_{50}$, TGI and $LC_{50}$ values (using 5 concentrations for each compound). The cell lines are maintained in DMEM containing 10% fetal bovine serum. 96 well microtiter plates are inoculated with cells in 100 μL for 24 hours at 37°C, 5% $CO_2$, 95% air and 100% relative humidity. 5000 HCT116 cells/well, 5000 NCIH460 cells/well, 10000 U251 cells/well and 5000 MDAMB231 cells/well are plated. A separate plate with these cell lines is also inoculated to determine cell viability before the addition of the compounds ($T_0$).

**Addition of experimental drugs**

Following 24-hour incubation, experimental drugs are added to the 96 well plates. Each plate contains one of the above cell lines and the following in triplicate: 5 different concentrations (0.01, 0.1, 1, 10 and 100 μM) of 4 different compounds, appropriate dilutions of a cytotoxic standard and control (untreated) wells. Compounds are dissolved in dimethylsulfoxide (DMSO) to make 20 mM stock solutions on the day of drug addition and frozen at -20°C. Serial dilutions of these 20 mM stock solutions are made in complete growth medium such that 100 μL of these drug solutions in medium, of final concentrations equaling 0.01, 0.1, 1, 10 and 100 μM can be added to the cells in triplicate. Standard drugs whose anti-cancer activity has been well documented and which are regularly used are doxorubicin and SAHA.

**End-point measurement**

Cells are incubated with compounds for 48 hours followed by the addition of 10 μL 3-(4,5-Dimethyl-2-thiazolyl)-2,5-diphenyl-2H-tetrazolium (MTT) solution per well and a subsequent incubation at 37°C, 5% $CO_2$, 95% air and 100% relative humidity, protected from light. After 4 hours, well contents are aspirated carefully followed by addition of 150 μL DMSO per well. Plates are agitated to ensure solution of the formazan crystals in DMSO and absorbance read at 570 nm.

**Calculation of $GI_{50}$, TGI and $LC_{50}$**

Percent growth is calculated for each compound's concentration relative to the control and zero measurement wells (To; viability right before compound addition).

If a test well's O.D. value is greater than the $T_0$ measurement for that cell line

$$\% \text{ Growth} = (\text{test} - \text{zero}) / (\text{control} - \text{zero}) \times 100$$

If a test well's O.D. value is lower than the To measurement for that cell line, then

$$\% \text{ Growth} = (\text{test} - \text{zero}) / \text{zero} \times 100$$

Plotting % growth versus experimental drug concentration, $GI_{50}$ is the concentration required to decrease % growth by 50%; TGI is the concentration required to decrease % growth by 100% and $LC_{50}$ is the concentration required to decrease % growth by 150%. Representative results of growth are shown in the Table VIII.

**Table VIII**

| Example No. | $GI_{50}$ (μM) | | |
|---|---|---|---|
| | DU-145 | HCT-116 | NCI-H460 |
| 2* | 3.5 | 2.5 | 8.5 |
| 4* | 1.93 | 2 | 1.65 |
| 38 | 4 | 6 | 3.75 |
| 40 | ND | 3.5 | 3.8 |
| 41 | ND | 7 | 4 |
| 44* | ND | 26 | 4.5 |
| 51 | ND | 30 | 4.0 |
| 73 | ND | 9.2 | 9.0 |
| 109 | ND | 20 | 0.1 |
| *Reference compound | | | |

Wherein, NA indicates No activity and ND indicates Not Done.

## Claims

**1.** A compound of the general formula (I),

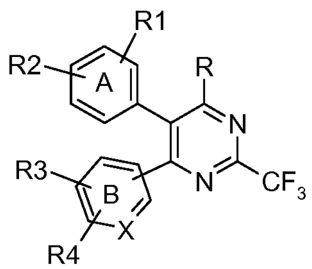

(I)

tautomeric forms, stereoisomers, polymorphs, solvates, and pharmaceutically acceptable salts and compositions thereof, wherein A represents substituted or unsubstituted phenyl group; B represents substituted or unsubstituted phenyl; X represents carbon atom;

- R represents substituted or unsubstituted groups selected from aryl; heteroaryl, the heteroaryl groups being selected from pyridyl, thienyl, furyl, pyrrolyl, oxazolyl, thiazolyl, imidazolyl, thiadiazolyl, tetrazolyl, pyrimidinyl, pyrazine, benzofuranyl, benzimidazolyl and benzothiazolyl; $-OSO_2R'$, wherein R' is selected from substituted or unsubstituted alkyl, aryl, alkyldialkylamino, haloalkyl, heterocyclyl and heteroaryl groups; or R is a substituted or unsubstituted heterocyclyl group chosen from morpholine, piperazine, piperidine, pyrrolidine and thiazolidine, the heterocyclyl group being optionally substituted with substitutents independently selected from substituted or unsubstitued heteroaryl, alkylaryl ($-CH_2$-Aryl), alkylheteroaryl ($-CH_2$-Heteroaryl), substituted heteroarylcarbonyl (-CO-Heteroaryl), cyanoalkyl, alkylsulfonyl, haloalkylsulfonyl, formyl and another substituted or unsubstituted heterocyclyl group; the attachment of the heterocyclyl group to the pyrimidine ring being through carbon or nitrogen;
- $R_1$ represents hydrogen, hydroxy, nitro, azido, halogens, substituted or unsubstituted groups selected from alkyl, haloalkyl, alkoxy, aryl, aryloxy, acyloxy, amino, hydrazine, monoalkylamino, dialkylamino, acylamino, alkylsufonyl, alkylsulfinyl, alkylthio, alkoxycarbonyl, alkoxyalkyl, sulfamoyl, $-SO_2NHNH_2$ $-SO_2Cl$, and carboxylic

acid;

- $R_2$ represents hydrogen, hydroxy, nitro, azido, halogens, substituted or unsubstituted groups selected from alkyl, haloalkyl, alkoxy, aryl, aryloxy, acyloxy, amino, hydrazine, monoalkylamino, dialkylamino, acylamino, alkylsufonyl, alkylsulfinyl, alkylthio, alkoxycarbonyl, alkoxyalkyl, sulfamoyl, $-SO_2NHNH_2$, $-SO_2Cl$, and carboxylic acid;
- $R_3$ represents hydrogen, hydroxy, nitro, azido, halogens, substituted or unsubstituted groups selected from alkyl, haloalkyl, alkoxy, aryl, aryloxy, acyloxy, amino, hydrazine, monoalkylamino, dialkylamino, acylamino, alkylsufonyl, alkylsulfinyl, alkylthio, alkoxycarbonyl, alkoxyalkyl, sulfamoyl, $-SO_2NHNH_2$, $-SO_2Cl$, and carboxylic acid;
- $R_4$ represents hydrogen, hydroxy, nitro, azido, halogens, substituted or unsubstituted groups selected from alkyl, haloalkyl, alkoxy, aryl, aryloxy, acyloxy, amino, hydrazine, monoalkylamino, dialkylamino, acylamino, alkylsufonyl, alkylsulfinyl, alkylthio, alkoxycarbonyl, alkoxyalkyl, sulfamoyl, $-SO_2NHNH_2$, $-SO_2Cl$, and carboxylic acid; and

when the groups R, $R_1$, $R_2$, $R_3$, $R_4$ and R' are substituted by one or more substituents these substituents are selected from halogens, hydroxy, nitro, cyano, ureas, azido, amino, imino-1-phenyl butanone, amide, thioamide, hydrazine, alkyl, alkoxy, haloalkyl, haloalkoxy, cycloalkyl, aryloxy, acyl groups comprising acetyl and benzoyl, haloacyl, acyloxyacyl, heterocyclyl, aryl, heteroaryl, monoalkylamino, dialkylamino, acylamino, alkoxycarbonyl groups comprising methoxycarbonyl and ethoxycarbonyl, aryloxycarbonyl, alkylsulfonyl, haloalkylsulfonyl, arylsulfonyl, alkylsulfinyl, arylsulfinyl, thioalkyl, thioaryl, sulfamoyl, alkoxyalkyl groups, carboxylic acids and its derivatives comprising hydroxamic acid, hydroxamates, esters, amides and acid halides; these substituents being further optionally substituted with substituents selected from hydroxy, alkoxy, halogens, haloalkyl, alkyl and aryl which in turn is optionally further substituted by groups comprising halogens and alkyl.

**2.** The heterocyclic compound of claim 1, selected from a group consisting of:

6-[4-(Methylsulfonyl)phenyl]-5-phenyl-2-(trifluoromethyl)pyrimidin-4-yl napthalenesulfonate;
6-[4-(Methylsulfonyl)phenyl]-5-phenyl-2-(trifluoromethyl)pyrimidin-4-yl-3-chloropropane-1-sulfonate;
6-[4-(Methylsulfonyl)phenyl]-5-phenyl-2-(trifluoromethyl)pyrimidin-4-yl-3-(trifluoromethyl)benzenesulfonate;
6-[4-(Methylsulfonyl)phenyl]-5-phenyl-2-(trifluoromethyl)pyrimidin-4-yl-2-(trifluoromethyl)benzenesulfonate;
6-[4-(Methylsulfonyl)phenyl]-5-phenyl-2-(trifluoromethyl)pyrimidin-4-yl-4-methylbenzenesulfonate;
6-[4-(Methylsulfonyl)phenyl]-5-phenyl-2-(trifluoromethyl)pyrimidin-4-yl-4-nitrobenzenesulfonate;
6-[4-(Methylsulfonyl)phenyl]-5-phenyl-2-(trifluoromethyl)pyrimidin-4-yl-4-trifluoromethoxybenzenesulfonate;
6-[4-(Methylsulfonyl)phenyl]-5-phenyl-2-(trifluoromethyl)pyrimidin-4-yl thiophene-2-sulfonate;
6-[4-(Methylsulfonyl)phenyl]-5-phenyl-2-(trifluoromethyl)pyrimidin-4-yl-4-fluorobenzenesulfonate;
6-[4-(Methylsulfonyl)phenyl]-5-phenyl-2-(trifluoromethyl)pyrimidin-4-yl-2-fluorobenzenesulfonate;
6-[4-(Methylsulfonyl)phenyl]-5-phenyl-2-(trifluoromethyl)pyrimidin-4-yl-(dimethylamino)propanesulfonate;
6-[4-(Methylsulfonyl)phenyl]-5-phenyl-4-(N-benzyl-piperazin-1-yl)-2-(trifluoromethyl)pyrimidine;
4-[4-(4-Fluorophenyl)-6-piperazin-1-yl-2-(trifluoromethyl)pyrimidin-5-yl] benzenesulfonamide;
4-[5-(4-Fluorophenyl)-6-piperazin-1-yl-2-(trifluoromethyl)pyrimidin-4-yl] benzenesulfonamide;
*N*-Methyl-4-[4-(methylsulfonyl)phenyl]-6-piperazin-1-yl-2-(trifluoromethyl)pyrimidin-5-yl]benzenesulfonamide;
4-[4-(Methylsulfonyl)phenyl]-6-piperazin-1-yl-2-(trifluoromethyl) pyrimidin-5-yl]benzenesulfonamide;
4-{4-(Morpholin-4yl)-6-[4-(methylsulfonyl)phenyl]-2-(trifluoromethyl) pyrimidin-5-yl}-*N*-methylbenzenesulfonamide;
5-{4-[4-(Methylsulfonyl)phenyl]-6-piperidin-1-yl-2-(trifluoromethyl) pyrimidin-5-yl]-*N*-methylbenzenesulfonamide;
4-[4-(Methylsulfonyl)phenyl]-6-{4-[(5-methylpyrazin-2-yl)carbonyl] piperazin-1-yl}-5-phenyl-2-(trifluoromethyl)pyrimidine;
6-[4-(Methylsulfonyl)phenyl]-5-phenyl-4-{4-[(1-methyl-1*H*-pyrrol-2-yl) carbonyl]piperazin-1-yl}-2-(trifluoromethyl)pyrimidine;
6-[4-(Methylsulfonyl)phenyl]-4-[4-(5-nitro-2-furoyl)piperazin-1-yl]-5-phenyl-2-(trifluoromethyl)pyrimidine;
*N*-Methyl-4-{4-[4-(5-nitro-2-furoyl)piperazin-1-yl]-6-[4-(methylsulfonyl) phenyl]-2-(trifluoromethyl)pyrimidin-5-yl}benzenesulfonamide;
4-{5-[4-Fluorophenyl]-4-[4-(5-nitro-2-furoyl)piperazin-1-yl]-2-(trifluoro methyl)pyrimidin-6-yl}benzenesulfonamide;
4-{6-[4-Fluorophenyl]-4-[4-(5-nitro-2-furoyl)piperazin-1-yl]-2-(trifluoro methyl)pyrimidin-5-yl}benzenesulfonamide;
6-[4-(Methylsulfonyl)phenyl]-4-{4-[(5-nitro-1*H*-pyrazol-3-yl)carbonyl] piperazin-1-yl}-5-phenyl-2-(trifluoromethyl)pyrimidine;
5,6-Diphenyl-4-[4-(5-nitro-2-furoyl)piperazin-1-yl]-2-(trifluoromethyl) pyrimidine;
6-[4-(Methylsulfonyl)phenyl]-5-phenyl-4-[4-(1,3-thiazol-2-ylmethyl) piperazin-1-yl]-2-(trifluoromethyl)pyrimidine;
4-[4-(Methylsulfonyl)phenyl]-5-phenyl-6-[4-(pyridin-4-ylmethyl) piperazin-1-yl]-2-(trifluoromethyl)pyrimidine;
6-[4-(Methylsulfonyl)phenyl]-4-{4-[(5-nitro-2-thienyl)methyl]piperazin-1-yl}-5-phenyl-2-(trifluoromethyl)pyrimidine;
4,5-Diphenyl-6-(4-pyridin-2-yl-piperazin-1-yl)-2-(trifluoromethyl) pyrimidine;
4-[4-(Methylsulfonyl)phenyl]-5-phenyl-6-(4-pyridin-2-yl-piperazin-1-yl)-2-(trifluoromethyl)pyrimidine;
3-[4-(4-Fluorophenyl)-6-piperazin-1-yl-2-(trifluoromethyl) pyrimidin-5-yl]benzenesulfonamide;
3-[5-Phenyl-6-piperazin-1-yl-2-(trifluoromethyl)pyrimidin-4-yl] benzenesulfonamide;
3-[5-(3-Aminosulfonylphenyl)]-6-piperazin-1-yl-2-(trifluoromethyl) pyrimidin-4-yl]benzenesulfonamide;
3-[4-(4-Fluorophenyl)-6-(4-pyridin-2-ylpiperazin-1-yl)-2-(trifluoromethyl) pyrimidin-5-yl]benzenesulfonamide;
3-[4-(4-Fluorophenyl)-6-(4-pyrimidin-2-ylpiperazin-1-yl)-2-(trifluoromethyl) pyrimidin-5-yl]benzenesulfonamide;
3-[5-Phenyl-6-(1,3-thiazolidin-3-yl)-2-(trifluoromethyl)pyrimidin-4-yl] benzenesulfonamide;
3-[6-(4-Pyrimidin-2-ylpiperazin-1-yl)]-5-phenyl-2-(trifluoromethyl) pyrimidin-4-yl]benzenesulfonamide;
3-[6-(4-Pyridin-2-ylpiperazin-1-yl)]-5-phenyl-2-(trifluoromethyl) pyrimidin-4-yl]benzenesulfonamide;
Ethyl-1-[5-(3-aminosulfonylphenyl)-6-(4-fluorophenyl)-2-(trifluoromethyl) pyrimidin-4-yl]piperidine-4-carboxylate;
Ethyl 1-[5-phenyl-6-(3-aminosulfonylphenyl)1-2-(trifluoromethyl) pyrimidin-4-yl]piperidine-4-carboxylate;
4-[5-Phenyl-6-(3-morpholinosulfonylphenyl)-2-(trifluoromethyl) pyrimidin-4-yl]morpholine;
3-[4-(4-Fluorophenyl)-6-morpholin-4-yl-2-(trifluoromethyl)pyrimidin-5-yl]benzenesulfonamide;
(3*R*)-1-[6-(4-Fluorophenyl)-5-(3-aminosulfonylphenyl)-2-(trifluoro methyl)pyrimidin-4-yl]pyrrolidin-3-ol;
Ethyl (2*S*,4*R*)-4-hydroxy-1-[6-(4-fluorophenyl)-5-(3-aminosulfonyl phenyl)-2-(trifluoromethyl)pyrimidin-4-yl]pyrrolidine-2-carboxylate;
4-[4-(2,6-Dimethoxypyrimidin-4-yl)piperazin-1-yl]-5-(3-aminosulfonyl phenyl)-6-(4-fluorophenyl)-2-(trifluoromethyl)pyrimidine;
5-(4-Fluorophenyl)-4-(4-pyridin-2-ylpiperazin-1-yl)-6-[4-(methylsulfonyl) phenyl]-2-(trifluoromethyl)pyrimidine;
4-(4-Methylsulfonylphenyl)-5-(4-fluorophenyl)-6-(4-pyrimidin-2-yl piperazin-1-yl)-2-(trifluoromethyl)pyrimidine;
4-[5-(4-Fluorophenyl)-6-[4-(methylsulfonyl)phenyl]-2-(trifluoromethyl) pyrimidin-4-yl]piperazine-1-carbaldehyde;
1'-[5-(4-Flurophenyl)-6-(4-methylsulfonylphenyl)-2-(trifluoromethyl) pyrimidin-4-yl]-1,4'-bipiperidine;
3-[4-(4-Fluorophenyl)-6-(1,4'-bipiperidin-1'-yl)-2-(trifluoromethyl) pyrimidin-5-yl]benzenesulfonamide;
3-[4-(2-Furoyl)piperazin-1-yl)-6-(4-fluorophenyl)-2-(trifluoromethyl) pyrimidin-5-yl]benzenesulfonamide;
5-(3-Aminosulfonylphenyl)-4-(4-fluorophenyl)-2-(trifluoromethyl)-6-{4-[3-(trifluoromethyl)phenyl]piperazin-1-yl}pyrimidine;
5-(4-Fluorophenyl)-4-(4-methylsulfonylphenyl)-2-(trifluoromethyl)-6-{4-[3-(trifluoromethyl)phenyl]piperazin-1-yl}pyrimidine;
3-[4-(4-Fluorophenyl)-6-(1,3-thiazolidin-3-yl)-2-(trifluoromethyl) pyrimidin-5-yl]benzenesulfonamide;
1-[5-[3-(Aminosulfonyl)phenyl]-6-(4-fluorophenyl)-2-(trifluoromethyl) pyrimidin-4-yl]pyrrolidine-2-carboxamide;
5-(3-Aminosulfonylphenyl)-4-(4-fluorophenyl)-2-(trifluoromethyl)-6-{4-[(trifluoromethyl)sulfonyl]piperazin-1--yl}pyrimidine;
3-[4-[4-(Methylsulfonyl)piperazin-1-yl]-6-(4-fluorophenyl)-2-(trifluoro methyl)pyrimidin-5-yl]benzenesulfonamide;
3-[4-[4-(Cyanomethyl)piperazin-1-yl]-6-(4-fluorophenyl)-2-(trifluoro methyl)pyrimidin-5-yl]benzenesulfonamide;
3-[4-(4-Fluorophenyl)-6-(1*H*-imidazol-1-yl)-2-(trifluoromethyl) pyrimidin-5-yl]benzenesulfonamide;
5-(4-Fluorophenyl)-4-(1*H*-imidazol-1-yl)-6-[4-(methylsulfonyl)phenyl]-2-(trifluoromethyl)pyrimidine;
3-[6-{4-[5-(trifluoromethyl)pyridin-2-yl]piperazin-1-yl}-5-phenyl-2-(trifluoromethyl)pyrimidin-4-yl]benzenesulfonamide;
3-[6-[4-[2,6-Dimethoxypyrimidin-4-yl]piperazin-1-yl}-5-phenyl-2-(trifluoromethyl)pyrimidin-4-yl]benzenesulfonamide;
3-[6-{4-[5-(Nitro)pyridin-2-yl]piperazin-1-yl}-5-phenyl-2-(trifluoromethyl)pyrimidin-4-yl]benzenesulfonamide;
3-[6-{4-[5-(Amino)pyridin-2-yl]piperazin-1-yl}-4-[4-fluorophenyl]-2-(trifluoromethyl)pyrimidin-5-yl]benzenesulfonamide;
4-[5-(Acetylamino)pyridin-2-yl]piperazin-1-yl-5-(4-fluorophenyl)-6-[4-(methylsulfonyl)phenyl]-2-(trifluoromethyl)pyrimidine;
N-({3-[4-pyridin-2-yl]piperazin-1-yl)-6-(4-fluorophenyl)-2-(trifluoromethyl)pyrimidin-5-yl]phenyl}sulfonyl) acetamide;
4-(4-Fluorophenyl)-5-(3-propionylaminosulfonylphenyl)-6-([4-pyridin-2-yl] piperazin-1-yl)-2-(trifluoromethyl)pyrimidine;
1-{5-[3-(Aminosulfonyl)phenyl]-6-(4-fluorophenyl)-2-(trifluoromethyl) pyrimidin-4-yl}piperidine-4-carboxylic acid;
4-[4-(Methoxyaminocarbonyl)piperidin-1-yl}-5-(4-fluorophenyl)-6-[4-(metlrylsulfonyl)phenyl]-2-(trifluoromethyl) pyrimidine;
S-Amino-1-[5,6-diphenyl-2-(trifluoromethyl)pyrimidin-4-yl]-3-methyl-1*H* -pyrazole-4-carbonitrile;
Ethyl-5-amino-1-[5,6-diphenyl-2-(trifluoromethyl)pyrimidin-4-yl]-3-(methylthio)-1H-pyrazole-4-carboxylate;
5-Amino-1-[5,6-diphenyl-2-(trifluoromethyl)pyrimidin-4-yl]-1*H*-pyrazole-4-carbonitrile;
3-*t*-Butyl-1-[5,6-diphenyl-2-(trifluoromethyl)pyrimidin-4-yl]-1*H*-pyrazol-5-amine;
4-(3,5-Dimethyl-1*H*-pyrazol-1-yl)-5,6-diphenyl-2-(trifluoromethyl) pyrimidine;
3-[4-(5-Amino-4-cyano-3-methyl-1*H*-pyrazol-1-yl)-6-(4-fluorophenyl) -2-(trifluoromethyl)pyrimidin-5-yl]benzenesulfonamide;
Ethyl-5-amino-1-[5-[3-(aminosulfonyl)phenyl]-6-(4-fluorophenyl)-2-(trifluoromethyl)pyrimidin-4-yl]-3-(methylthio)-1*H*-pyrazole-4-carboxylate;
4-[4-(Methylsulfonyl)phenyl]-5-phenyl-2-(trifluoromethyl)-6-[5-(trifluoro methyl)-1*H*-pyrazol-1-yl]pyrimidine;
5-Amino-1-[5,6-diphenyl-2-(trifluoromethyl)pyrimidin-4-yl]-1*H*-pyrazole-4-carbothloamide;
*N*-{1-[5,6-Diphenyl-2-(trifluoromethyl)pyrimidin-4-yl]-3-*t*-butyl-1*H*-pyrazol-5-yl}-4-methoxybenzamide;
*N*-{1-[5,6-Diphenyl-2-(trifluoromethyl)pyrimidin-4-yl]-3-*t*-butyl-1*H*-pyrazol-5-yl}-3-fluorobenzamide;
*N*-{1-[5,6-Diphenyl-2-(trifluoromethyl)pyrimidin-4-yl]-3-*t*-butyl-1*H*-pyrazol-5-yl}-4-(trifluoromethyl)benzamide;
Ethyl-5-amino-1-[5-phenyl-6-[4-(methylsulfonyl)phenyl]-2-(trifluoromethyl)pyrimidin-4-yl]-3-(methylthio)-1*H* pyrazole-4-carboxylate;
5-Amino-1-[5,6-diphenyl-2-(trifluoromethyl)pyrimidin-4-yl]-3-(methyl thio)-*N*-phenyl-1*H*-pyrazole-4-carboxamide;
5-Amino-*N*-(4,5-dimethylphenyl)-1-[5-(4-fluorophenyl)-6-pyridin-4-yl -2-(trifluoromethyl)pyrimidin-4-yl]-3-(methylthio)-1*H*-pyrazole-4-carboxamide;
1-(2,6-Dichlorophenyl)-3-{1-[5,6-diphenyl-2-(trifluoromethyl) pyrimidin-4-yl]-3-t-butyl-1H-pyrazol-5-yl}urea;
4-[4-(Methylthio) phenyl]-5,6-diphenyl-2-(trifluoromethyl)pyrimidine; and 5-Phenyl-4-[4-(methylsulfonyl)phenyl]-6-[4-(methylthio)phenyl]-2-(trifluoromethyl)pyrimidine.

**3.** A pharmaceutical composition comprising a compound of formula (I) as claimed in claim 1, as an active ingredient along with a pharmaceutically acceptable carrier, diluent, excipient or solvate.

**4.** A pharmaceutical composition as claimed in claim 3, wherein the pharmaceutical composition is in a tablet, capsule, powder, syrup, solution, aerosol or suspension.

**5.** A pharmaceutical composition as claimed in claim 3, wherein the amount of the compound in the composition is less than 70 % by weight.

**6.** Use of a compound according to claim 1 or 2 in the manufacture of a medicament for treatment of a pain disorder, inflammation, or an immunological disease.

**7.** Use of a compound according to claim 1 or 2 in the manufacture of a medicament for treatment of rheumatoid arthritis; osteoporosis; multiple myeloma; uveitis; acute and chronic myelogenous leukemia; ischemic heart disease; atherosclerosis; cancer; ischemic-induced cell damage; pancreatic beta cell destruction; osteoarthritis; rheumatoid spondylitis; gouty arthritis; inflammatory bowel disease; adult respiratory distress syndrome (ARDS); psoriasis; Crohn's disease; allergic rhinitis; ulcerative colitis; anaphylaxis; contact dermatitis; muscle degeneration; cachexia; asthma; bone resorption diseases; ischemia reperfusion injury; brain trauma; multiple sclerosis; sepsis; septic shock; toxic shock syndrome or fever and myalgias due to infection.

**8.** Use of a compound according to claim 1 or 2 in the manufacture of a medicament for lowering plasma concentrations of any one of, a combination of, or all of TNF-$\alpha$, IL-1$\beta$ and IL-6.

**9.** Use of a compound according to claim 1 or 2 in the manufacture of a medicament for inhibiting production of cytokines as selected from TNF-$\alpha$, IL-1$\beta$ and IL-6.

**10.** Use of a compound according to claim 1 or 2 in the manufacture of a medicament for treating immunological diseases,

those mediated by cytokines such as TNF-$\alpha$, IL-1$\beta$ and IL-6.

**11.** A compound according to claim 1 or 2 for use in a method of treatment of the human or animal body by therapy.

**12.** The compound of claim 11 which is for use in a method of treatment of a pain disorder, inflammation or an immunological disease.

**Patentansprüche**

**1.** Verbindung der allgemeinen Formel (I)

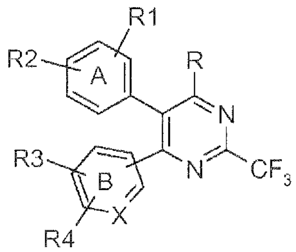

(I)

,

tautomere Formen, Stereoisomere, Polymorphe, Solvate und pharmazeutisch annehmbare Salze und Zusammensetzungen davon, wobei A für eine substituierte oder unsubstituierte Phenylgruppe steht; B für eine substituierte oder unsubstituierte Phenylgruppe steht; X für ein Kohlenstoffatom steht;

- R steht für: substituierte oder unsubstituierte Gruppen, ausgewählt aus Aryl; Heteroaryl, wobei die Heteroarylgruppen ausgewählt sind aus Pyridyl, Thienyl, Furyl, Pyrrolyl, Oxazolyl, Thiazolyl, Imidazolyl, Thiadiazolyl, Tetrazolyl, Pyrimidinyl, Pyrazin, Benzofuranyl, Benzimidazolyl und Benzothiazolyl; $-OSO_2R'$, wobei R' ausgewählt ist aus substituierten oder unsubstituierten Alkyl-, Aryl-, Alkyldialkylamino-, Halogenalkyl-, Heterocyclyl- und Heteroarylgruppen; oder R ist eine substituierte oder unsubstituierte Heterocyclylgruppe, ausgewählt aus Morpholin, Piperazin, Piperidin, Pyrrolidin und Thiazolidin, wobei die Heterocyclylgruppe optional substituiert ist mit Substituenten, die unabhängig ausgewählt sind aus substituiertem oder unsubstituiertem Heteroaryl, Alkylaryl ($-CH_2$-Aryl), Alkylheteroaryl ($-CH_2$-Heteroaryl), substituiertem Heteroarylcarbonyl (-CO-Heteroaryl), Cyanoalkyl, Alkylsulfonyl, Halogenalkylsulfonyl, Formyl und einer anderen substituierten oder unsubstituierten Heterocyclylgruppe; wobei die Anbindung der Heterocyclylgruppe an den Pyrimidinring über Kohlenstoff oder Stickstoff erfolgt;
- $R_1$ steht für: Wasserstoff, Hydroxy, Nitro, Azido, Halogene, substituierte oder unsubstituierte Gruppen, ausgewählt aus Alkyl, Halogenalkyl, Alkoxy, Aryl, Aryloxy, Acyloxy, Amino, Hydrazin, Monoalkylamino, Dialkylamino, Acylamino, Alkylsulfonyl, Alkylsulfinyl, Alkylthio, Alkoxycarbonyl, Alkoxyalkyl, Sulfamoyl, $-SO_2NHNH_2$, $-SO_2Cl$ und Carbonsäure;
- $R_2$ steht für: Wasserstoff, Hydroxy, Nitro, Azido, Halogene, substituierte oder unsubstituierte Gruppen, ausgewählt aus Alkyl, Halogenalkyl, Alkoxy, Aryl, Aryloxy, Acyloxy, Amino, Hydrazin, Monoalkylamino, Dialkylamino, Acylamino, Alkylsulfonyl, Alkylsulfinyl, Alkylthio, Alkoxycarbonyl, Alkoxyalkyl, Sulfamoyl, $-SO_2NHNH_2$, $-SO_2Cl$ und Carbonsäure;
- $R_3$ steht für: Wasserstoff, Hydroxy, Nitro, Azido, Halogene, substituierte oder unsubstituierte Gruppen, ausgewählt aus Alkyl, Halogenalkyl, Alkoxy, Aryl, Aryloxy, Acyloxy, Amino, Hydrazin, Monoalkylamino, Dialkylamino, Acylamino, Alkylsulfonyl, Alkylsulfinyl, Alkylthio, Alkoxycarbonyl, Alkoxyalkyl, Sulfamoyl, $-SO_2NHNH_2$, $-SO_2Cl$ und Carbonsäure;
- $R_4$ steht für: Wasserstoff, Hydroxy, Nitro, Azido, Halogene, substituierte oder unsubstituierte Gruppen, ausgewählt aus Alkyl, Halogenalkyl, Alkoxy, Aryl, Aryloxy, Acyloxy, Amino, Hydrazin, Monoalkylamino, Dialkylamino, Acylamino, Alkylsulfonyl, Alkylsulfinyl, Alkylthio, Alkoxycarbonyl, Alkoxyalkyl, Sulfamoyl, $-SO_2NHNH_2$, $-SO_2Cl$ und Carbonsäure; und

wenn die Gruppen R, $R_1$, $R_2$, $R_3$, $R_4$ und R' substituiert sind mit einem oder mehreren Substituenten, diese Substituenten ausgewählt sind aus Halogenen, Hydroxy, Nitro, Cyano, Harnstoffen, Azido, Amino, Imino-1-phenylbutanon, Amid, Thioamid, Hydrazin, Alkyl, Alkoxy, Halogenalkyl, Halogenalkoxy, Cycloalkyl, Aryloxy, Acylgruppen, umfassend Acetyl und Benzoyl, Halogenacyl, Acyloxyacyl, Heterocyclyl, Aryl, Heteroaryl, Monoalkylamino, Dialkylamino, Acylamino, Alkoxycarbonylgruppen, umfassend Methoxycarbonyl und Ethoxycarbonyl, Aryloxycarbonyl, Alkylsulfonyl, Halogenalkylsulfonyl, Arylsulfonyl, Alkylsulfinyl, Arylsulfinyl, Thioalkyl, Thioaryl, Sulfamoyl, Alkoxyalkylgruppen, Carbonsäuren und deren Derivate, umfassend Hydroxamsäure, Hydroxamate, Ester, Amide und Säurehalogenide; wobei diese Substituenten des Weiteren optional substituiert sind mit Substituenten, ausgewählt aus Hydroxy, Alkoxy, Halogenen, Halogenalkyl, Alkyl und Aryl, die ihrerseits optional des Weiteren substituiert sind mit Gruppen, umfassend Halogene und Alkyl.

**2.** Heterocyclische Verbindung nach Anspruch 1, ausgewählt aus der Gruppe, bestehend aus:

6-[4-(Methylsulfonyl)phenyl]-5-phenyl-2-(trifluormethyl)pyrimidin-4-ylnapthalinsulfonat;
6-[4-(Methylsulfonyl)phenyl]-5-phenyl-2-(trifluormethyl)pyrimidin-4-yl-3-chlorpropan-1-sulfonat;
6-[4-(Methylsulfonyl)phenyl]-5-phenyl-2-(trifluormethyl)pyrimidin-4-yl-3-(trifluormethyl)benzolsulfonat;
6-[4-(Methylsulfonyl)phenyl]-5-phenyl-2-(trifluormethyl)pyrimidin-4-yl-2-(trifluormethyl)benzolsulfonat;
6-[4-(Methylsulfonyl)phenyl]-5-phenyl-2-(trifluormethyl)pyrimidin-4-yl-4-methylbenzolsulfonat;
6-[4-(Methylsulfonyl)phenyl]-5-phenyl-2-(trifluormethyl)pyrimidin-4-yl-4-nitrobenzolsulfonat;
6-[4-(Methylsulfonyl)phenyl]-5-phenyl-2-(trifluormethyl)pyrimidin-4-yl-4-trifluormethoxybenzolsulfonat;
6-[4-(Methylsulfonyl)phenyl]-5-phenyl-2-(trifluormethyl)pyrimidin-4-yl-thiophen-2-sulfonat;
6-[4-(Methylsulfonyl)phenyl]-5-phenyl-2-(trifluormethyl)pyrimidin-4-yl-4-fluorbenzolsulfonat;
6-[4-(Methylsulfonyl)phenyl]-5-phenyl-2-(trifluormethyl)pyrimidin-4-yl-2-fluorbenzolsulfonat;
6-[4-(Methylsulfonyl)phenyl]-5-phenyl-2-(trifluormethyl)pyrimidin-4-yl-(dimethylamino)propansulfonat;
6-[4-(Methylsulfonyl)phenyl]-5-phenyl-4-(N-benzyl-piperazin-1-yl)-2-(trifluormethyl)pyrimidin;
4-[4-(4-Fluorphenyl)-6-piperazin-1-yl-2-(trifluormethyl)pyrimidin-5-yl]benzolsulfonamid;
4-[5-(4-Fluorphenyl)-6-piperazin-1-yl-2-(trifluormethyl)pyrimidin-4-yl]benzolsulfonamid;
*N*-Methyl-4-[4-(methylsulfonyl)phenyl]-6-piperazin-1-yl-2-(trifluormethyl)pyrimidin-5-yl]benzolsulfonamid;
4-[4-(Methylsulfonyl)phenyl]-6-piperazin-1-yl-2-(trifluormethyl)pyrimidin-5-yl]benzolsulfonamid;
4-{4-(Morpholin-4-yl)-6-[4-(methylsulfonyl)phenyl]-2-(trifluormethyl)pyrimidin-5-yl}-*N*-methylbenzolsulfonamid;
5-{4-[4-(Methylsulfonyl)phenyl]-6-piperidin-1-yl-2-(trifluormethyl)pyrimidin-5-yl}-*N*-methylbenzolsulfonamid;
4-[4-(Methylsulfonyl)phenyl]-6-{4-[(5-methylpyrazin-2-yl)carbonyl]piperazin-1-yl}-5-phenyl-2-(trifluormethyl)pyrimidin;
6-[4-(Methylsulfonyl)phenyl]-5-phenyl-4-{4-[(1-methyl-1*H*-pyrrol-2-yl)carbonyl]piperazin-1-yl}-2-(trifluormethyl)pyrimidin;
6-[4-(Methylsulfonyl)phenyl]-4-[4-(5-nitro-2-furoyl)piperazin-1-yl]-5-phenyl-2-(trifluormethyl)pyrimidin;
*N*-Methyl-4-{4-[4-(5-nitro-2-furoyl)piperazin-1-yl]-6-[4-(methylsulfonyl)phenyl]-2-(trifluormethyl)pyrimidin-5-yl}benzolsulfonamid;
4-{5-[4-Fluorphenyl]-4-[4-(5-nitro-2-furoyl)piperazin-1-yl]-2-(trifluormethyl)pyrimidin-6-yl}benzolsulfonamid;
4-{6-[4-Fluorphenyl]-4-[4-(5-nitro-2-furoyl)piperazin-1-yl]-2-(trifluormethyl)pyrimidin-5-yl}benzolsulfonamid;
6-[4-(Methylsulfonyl)phenyl]-4-{4-[(5-nitro-1H-pyrazol-3-yl)carbonyl]piperazin-1-yl}-5-phenyl-2-(trifluormethyl)pyrimidin;
5,6-Diphenyl-4-[4-(5-nitro-2-furoyl)piperazin-1-yl]-2-(trifluormethyl)pyrimidin;
6-[4-(Methylsulfonyl)phenyl]-5-phenyl-4-[4-(1,3-thiazol-2-ylmethyl)piperazin-1-yl]-2-(trifluormethyl)pyrimidin;
4-[4-(Methylsulfonyl)phenyl]-5-phenyl-6-[4-(pyridin-4-ylmethyl)piperazin-1-yl]-2-(trifluormethyl)pyrimidin;
6-[4-(Methylsulfonyl)phenyl]-4-{4-[(5-nitro-2-thienyl)methyl]piperazin-1-yl}-5-phenyl-2-(trifluormethyl)pyrimidin;
4,5-Diphenyl-6-(4-pyridin-2-yl-piperazin-1-yl)-2-(trifluormethyl)pyrimidin;
4-[4-(Methylsulfonyl)phenyl]-5-phenyl-6-(4-pyridin-2-yl-piperazin-1-yl)-2-(trifluormethyl)pyrimidin;
3-[4-(4-Fluorphenyl)-6-piperazin-1-yl-2-(trifluormethyl)pyrimidin-5-yl]benzolsulfonamid;
3-[5-Phenyl-6-piperazin-1-yl-2-(trifluormethyl)pyrimidin-4-yl]benzolsulfonamid;
3-[5-(3-Aminosulfonylphenyl)]-6-piperazin-1-yl-2-(trifluormethyl)pyrimidin-4-yl]benzolsulfonamid;
3-[4-(4-Fluorphenyl)-6-(4-pyridin-2-ylpiperazin-1-yl)-2-(trifluormethyl)pyrimidin-5-yl]benzolsulfonamid;
3-[4-(4-Fluorphenyl)-6-(4-pyrimidin-2-ylpiperazin-1-yl)-2-(trifluormethyl)pyrimidin-5-yl]benzolsulfonamid;
3-[5-Phenyl-6-(1,3-thiazolidin-3-yl)-2-(trifluormethyl)pyrimidin-4-yl]benzolsulfonamid;
3-[6-(4-Pyrimidin-2-ylpiperazin-1-yl)]-5-phenyl-2-(trifluormethyl)pyrimidin-4-yl]benzolsulfonamid;
3-[6-(4-Pyridin-2-ylpiperazin-1-yl)]-5-phenyl-2-(trifluormethyl)pyrimidin-4-yl]benzolsulfonamid;
Ethyl-1-[5-(3-aminosulfonylphenyl)-6-(4-fluorphenyl)-2-(trifluormethyl)pyrimidin-4-yl]piperidin-4-carboxylat;
Ethyl-1-[5-phenyl-6-(3-aminosulfonylphenyl)1-2-(trifluormethyl)pyrimidin-4-yl]piperidin-4-carboxylat;
4-[5-Phenyl-6-(3-morpholinosulfonylphenyl)-2-(trifluormethyl)pyrimidin-4-yl]morpholin;
3-[4-(4-Fluorphenyl)-6-morpholin-4-yl-2-(trifluormethyl)pyrimidin-5-yl]benzolsulfonamid;
(3R)-1-[6-(4-Fluorphenyl)-5-(3-aminosulfonylphenyl)-2-(trifluormethyl)pyrimidin-4-yl]pyrrolidin-3-ol;
Ethyl-(2*S*,4*R*)-4-hydroxy-1-[6-(4-fluorphenyl)-5-(3-aminosulfonylphenyl)-2-(trifluormethyl)pyrimidin-4-yl]pyrrolidin-2-carboxylat;
4-[4-(2,6-Dimethoxypyrimidin-4-yl)piperazin-1-yl]-5-(3-aminosulfonylphenyl)-6-(4-fluorphenyl)-2-(trifluormethyl)pyrimidin;
5-(4-Fluorphenyl)-4-(4-pyridin-2-ylpiperazin-1-yl)-6-[4-(methylsulfonyl)phenyl]-2-(trifluormethyl)pyrimidin;
4-(4-Methylsulfonylphenyl)-5-(4-fluorphenyl)-6-(4-pyrimidin-2-yl-piperazin-1-yl)-2-(trifluormethyl)pyrimidin;
4-[5-(4-Fluorphenyl)-6-[4-(methylsulfonyl)phenyl]-2-(trifluormethyl)pyrimidin-4-yl]piperazin-1-carbaldehyd;
1'-[5-(4-Fluorphenyl)-6-(4-methylsulfonylphenyl)-2-(trifluormethyl)pyrimidin-4-yl]-1,4'-bipiperidin;
3-[4-(4-Fluorphenyl)-6-(1,4'-bipiperidin-1'-yl)-2-(trifluormethyl)pyrimidin-5-yl]benzolsulfonamid;
3-[4-(2-Furoyl)piperazin-1-yl)-6-(4-fluorphenyl)-2-(trifluormethyl)pyrimidin-5-yl]benzolsulfonamid;
5-(3-Aminosulfonylphenyl)-4-(4-fluorphenyl)-2-(trifluormethyl)-6-{4-[3-(trifluormethyl)phenyl]piperazin-1-yl}pyrimidin;
5-(4-Fluorphenyl)-4-(4-methylsulfonylphenyl)-2-(trifluormethyl)-6-{4-[3-(trifluormethyl)phenyl]piperazin-1-yl}pyrimidin;
3-[4-(4-Fluorphenyl)-6-(1,3-thiazolidin-3-yl)-2-(trifluormethyl)pyrimidin-5-yl]benzolsulfonamid;
1-[5-[3-(Aminosulfonyl)phenyl]-6-(4-fluorphenyl)-2-(trifluormethyl)pyrimidin-4-yl]pyrrolidin-2-carboxamid;
5-(3-Aminosulfonylphenyl)-4-(4-fluorphenyl)-2-(trifluormethyl)-6-{4-[(trifluormethyl)sulfonyl]piperazin-1-yl}pyrimidin;
3-[4-[4-(Methylsulfonyl)piperazin-1-yl]-6-(4-fluorphenyl)-2-(trifluormethyl)pyrimidin-5-yl]benzolsulfonamid;
3-[4-[4-(Cyanomethyl)piperazin-1-yl]-6-(4-fluorphenyl)-2-(trifluormethyl)pyrimidin-5-yl]benzolsulfonamid;
3-[4-(4-Fluorphenyl)-6-(1*H*-imidazol-1-yl)-2-(trifluormethyl)pyrimidin-5-yl]benzolsulfonamid;
5-(4-Fluorphenyl)-4-(1*H*-imidazol-1-yl)-6-[4-(methylsulfonyl)phenyl]-2-(trifluormethyl)pyrimidin;
3-[6-{4-[5-(trifluormethyl)pyridin-2-yl]piperazin-1-yl}-5-phenyl-2-(trifluormethyl)pyrimidin-4-yl]benzolsulfonamid;
3-[6-14-[2,6-Dimethoxypyrimidin-4-yllpiperazin-1-yl}-5-phenyl-2-(trifluormethyl)pyrimidin-4-yl]benzolsulfonamid;
3-[6-{4-[5-(Nitro)pyridin-2-yl]piperazin-1-yl}-5-phenyl-2-(trifluormethyl)pyrimidin-4-yl]benzolsulfonamid;
3-[6-{4-[5-(Amino)pyridin-2-yl]piperazin-1-yl}-4-[4-fluorphenyl]-2-(trifluormethyl)pyrimidin-5-yl]benzolsulfonamid;
4-[5-(Acetylamino)pyridin-2-yl]piperazin-1-yl-5-(4-fluorphenyl)-6-[4-(methylsulfonyl)phenyl]-2-(trifluormethyl)pyrimidin;
N-({3-[4-Pyridin-2-yl]piperazin-1-yl)-6-(4-fluorphenyl)-2-(trifluormethyl)pyrimidin-5-yl]phenyl}sulfonyl)acetamid;
4-(4-Fluorphenyl)-5-(3-propionylaminosulfonylphenyl)-6-([4-pyridin-2-yl]piperazin-1-yl)-2-(trifluormethyl)pyrimidin;
1-{5-[3-(Aminosulfonyl)phenyl]-6-(4-fluorphenyl)-2-(trifluormethyl)pyrimidin-4-yl}piperidin-4-carbonsäure;
4-[4-(Methoxyaminocarbonyl)piperidin-1-yl}-5-(4-fluorphenyl)-6-[4-(methylsulfonyl)phenyl]-2-(trifluormethyl)pyrimidin;
5-Amino-1-[5,6-diphenyl-2-(trifluormethyl)pyrimidin-4-yl]-3-methyl-1*H*-pyrazol-4-carbonitril;
Ethyl-5-amino-1-[5,6-diphenyl-2-(trifluormethyl)pyrimidin-4-yl]-3-(methylthio)-1H-pyrazol-4-carboxylat;
5-Amino-1-[5,6-diphenyl-2-(trifluormethyl)pyrimidin-4-yl]-1*H*-pyrazol-4-carbonitril;
3-*t*-Butyl-1-[5,6-diphenyl-2-(trifluormethyl)pyrimidin-4-yl]-1*H*-pyrazol-5-amin;
4-(3,5-Dimethyl-1*H*-pyrazol-1-yl)-5,6-diphenyl-2-(trifluormethyl)pyrimidin;
3-[4-(5-Amino-4-cyano-3-methyl-1*H*-pyrazol-1-yl)-6-(4-fluorphenyl)-2-(trifluormethyl)pyrimidin-5-yl]benzolsulfonamid;
Ethyl-5-amino-1-[5-[3-(aminosulfonyl)phenyl]-6-(4-fluorphenyl)-2-(trifluormethyl)pyrimidin-4-yl]-3-(methylthio)-1*H*-pyrazol-4-carboxylat;
4-[4-(Methylsulfonyl)phenyl]-5-phenyl-2-(trifluormethyl)-6-[5-(trifluormethyl)-1*H*-pyrazol-1-yl]pyrimidin;
5-Amino-1-[5,6-diphenyl-2-(trifluormethyl)pyrimidin-4-yl]-1*H*-pyrazol-4-carbothioamid;
*N*-{1-[5,6-Diphenyl-2-(trifluormethyl)pyrimidin-4-yl]-3-*t*-butyl-1*H*-pyrazol-5-yl}-4-methoxybenzamid;
*N*-{1-[5,6-Diphenyl-2-(trifluormethyl)pyrimidin-4-yl]-3-*t*-butyl-1*H*-pyrazol-5-yl}-3-fluorbenzamid;
*N*-(1-[5,6-Diphenyl-2-(trifluormethyl)pyrimidin-4-yl]-3-*t*-butyl-1*H*-pyrazol-5-yl}-4-(trifluormethyl)benzamid;
Ethyl-5-amino-1-[5-phenyl-6-[4-(methylsulfonyl)phenyl]-2-(trifluormethyl)pyrimidin-4-yl]-3-(methylthio)-1*H*-pyrazol-4-carboxylat;
5-Amino-1-[5,6-diphenyl-2-(trifluormethyl)pyrimidin-4-yl]-3-(methylthio)-*N*-phenyl-1*H*-pyrazol-4-carboxamid;
5-Amino-*N*-(4,5-dimethylphenyl)-1-[5-(4-fluorphenyl)-6-pyridin-4-yl-2-(trifluormethyl)pyrimidin-4-yl]-3-(methylthio)-1*H*-pyrazol-4-carboxamid;
1-(2,6-Dichlorphenyl)-3-{1-[5,6-diphenyl-2-(trifluormethyl)pyrimidin-4-yl]-3-t-butyl-1H-pyrazol-5-yl}harnstoff;
4-[4-(Methylthio)phenyl]-5,6-diphenyl-2-(trifluormethyl)pyrimidin und
5-Phenyl-4-[4-(methylsulfonyl)phenyl]-6-[4-(methylthio)phenyl]-2-(trifluormethyl)pyrimidin.

3. Pharmazeutische Zusammensetzung, umfassend eine Verbindung der Formel (I) wie in Anspruch 1 beansprucht, als aktiven Bestandteil zusammen mit einem pharmazeutisch annehmbaren Träger, Verdünnungsmittel, Hilfsstoff oder Solvat.

4. Pharmazeutische Zusammensetzung wie in Anspruch 3 beansprucht, wobei die pharmazeutische Zusammensetzung eine Tablette, eine Kapsel, ein Pulver, ein Sirup, eine Lösung, ein Aerosol oder eine Suspension ist.

5. Pharmazeutische Zusammensetzung wie in Anspruch 3 beansprucht, wobei die Menge der Verbindung in der Zusammensetzung weniger als 70 Gew.-% beträgt.

6. Verwendung einer Verbindung nach Anspruch 1 oder 2 zur Herstellung eines Medikaments zur Behandlung einer Schmerzerkrankung, einer Entzündung oder einer immunologischen Erkrankung.

7. Verwendung einer Verbindung nach Anspruch 1 oder 2 zur Herstellung eines Medikaments zur Behandlung von rheumatoider Arthritis; Osteoporose; multiplen Myeloma; Uveitis; akuter und chronischer myelogener Leukämie; ischämischer Herzerkrankung; Atherosklerose; Krebs; ischämisch induzierter Zellschädigung; pankreatischer Betazellenzerstörung; Osteoarthritis; rheumatoider Spondylitis; gichtartiger Arthritis; entzündlicher Darmerkrankung; Atemnotsyndrom bei Erwachsenen (ARDS); Psoriasis; Crohnscher Erkrankung; allergischer Rhinitis; ulzerativer Colitis; Anaphylaxe; Kontaktdermatitis; Muskeldegeneration; Kachexie; Asthma; Knochenresorptionserkrankungen; ischämischer Reperfusionsverletzung; Gehirntrauma; multipler Sklerose; Sepsis; septischem Schock; toxischem Schocksyndrom oder Fieber und Myalgien aufgrund von Infektionen.

8. Verwendung einer Verbindung nach Anspruch 1 oder 2 zur Herstellung eines Medikaments zur Senkung der Plasmakonzentration von einem oder einer Kombination oder allen von TNF-$\alpha$, IL-1$\beta$ und IL-6.

9. Verwendung einer Verbindung nach Anspruch 1 oder 2 zur Herstellung eines Medikaments zur Hemmung der Produktion von Zytokinen wie ausgewählt aus TNF-$\alpha$, IL-1$\beta$ und IL-6.

10. Verwendung einer Verbindung nach Anspruch 1 oder 2 zur Herstellung eines Medikaments zur Behandlung immunologischer Erkrankungen, denjenigen vermittelt durch Zytokine wie zum Beispiel TNF-$\alpha$, IL-1$\beta$ und IL-6.

11. Verbindung nach Anspruch 1 oder 2 zur Verwendung in einem Verfahren zur Behandlung des menschlichen oder tierischen Körpers durch Therapie.

12. Verbindung nach Anspruch 11, welche vorgesehen ist zur Verwendung in einem Verfahren zur Behandlung einer Schmerzerkrankung, einer Entzündung oder einer immunologischen Erkrankung.

## Revendications

1. Composé de formule générale (I)

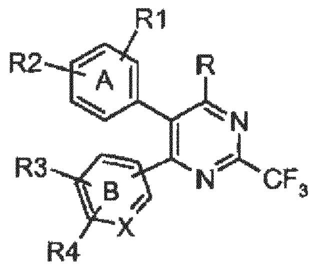

(I)

formes tautomères, stéréoisomères, formes polymorphes, solvats, et sels pharmacologiquement admissibles, d'un tel composé et compositions d'un tel composé,
dans laquelle formule

- A représente un groupe phényle, avec ou sans substituant(s) ;
B représente un groupe phényle, avec ou sans substituant(s),

étant entendu que X représente un atome de carbone ;
R représente un groupe, avec ou sans substituant(s), choisi parmi :

les groupes aryle,
les groupes hétéroaryle choisis parmi pyridyle, thiényle, furyle, pyrrolyle, oxazolyle, thiazolyle, imidazolyle, thiadiazolyle, tétrazolyle, pyrimidinyle, pyrazinyle, benzofuranyle, benzimidazolyle et benzothiazolyle,
les groupes symbolisés par $-OSO_2R'$ où R' représente une entité choisie parmi les groupes alkyle, aryle, dialkyl-amino-alkyle, halogéno-alkyle, hétérocyclyle ou hétéroaryle, avec sans substituant(s),
ou bien R représente un groupe hétérocyclyle avec ou sans substituant(s), choisi parmi les groupes dérivés de morpholine, de pipérazine, de pipéridine, de pyrrolidine ou de thiazolidine, lequel groupe hétérocyclyle, en option, porte un ou des substituant(s) choisi(s) indépendamment parmi les groupes hétéroaryle avec ou sans substituant(s), aryl-alkyle ($-CH_2$-Aryle), hétéroaryl-alkyle ($-CH_2$-Hétéroaryle), hétéroaryl-carbonyle à substituant(s) (-CO-Hétéroaryle), cyano-alkyle, alkyl-sulfonyle, halogéno-alkyl-sulfonyle et formyle et un autre groupe hétérocyclyle avec ou sans substituant(s), étant entendu que la liaison du groupe hétérocyclyle au cycle pyrimidine est établie par l'intermédiaire d'un atome de carbone ou d'azote ;
$R_1$ représente un atome d'hydrogène ou d'halogène, un groupe hydroxyle, nitro ou azido, ou un groupe, avec ou sans substituant(s), choisi parmi les groupes alkyle, halogéno-alkyle, alcoxy, aryle, aryl-oxy, acyl-oxy, amino, hydrazino, monoalkyl-amino, dialkylamino, acyl-amino, alkyl-sulfonyle, alkyl-sulfinyle, alkyl-thio, alcoxy-carbonyle, alcoxy-alkyle, sulfamyle, hydrazino-sulfonyle ($-SO_2NHNH_2$), chloro-sulfonyle ($-SO_2Cl$) et carboxyle ;

- $R_2$ représente un atome d'hydrogène ou d'halogène, un groupe hydroxyle, nitro ou azido, ou un groupe, avec ou sans substituant(s), choisi parmi les groupes alkyle, halogéno-alkyle, alcoxy, aryle, aryl-oxy, acyl-oxy, amino, hydrazino, monoalkyl-amino, dialkylamino, acyl-amino, alkyl-sulfonyle, alkyl-sulfinyle, alkyl-thio, alcoxy-carbonyle, alcoxy-alkyle, sulfamyle, hydrazino-sulfonyle ($-SO_2NHNH_2$), chloro-sulfonyle ($-SO_2Cl$) et carboxyle ;
$R_3$ représente un atome d'hydrogène ou d'halogène, un groupe hydroxyle, nitro ou azido, ou un groupe, avec ou sans substituant(s), choisi parmi les groupes alkyle, halogéno-alkyle, alcoxy, aryle, aryl-oxy, acyl-oxy, amino, hydrazino, monoalkyl-amino, dialkylamino, acyl-amino, alkyl-sulfonyle, alkyl-sulfinyle, alkyl-thio, alcoxy-carbonyle, alcoxy-alkyle, sulfamyle, hydrazino-sulfonyle ($-SO_2NHNH_2$), chloro-sulfonyle ($-SO_2Cl$) et carboxyle ;
et $R_4$ représente un atome d'hydrogène ou d'halogène, un groupe hydroxyle, nitro ou azido, ou un groupe, avec ou sans substituant(s), choisi parmi les groupes alkyle, halogéno-alkyle, alcoxy, aryle, aryl-oxy, acyl-oxy, amino, hydrazino, monoalkyl-amino, dialkylamino, acyl-amino, alkyl-sulfonyle, alkyl-sulfinyle, alkyl-thio, alcoxy-carbonyle, alcoxy-alkyle, sulfamyle, hydrazino-sulfonyle ($-SO_2NHNH_2$), chloro-sulfonyle ($-SO_2Cl$) et carboxyle ;

étant entendu que, quand les groupes symbolisés par R, $R_1$, $R_2$, $R_3$, $R_4$ et R' portent un ou plusieurs substituant(s), celui-ci ou ceux-ci est ou sont choisi(s) parmi les atomes d'halogène et les groupes hydroxyle, nitro, cyano, dérivés d'urée, azido, amino, de type imino-1-phényl-butanone, amido, thioamido, hydrazino, alkyle, alcoxy, halogéno-alkyle, halogéno-alcoxy, cycloalkyle, aryl-oxy, acyle (y compris acétyle et benzoyle), halogénoacyle, acyl-oxy-acyle, hétérocyclyle, aryle, hétéroaryle, monoalkyl-amino, dialkyl-amino, acyl-amino, alcoxy-carbonyle (y compris méthoxy-carbonyle et éthoxy-carbonyle), aryl-oxy-carbonyle, alkyl-sulfonyle, halogéno-alkyl-sulfonyle, aryl-sulfonyle, alkyl-sulfinyle, aryl-sulfinyle, alkyl-thio, aryl-thio, sulfamyle, alcoxy-alkyle, et carboxyle et ses dérivés (y compris les groupes de type acide hydroxamique, hydroxamate, ester, amide ou halogénure d'acide) ; lesquels substituants peuvent, en option, porter eux-mêmes un autre ou d'autres substituant(s) choisi(s) parmi les atomes d'halogène et les groupes hydroxyle, alcoxy, halogéno-alkyle, alkyle et aryle, lesquels, en option, peuvent porter à tour ou des substituant(s) halogéno et/ou alkyle.

**2.** Composé hétérocyclique conforme à la revendication 1, qui est choisi dans l'ensemble formé par les suivants :

naphtalènesulfonate de 6-[4-(méthyl-sulfonyl)-phényl]-5-phényl-2-(trifluoro-méthyl)-pyrimidin-4-yle
3-chloro-propane-1-sulfonate de 6-[4-(méthyl-sulfonyl)-phényl]-5-phényl-2-(trifluoro-méthyl)-pyrimidin-4-yle
3-(trifluoro-méthyl)-benzènesulfonate de 6-[4-(méthyl-sulfonyl)-phényl]-5-phényl-2-(trifluoro-méthyl)-pyrimidin-4-yle
2-(trifluoro-méthyl)-benzènesulfonate de 6-[4-(méthyl-sulfonyl)-phényl]-5-phényl-2-(trifluoro-méthyl)-pyrimidin-4-yle
4-méthyl-benzènesulfonate de 6-[4-(méthyl-sulfonyl)-phényl]-5-phényl-2-(trifluoro-méthyl)-pyrimidin-4-yle
4-nitro-benzènesulfonate de 6-[4-(méthyl-sulfonyl)-phényl]-5-phényl-2-(trifluoro-méthyl)-pyrimidin-4-yle
4-(trifluoro-méthoxy)-benzènesulfonate de 6-[4-(méthyl-sulfonyl)-phényl]-5-phényl-2-(trifluoro-méthyl)-pyrimidin-4-yle

thiophène-2-sulfonate de 6-[4-(méthyl-sulfonyl)-phényl]-5-phényl-2-(trifluoro-méthyl)-pyrimidin-4-yle
4-fluoro-benzènesulfonate de 6-[4-(méthyl-sulfonyl)-phényl]-5-phényl-2-(trifluoro-méthyl)-pyrimidin-4-yle
2-fluoro-benzènesulfonate de 6-[4-(méthyl-sulfonyl)-phényl]-5-phényl-2-(trifluoro-méthyl)-pyrimidin-4-yle
(diméthyl-amino)-propanesulfonate de 6-[4-(méthyl-sulfonyl)-phényl]-5-phényl-2-(trifluoro-méthyl)-pyrimidin-4-yle
6-[4-(méthyl-sulfonyl)-phényl]-5-phényl-4-(N-benzyl-pipérazin-1-yl)-2-(trifluoro-méthyl)-pyrimidine
4-[4-(4-fluoro-phényl)-6-(pipérazin-1-yl)-2-(trifluoro-méthyl)-pyrimidin-5-yl]-benzènesulfonamide
4-[5-(4-fluoro-phényl)-6-(pipérazin-1-yl)-2-(trifluoro-méthyl)-pyrimidin-4-yl]-benzènesulfonamide
N-méthyl-4-{[4-(méthyl-sulfonyl)-phényl]-6-(pipérazin-1-yl)-2-(trifluoro-méthyl)-pyrimidin-5-yl}-benzènesalfonamide
4-{[4-(méthyl-sulfonyl)-phényl]-6-(pipérazin-1-yl)-2-(trifluoro-méthyl)-pyrimidin-5-yl}-benzènesulfonamide
4- {4-(morpholin-4-yl)-6-[4-(méthyl-sulfonyl)-phényl]-2-(trifluoro-méthyl)-pyrimidin-5-yl}-N-méthyl-benzènesulfonamide
5-{4-[4-(méthyl-sulfonyl)-phényl]-6-(pipéridin-1-yl)-2-(trifluoro-méthyl)-pyrimidin-5-yl}-N-méthyl-benzènesulfonamide
4-[4-(méthyl-sulfonyl)-phényl]-6-{4-[(5-méthyl-pyrazin-2-yl)-carbonyl]-pipérazin-1-yl}-5-phényl-2-(trifluoro-méthyl)-pyrimidine
6-[4-(méthyl-sulfonyl)-phényl]-5-phényl-4-{4-[(1-méthyl-1H-pyrrol-2-yl)-carbonyl]-pipérazin-1-yl}-2-(trifluoro-méthyl)-pyrimidine
6-[4-(méthyl-sulfonyl)-phényl]-4-[4-(5-nitro-2-furoyl)-pipérazin-1-yl]-5-phényl-2-(trifluoro-méthyl)-pyrimidine
N-méthyl-4-{4-[4-(5-nitro-2-furoyl)-pipérazin-1-yl]-6-[4-(méthyl-sulfonyl)-phényl]-2-(trifluoro-méthyl)-pyrimidin-5-yl}-benzène-sulfonamide
4- {5-(4-fluoro-phényl)-4-[4-(5-nitro-2-furoyl)-pipérazin-1-yl]-2-(trifluoro-méthyl)-pyrimidin-6-yl}-benzènesulfonamide
4-{6-(4-fluoro-phényl)-4-[4-(5-nitro-2-furoyl)-pipérazin-1-yl] - 2-(trifluoro-méthyl)-pyrimidin-5-yl}-benzènesulfonamide
6-[4-(méthyl-sulfonyl)-phényl]-4-{4-[(5-nitro-1H-pyrazol-3-yl)-carbonyl]-pipérazin-1-yl}-5-phényl-2-(trifluoro-méthyl)-pyrimidine
5,6-diphényl-4-[4-{5-nitro-2-furoyl}-pipérazin-1-yl]-2-(trifluoro-méthyl)-pyrimidine
6-[4-(méthyl-sulfonyl)-phényl]-5-phényl-4-{4-[(1,3-thiazol-2-yl)-méthyl]-pipérazin-1-yl}-2-(trifluoro-méthyl)-pyrimidine
4-[4-(méthyl-sulfonyl)-phényl]-5-phényl-6-{4-[pyridin-4-yl)-méthyl]-pipérazin-1-yl}-2-(trifluoro-méthyl)-pyrimidine
6-[4-(méthyl-sulfonyl)-phényl]-4-{4-[(5-nitro-thién-2-yl)-méthyl]-pipérazin-1-yl}-5-phényl-2-(trifluoro-méthyl)-pyrimidine
4,5-diphényl-6-[4-(pyridin-2-yl)-pipérazin-1-yl]-2-(trifluoro-méthyl)-pyrimidine
4-[4-(méthyl-sulfonyl)-phényl]-5-phényl-6-[4-(pyridin-2-yl)-pipérazin-1-yl]-2-(trifluoro-méthyl)-pyrimidine
3-[4-(4-fluoro-phényl)-6-(pipérazin-1-yl)-2-(trifluoro-méthyl)-pyrimidin-5-yl]-benzènesulfonamide
3-[5-phényl-6-(pipérazin-1-yl)-2-(trifluoro-méthyl)-pyrimidin-4-yl]-benzènesulfonamide
3-[5-(3-sulfamyl-phényl)-6-(pipérazin-1-yl)-2-(trifluoro-méthyl)-pyrimidin-5-yl]-benzènesulfonamide
3- {4-(4-fluoro-phényl)-6-[4-(pyridin-2-yl)-pipérazin-1-yl]-2-(trifluoro-méthyl)-pyrimidin-5-yl}-benzénesulfonamide
3-{4-(4-fluoro-phényl)-6-[4-(pyrimidin-2-yl)-pipérazin-1-yl]-2-(trifluoro-méthyl)-pyrimidin-5-yl}-benzènesulfonamide
3-[5-phényl-6-(1,3-thiazolidin-3-yl)-2-(trifluoro-méthyl)-pyrimidin-4-yl]-benzènesulfonamide
3-{6-[4-(pyrimidin-2-yl)-pipérazin-1-yl]-5-phényl-2-(trifluoro-méthyl)-pyrimidin-4-yl}-benzènesulfonamide
3-{6-[4-(pyridin-2-yl)-pipérazin-1-yl]-5-phényl-2-(trifluoro-méthyl)-pyrimidin-4-yl}-benzènesulfonamide
1-[5-(3-sulfamyl-phényl)-6-(4-fluoro-phényl)-2-(trifluoro-méthyl)-pyrimidin-4-yl]-pipéridine-4-carboxylate d'éthyle
1-[5-phényl-6-(3-sulfamyl-phényl)-2-(trifluoro-méthyl)-pyrimidin-4-yl]-pipéridine-4-carboxylate d'éthyle
4-{5-phényl-6-[3-(morpholino-sulfonyl)-phényl]-2-(trifluoro-méthyl)-pyrimidin-4-yl}-morpholine
3-[4-(4-fluoro-phényl)-6-(morpholin-4-yl)-2-(trifluoro-méthyl)-pyrimidin-5-yl]-benzènesulfonamide
(3R)-1-[6-(4-fluoro-phényl)-5-(3-sulfamyl-phényl)-2-(trifluoro-méthyl)-pyrimidin-4-yl]-pyrrolidine-3-ol
(2S,4R)-4-hydroxy-1-[6-(4-fluoro-phényl)-5-(3-sulfamyl-phényl)-2-(trifluoro-méthyl)-pyrimidin-4-yl]-pyrrolidine-2-carboxylate d'éthyle
4-[4-(2,6-diméthoxy-pyrimidin-4-yl)-pipérazin-1-yl]-5-(3-sulfamyl-phényl)-6-(4-fluoro-phényl)-2-(trifluoro-méthyl)-pyrimidine
5-(4-fluoro-phényl)-4-[4-(pyridin-2-yl)-pipérazin-1-yl]-6-[4-(méthyl-sulfonyl)-phényl]-2-(trifluoro-méthyl)-pyrimidine
4-[4-(méthyl-sulfonyl)-phényl]-5-(4-fluoro-phényl)-6-[4-(pyrimidin-2-yl)-pipérazin-1-yl]-2-(trifluoro-méthyl)-pyrimidine
4-{5-(4-fluoro-phényl)-6-[4-(méthyl-sulfonyl)-phényl]-2-(trifluoro-méthyl)-pyrimidin-4-yl}-pipérazine-1-carbaldéhyde
1'-{5-(4-fluoro-phényl)-6-[4-(méthyl-sulfonyl)-phényl]-2-(trifluoro-méthyl)-pyrimidin-4-yl}-1,4'-bipipéridine
3-[4-(4-fluoro-phényl)-6-(1,4'-bipipéridin-1'-yl)-2-(trifluoro-méthyl)-pyrimidin-5-yl]-benzènesulfonamide
3-{4-[(2-furoyl)-pipérazin-1-yl]-6-(4-fluoro-phényl)-2-(trifluoro-méthyl)-pyrimidin-5-yl}-benzènesulfonamide
5-(3-sulfamyl-phényl)-4-(4-fluoro-phényl)-2-(trifluoro-méthyl)-6-{4-[3-(trifluoro-méthyl)-phényl]-pipérazin-1-yl}-pyrimidine
5-(4-fluoro-phényl)-4-[4-(méthyl-sulfonyl)-phényl]-2-(trifluoro-méthyl)-6-{4-[3-(trifluoro-méthyl)-phényl]-pipérazin-1-yl}-pyrimidine
3-[4-(4-fluoro-phényl)-6-(1,3-thiazolidin-3-yl)-2-(trifluoro-méthyl)-pyrimidin-5-yl]-benzènesulfonamide
1-[5-(3-sulfamyl-phényl)-6-(4-fluoro-phényl)-2-(trifluoro-méthyl)-pyrimidin-4-yl]-pyrrolidine-2-carboxamide
5-(3-sulfamyl-phényl)-4-(4-fluoro-phényl)-2-(trifluoro-méthyl)-6-{4-[3-(trifluoro-méthyl)-sulfonyl]-pipérazin-1-yl}-pyrimidine
3-{4-[4-(méthyl-sulfonyl)-pipérazin-1-yl]-6-(4-fluoro-phényl)-2-(trifluoro-méthyl)-pyrimidin-5-yl}-benzénesulfonamide
3-{4-[4-(cyano-méthyl)-pipérazin-1-yl]-6-(4-fluoro-phényl)-2-(trifluoro-méthyl)-pyrimidin-5-yl}-benzènesulfonamide
3-[4-(4-fluoro-phényl)-6-(1H-imidazol-1-yl)-2-(trifluoro-méthyl)-pyrimidin-5-yl}-benzènesulfonamide
5-(4-fluoro-phényl)-4-(1H-imidazol-1-yl)-6-[4-(méthyl-sulfonyl)-phényl]-2-(trifluoro-méthyl)-pyrimidine
3-(6-{4-[5-(trifluoro-méthyl)-pyridin-2-yl]-pipérazin-1-yl}-5-phényl-2-(trifluoro-méthyl)-pyrimidin-4-yl)-benzènesulfonamide
3-{6-[4-(2,6-diméthoxy-pyrimidin-4-yl)-pipérazin-1-yl]-5-phényl-2-(trifluoro-méthyl)-pyrimidin-4-yl}-benzènesulfonamide
3-{6-[4-(5-nitro-pyridin-2-yl)-pipérazin-1-yl]-5-phényl-2-(trifluoro-méthyl)-pyrimidin-4-yl}-benzènesulfonamide
3-{6-[4-(5-amino-pyridin-2-yl)-pipérazin-1-yl]-4-(4-fluoro-phényl)-2-(trifluoro-méthyl)-pyrimidin-5-yl}-benzènesulfonainide
4-{[5-(acétyl-amino)-pyridin-2-yl]-pipérazin-1-yl}-5-(4-fluoro-phényl)-6-[4-(méthyl-sulfonyl)-phényl]-2-(trifluoro-méthyl)-pyrimidine
N-[(3-{4-[(pyridin-2-yl)-pipérazin-1-yl]-6-(4-fluoro-phényl)-2-(trifluoro-méthyl)-pyrimidin-5-yl}-phényl)-sulfonyl]-acétamide
4-(4-fluoro-phényl)-5-[3-(propionyl-sulfamyl)-phényl]-6-[4-(pyridin-2-yl)-pipérazin-1-yl]-2-(trifluoro-méthyl)-pyrimidine
acide 1-{5-{3-sulfamyl-phényl)-6-(4-fluoro-phényl)-2-{trifluoro-méthyl)-pyrimidin-4-yl}-pipéridine-4-carboxylique
4-[4-(méthoxy-carbamyl)-pipéridin-1-yl]-5-(4-fluoro-phényl)-6-[4-(méthyl-sulfonyl)-phényl]-2-(trifluoro-méthyl)-pyrimidine
5-amino-1-[5,6-diphényl-2-(trifluoro-méthyl)-pyrimidin-4-yl]-3-méthyl-1H-pyrazole-4-carbonitrile
5-amino-1-[5,6-diphényl-2-(trifluoro-méthyl)-pyrimidin-4-yl]-3-(méthyl-thio)-1H-pyrazole-4-carboxylate d'éthyle
5-amino-1-[5,6-diphényl-2-(trifluoro-méthyl)-pyrimidin-4-yl]-1H-pyrazole-4-carbonitrile
3-tertiobutyl-1-[5,6-diphényl-2-(trifluoro-méthyl)-pyrimidin-4-yl]-1H-pyrazole-5-amine
4-(3,5-diméthyl-1H-pyrazol-1-yl)-5,6-diphényl-2-(trifluoro-méthyl)-pyrimidine
3-[4-(5-amino-4-cyano-3-méthyl-1H-pyrazol-1-yl)-6-(4-fluoro-phényl)-2-(trifluoro-méthyl)-pyrimidin-5-yl]-benzénesulfonamide
5-amino-1-[5-(3-sulfamyl-phényl)-6-(4-fluoro-phényl)-2-(trifluoro-méthyl)-pyrimidin-4-yl]-3-(méthyl-thio)-1H-pyrazole-4-carboxylate d'éthyle
4-[4-(méthyl-sulfonyl)-phényl]-5-phényl-2-(trifluoro-méthyl)-6-[5-(trifluoro-méthyl)-1H-pyrazol-1-yl]-pyrimidine
5-amino-1-[5,6-diphényl-2-(trifluoro-méthyl)-pyrimidin-4-yl]-1H-pyrazole-4-carbothioamide
N-{1-[5,6-diphényl-2-(trifluoro-méthyl)-pyrimidin-4-yl]-3-tertiobutyl-1H-pyrazol-5-yl}-4-méthoxy-benzamide
N- {1-[5,6-diphényl-2-(trifluoro-méthyl)-pyrimidin-4-yl]-3 -tertiobutyl-1H-pyrazol-5-yl}-3-fluoro-benzamide
N-{1-[5,6-diphényl-2-(trifluoro-méthyl)-pyrimidin-4-yl]-3-tertiobutyl-1H-pyrazol-5-yl}-4-(trifluoro-méthyl)-benzamide
5-amino-1-{5-phényl-6-[4-(méthyl-sulfonyl)-phényl]-2-(trifluoro-méthyl)-pyrimidin-4-yl}-3-(méthyl-thio)-1H-pyrazole-4-carboxylate d'éthyle
5-amino-1-[5,6-diphényl-2-(trifluoro-méthyl)-pyrimidin-4-yl]-3-(méthyl-thio)-N-phényl-1 H-pyrazole-4-carboxamide

5-amino-N-(4,5-diméthyl-phényl)-1-[5-(4-fluoro-phényl)-6-(pyridin-4-yl)-2-(trifluoro-méthyl)-pyrimidin-4-yl]-3-(méthyl-thio)-1H-pyrazole-4-carboxamide
1-(2,6-dichloro-phényl)-3-{1-[5,6-diphényl-2-(trifluoro-méthyl)-pyrimidin-4-yl]-3-tertiobutyl-1H-pyrazol-5-yl)-urée
4-[4-(méthyl-thio)-phényl]-5,6-diphényl-2-(trifluoro-méthyl)-pyrimidine
5-phényl-4-[4-(méthyl-sulfonyl)-phényl]-6-[4-(méthyl-thio)-phényl]-2-(trifluoro-méthyl)-pyrimidine.

**3.** Composition pharmaceutique comprenant un composé de formule (I), conforme à la revendication 1, en qualité d'ingrédient actif, ainsi qu'un véhicule, un diluant, un excipient ou un solvat, pharmacologiquement admissible.

**4.** Composition pharmaceutique conforme à la revendication 3, laquelle composition pharmaceutique se présente sous forme de comprimé, de capsule, de poudre, de sirop, de solution, d'aérosol ou de suspension.

**5.** Composition pharmaceutique conforme à la revendication 3, dans laquelle composition la proportion du composé vaut moins de 70 % en poids

**6.** Utilisation d'un composé conforme à la revendication 1 ou 2 dans la fabrication d'un médicament conçu pour le traitement d'une douleur, d'une inflammation ou d'une maladie immunitaire.

**7.** Utilisation d'un composé conforme à la revendication 1 ou 2 dans la fabrication d'un médicament conçu pour le traitement de l'un des états suivants : polyarthrite rhumatoïde, ostéoporose, myélome multiple, uvéite, leucémie myélogène aiguë ou chronique, cardiopathie ischémique, athérosclérose, cancers, dommage cellulaire induit par ischémie, destruction des cellules bêta du pancréas, arthrose, spondylarthrite ankylosante, arthrite goutteuse, affections intestinales inflammatoires, syndrome de détresse respiratoire de l'adulte (SDRA), psoriasis, maladie de Crohn, rhinite allergique, colite ulcéreuse, anaphylaxie, dermatite de contact, dégénérescence musculaire, cachexie, asthme, maladies de la résorption osseuse, lésions d'ischémie-reperfusion, traumatisme crânien, sclérose en plaques, septicémie, choc septique, syndrome du choc toxique, ou fièvre et myalgies dues à une infection.

**8.** Utilisation d'un composé conforme à la revendication 1 ou 2 dans la fabrication d'un médicament conçu pour abaisser la ou les concentrations plasmatiques de l'une des cytokines TNF-$\alpha$, IL-1$\beta$ et IL-6, ou d'une combinaison de deux d'entre elles ou des trois.

**9.** Utilisation d'un composé conforme à la revendication 1 ou 2 dans la fabrication d'un médicament conçu pour inhiber la production de cytokines choisies parmi les TNF-$\alpha$, IL-1$\beta$ et IL-6.

**10.** Utilisation d'un composé conforme à la revendication 1 ou 2 dans la fabrication d'un médicament conçu pour traiter des maladies immunitaires, notamment celles à médiation par des cytokines telles que les TNF-$\alpha$, IL-1$\beta$ et IL-6.

**11.** Composé conforme à la revendication 1 ou 2 pour utilisation dans un procédé de traitement thérapeutique du corps d'un humain ou d'un animal.

**12.** Composé conforme à la revendication 11, pour utilisation dans un procédé de traitement d'une douleur, d'une inflammation ou d'une maladie immunitaire.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 02074298A1 A
- US 6004813 A
- US 5527546 A
- US 5166137 A
- US 6420385 B
- US 5728704 A
- US 6410729 B
- US 20050107413 A
- WO 2004009560 A
- WO 03084935 A
- WO 9624585 A
- US 6028072 A
- US 5486534 A


### Non-patent literature cited in the description


- **HAWORTH et al.** *Eur J Immunol,* 1991, vol. 21, 2575-79
- **BRENNAN et al.** *Lancet,* 1989, vol. 2, 244-7
- **AREND et al.** *Arthritis Rheum,* 1995, vol. 38, 151-60
- **GOLDENBERG.** *Clin Ther,* 1999, vol. 21, 75-87
- **LUONG et al.** *Ann Pharmacother,* 2000, vol. 34, 743-60
- **LKEIN et al.** *Blood,* 1991, vol. 78 (5), 1198-1204
- **LU et al.** *Err. J. Immunol,* 1992, vol. 22, 2819-24
- **MICHAEL. J. TISDALE.** Biology of Cachexia. *Journal of National Cancer Institute,* 03 December 1997, vol. 89 (23
- **CHANDRASEKHAR et al.** *Clinical Immunol Immunopathol.,* 1990, vol. 55, 382
- **FIRESTEIN.** *Am. J. Pathol.,* 1992, vol. 140, 1309
- **DINARELLO.** *Eur. Cytokine Netw,* 1994, vol. 5, 517-531
- **BRAHN et al.** *Lymphokine Cytokine Res.,* 1992, vol. 11, 253
- **COOPER.** *Clin. Exp.Immunol.,* 1992, vol. 898, 244
- **JAQUES et al.** Enantiomers, Racemates and Resolution. Wiley Interscience, 1981
- *J. Pharmacol. Exp.Ther,* 1994, vol. 271, 1705
- *Proc. Natl. Acad. Sci. USA,* 1999, vol. 96, 7563
- *Proc. Nat. Acad. Sci. USA,* 1991, vol. 88, 2692-2696
- *J. Clin. Immunoassay,* 1992, vol. 15, 116-120
- *Physiol. Res.,* 2003, vol. 52, 593-598
- **WINTER et al.** *Proc.Soc.Exp.Biol.Med,* 1962, vol. 111, 544
- **ARZNEIM-FORSCH.** *Drug Res.,* 1993, vol. 43 (I), 44-50
- **OTTERNESS ; BLIVEN.** Laboratory Models for Testing NSAIDs. *Non-Steroidal Anti-Inflammatory Drugs,* 1985
- **MARCO ROMANO et al.** *Journal of clinical Investigation,* 1992, 2409-2421
- **THEISEN-POPP et al.** *Agents Actions,* 1994, vol. 42, 50-55
- *J.Pharmacol.Exp.Ther.,* 1998, vol. 284, 714
- **LES SEKUT et al.** *J Lab Clin Med,* 1994, vol. 124, 813-20